(19) Europäisches Patentamt / European Patent Office / Office européen des brevets

(11) EP 3 237 640 B1

(12) **EUROPEAN PATENT SPECIFICATION**

(45) Date of publication and mention of the grant of the patent:
**08.07.2020 Bulletin 2020/28**

(21) Application number: **15831140.7**

(22) Date of filing: **23.12.2015**

(51) Int Cl.:
***C12Q 1/6886*** *(2018.01)*

(86) International application number:
**PCT/EP2015/081170**

(87) International publication number:
**WO 2016/102674 (30.06.2016 Gazette 2016/26)**

(54) **A DNA-METHYLATION TEST FOR PROSTATE CANCER**

DNS-METHYLIERUNGSTEST AUF PROSTATAKREBS

TEST DE MÉTHYLATION D'ADN POUR LE CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2014 EP 14199999**

(43) Date of publication of application:
**01.11.2017 Bulletin 2017/44**

(73) Proprietor: **University College Dublin National University Of Ireland, Dublin**
**Dublin 4 (IE)**

(72) Inventor: **PERRY, Antoinette**
**Dublin 4 (IE)**

(74) Representative: **Purdylucey Intellectual Property**
**6-7 Harcourt Terrace**
**D02 FH73 Dublin 2 (IE)**

(56) References cited:
**EP-A1- 1 780 292**
**EP-A1- 1 918 710**
**EP-A1- 2 213 749**
**WO-A1-2013/041731**
**WO-A1-2014/012176**
**WO-A2-2007/022146**
**US-A1- 2014 274 767**

- **TUGCU V ET AL: "676 PHOTOSELECTIVE VAPORIZATION OF THE PROSTATE (PVP) VERSUS TRANSURETHRAL RESECTION OF THE PROSTATE (TURP): A PROSPECTIVE NONRANDOMIZED BI-CENTRE TRIAL, 2-YEAR FOLLOW-UP", EUROPEAN UROLOGY SUPPLEMENTS, ELSEVIER BV, NL, vol. 6, no. 2, 1 March 2007 (2007-03-01), page 191, XP027162240, ISSN: 1569-9056 [retrieved on 2007-03-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 237 640 B1

## Description

### Technical Field

The invention relates to the detection of a biomarker in a biological sample to test for the presence of prostate cancer. Specifically, the invention relates to the detection of a plurality of biomarkers in a biological sample to distinguish the presence of aggressive prostate cancer from non-aggressive prostate cancer or no cancer.

### Background to the Invention

Prostate cancer (PCa) is the most common non-cutaneous malignancy in men in the Western world. An estimated 1.1 million new cases were diagnosed in 2012, accounting for 15% of all male cancers worldwide. Ireland is currently experiencing one of the highest incidences of PCa in Europe, with approximately 3,000 new cases diagnosed per annum, representing 30% of all invasive cancers in men. With an ageing Western population and spread of Western culture (particularly diet), the global incidence is predicted to rise dramatically; the National Cancer Registry predicts the incidence in Ireland to rise by between 104-288% by 2040.

It is often said that "most men die *with* and *not because of* their prostate cancer". This is explained by the fact that most prostate tumours have a slow, long natural trajectory, posing little likelihood of clinical manifestation, and deemed indolent in nature; 10-year survival rates for PCa are close to 100%. Nevertheless, a proportion of prostate tumours are highly aggressive, and are associated with the lethal form of the disease. Whilst PSA (prostate specific antigen) screening and improvements in treatments have reduced PCa mortality, this disease accounted for an -307,000 deaths in 2012, making it the 5$^{th}$ leading cause of male cancer-related deaths worldwide. Identifying molecular correlates to discern between aggressive and indolent tumors at an early stage (whilst potentially curable), is one of the greatest unmet clinical needs in this field. This will become even more pressing as the differential between the total number of PCa cases diagnosed and the number of lethal PCa cases grows.

Early detection and diagnosis of PCa involves a combination of a PSA blood test, a digital rectal examination (DRE) and histological examination of transrectal ultrasound (TRUS)-guided biopsy cores, respectively. Several major problems confound the early detection of PCa. There are an estimated 25-45 million PSA tests performed worldwide every year, Widespread PSA testing has significantly increased PCa incidence and led to overtreatment of low-risk disease with little likelihood of clinical manifestation. A further problem with PSA is its poor tumour-specificity; its high false-positive rate means that two-thirds of men who undergo invasive TRUS-biopsy have no tumour diagnosed. There are an estimated 10 million prostate biopsies performed worldwide/annum. Unnecessary TRUS-biopsies create an enormous burden on our healthcare system and cause significant anxiety, trauma and co-morbidities for patients. Finally, TRUS-biopsies are needle biopsies that sample <5% of the prostate and can thus miss tumour foci or indeed miss high-grade aggressive tumours. Studies addressing the economic burden of cancer in the EU, have estimated costs for PCa diagnosis and treatment over the next 20 years per 100,000 men at €30,284,000 (unscreened population) and €60,695,000 (screened population), €23,669,000 of which can be attributed to over-detected cancers.

Currently, there are no commercially available molecular diagnostics for PCa in widespread clinical practice. Progensa® (Gen-Probe) is a urine-based test of *PCA3* gene expression performed after DRE, with FDA approval for use in men who have had ≥1 previous negative biopsies and for whom a repeat biopsy would be recommended based on current standard of care. The test is used to guide the decision to perform a repeat biopsy only. Its prognostic value is debated and research efforts combining it with the fusion-transcript *TMPRSS2-ERG* are underway in an attempt to address this.

Prolaris® (Myriad Genetics) and onco*type*DX® Prostate Cancer Assay (Genomic Health) are two examples of prognostic gene expression signatures (46 genes and 17 genes, respectively) that are analysed on biopsy tissues to aid prediction of PCa aggressiveness in conjunction with other clinical parameters (Gleason score, PSA). Both tests provide a more individualised risk-assessment of the underlying biology of the patient's tumour and are therefore aimed at guiding the decision between active surveillance and radical treatment in men diagnosed with PCa.

MDxHealth's product ConfirmMDx™ is a PCR-based assay, which measures methylation of a 3-gene panel (*GSTP1*, *RARβ, APC*) in biopsy cores. It is positioned to distinguish patients with a true-negative prostate biopsy from those with occult cancer and akin to Progensa®, is used to guide the decision to perform a repeat biopsy. This same 3-gene panel (ProCaM™) has also been investigated as a urine test to predict biopsy results for PCa, although these studies were inadequately powered.

EP1918710, WO2013041731, EP2213749, Tugcu et al. (European Urology Supplements, vol. 6(3), pp 191 (2007)) and US 2014/274767 describe methylation markers and different sets of methylation markers associated with aggressive prostate cancer. EP1918710, WO2013041731 and EP2213749 all describe similar subject matter in the use of biomarkers GSTP1, APC, and PTGS2; while Tugcu *et al.* describes the use of biomarkers IGFBP3 and GSTP1; and US 2014/274767 describes the use of biomarkers GSTP1, IGFBP3, APC, and PTGS2; all in the act of predicting recurrence or aggressiveness of prostate cancer.

It is an object of the subject invention to overcome at least one of the above-mentioned problems.

## Statements of Invention

In contrast to these prior art technologies, the test presented herein (called epigenetic Cancer of the Prostate test in urine or epiCaPture) is an example of a "first in field" for urine diagnostics of potentially lethal, high-risk PCa. The panel of genes encompasses multiple dysregulated pathways in PCa, which is necessary to address the heterogeneity of the disease. These pathways include intracellular detoxification, the IGF axis, the Wnt axis and inflammation. The test presented herein addresses the unmet clinical needs confounding early detection of PCa. The test is a non-invasive DNA methylation test performed using urine or urine cell-sediment. It comprises a panel of at least 6 genes and an internal control gene. The test described herein offers significant commercial potential as a liquid biopsy for early non-invasive detection of high-risk, potentially lethal PCa. The data show that the test offers the unique advantages of i) better tumour-specificity than PSA, and ii) selective identification of high-risk PCa.

According to the invention, there is provided, as set out in the appended claims, a method of determining the risk of aggressive prostate cancer in an individual diagnosed with prostate cancer, comprising a step of assaying a biological sample obtained from the individual for the presence of at least four methylated regulatory DNA sequence selected from group comprising SEQ ID NOs. to 1 to 6, and calculating a normalized index of methylation (NIM) score, and correlating the presence of the at least four methylated regulatory DNA sequence and the calculated normalized index of methylation (NIM) score with the risk of aggressive prostate cancer. The methylated regulatory DNA sequences as defined by SEQ ID NOs. 1 to 16 correspond to the genes *GSTP1, SFRP2, IGFBP3, IGFBP7, APC, PTGS-2, LXN, MAGPIE-1B*, *DNAH10, ZMIZ1, CENPV, OR2L13, MTMR8, F3, CDH8* and *GALNTL6,* respectively, and correlating the presence of the methylated regulatory DNA sequence(s) of these genes with an increased risk of aggressive prostate cancer. The presence of the methylated regulatory DNA sequence may be detected directly by quantitatively assaying for methylation of the DNA regulatory sequences located adjacent to the associated gene(s).

In one embodiment, the method further comprises the step of assaying for the presence of the *PSA* gene in addition to at least four methylated regulatory DNA sequence corresponding to the genes selected from group comprising *GSTP1*, *SFRP2*, *IGFBP3*, *IGFBP7, APC, PTGS-2, LXN, MAGPIE-1B, DNAH10, ZMIZ1, CENPV, OR2L13, MTMR8, F3, CDH8* and *GALNTL6,* wherein the presence of PSA in combination with positive detection of the at least four methylated regulatory DNA sequence corresponding to the genes selected from group comprising *GSTP1*, *SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6,* correlates with an increased risk of aggressive prostate cancer compared with an individual with prostate cancer who is positive for PSA detection but negative for detection of the at least one methylated regulatory DNA sequence.

In a preferred embodiment, the method involves assaying for methylation of a DNA regulatory sequence specific to the or each gene selected from group comprising *GSTP1, SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6.* Preferably, the DNA regulatory sequence is one or both of SEQ ID NOs 1 or 3, which are specific to the biomarkers *GSTP1* and *IGFBP3*, respectively. Specifically, the DNA regulatory sequence is one or more selected from SEQ ID NOs: 1 to 16, and is specific to the biomarkers selected from *GSTP1, SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6,* respectively. Preferably, the DNA regulatory sequence assayed for is all of SEQ ID NOs: 1 to 16, and are specific to the biomarkers *GSTP1, SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6,* respectively. Ideally, the DNA regulatory sequence assayed for is all of SEQ ID NOs: 1 to 6 and are specific to the biomarkers *GSTP1, SFRP2, IGFBP3, IGFBP7, APC,* and *PTGS-2*, respectively. Preferably, assaying for methylation of the DNA regulatory sequence specific to the or each biomarker is combined with assaying for PSA (internal control, SEQ ID NO. 18).

In one embodiment, the method comprises assaying for at least four DNA regulatory sequence specific to their corresponding genes, at least three DNA regulatory sequences selected from the group comprising: SEQ ID NO.'s 1 to 16, or variants thereof, and at least one selected from the group comprising: SEQ ID NO.'s 17 and 18. Preferably, both SEQ ID NO. 17 and SEQ ID NO. 18 are determined in the method. In this way, the method employs at least three "positive" regulatory DNA sequences (*i.e.* a regulatory DNA sequence associated with presence of the cancer), and at least one "control" biomarker (*i.e.* a biomarker associated with prostate-derived DNA). Ideally, the "positive" regulatory DNA sequences are selected from the group comprising SEQUENCE ID NO's 1 to 6.

The two controls may be used in the method:

1) ACTB (SEQ ID NO. 17): ACTB is measured by quantitative PCR (qPCR) and verifies and quantifies the presence of bisulfite modified DNA in each test sample. The quantity of ACTB is used to calculate an epiCaPture score (a score derived from the method of the invention). The amount of each gene in the method must be normalised relative to the amount of input bisulfite modified DNA in each test sample.

2) KLK3 (SEQ ID NO. 18). Expression of the KLK3 gene (the gene encoding PSA, Prostate Specific Antigen) is measured by quantitative RT-PCR and is used as a positive control to confirm the presence of prostate-derived nucleic acids in the test sample. This is important to carry out, in order to show that a test sample which appears negative for prostate cancer as determined by the method of the invention, is indeed truly negative and it is not simply a virtue of no prostate-derived material present in the bio-specimen. The expression of the KLK3 gene is measured using a commercially available qPCR assay, such as Integrated DNA Technologies (Assay ID Hs.PT.58.38546086).

In one embodiment, the method comprises assaying a biological sample obtained from the individual for the presence of at least four methylated regulatory DNA sequence selected from group comprising SEQ ID NOs. to 1 to 16 and correlating the presence or absence of the at least four methylated regulatory DNA sequence with the risk of aggressive prostate cancer.

In one embodiment, the method comprises assaying a biological sample obtained from the individual for the presence of at least four methylated regulatory DNA sequence selected from group comprising SEQ ID NOs. to 1 to 12 and 14 and correlating the presence or absence of the at least four methylated regulatory DNA sequence with the risk of aggressive prostate cancer.

In one embodiment, the method comprises a step of assaying a biological sample obtained from the individual for the presence of a methylated regulatory DNA sequence from at least three sequences selected from group comprising: SEQUENCE ID NO's 1 to 16 where the DNA regulatory sequences are specific to the biomarkers (genes) *GSTP1, SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6.* In a preferred embodiment, the method comprises a step of assaying a biological sample for three, four, five, or six regulatory DNA sequence selected from group comprising: SEQUENCE ID NO's 1 to 16, where the regulatory DNA sequences are specific to the biomarkers (genes) *GSTP1*, *SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6.* In a particularly preferred embodiment of the invention, the method comprises a step of assaying a biological sample obtained from the individual for the presence of a methylated regulatory DNA sequences defined by the group comprising, or consisting essentially of, SEQUENCE ID NO's 1 to 6where the regulatory DNA sequences are specific to the biomarkers (genes) *GSTP1, SFRP2, IGFBP3, IGFBP7, APC* and *PTGS*-2. Preferably, assaying for methylation of the regulatory DNA sequences where the regulatory DNA sequences are specific to the biomarkers (genes) *GSTP1*, *SFRP2, IGFBP3, IGFBP7, APC, PTGS-2,* is combined with assaying for PSA.

Where a patient is found to be positive for the methylated regulatory DNA sequences of SEQ ID NOs 1 to 6 this correlates with a positive identification of prostate cancer at 97.92% specificity and a false positive rate of 0.04 versus 21% specificity and a false positive rate of 0.79 using PSA detection (applying a threshold of 4ng/ml).

Where a patient is found to be positive for all six of the methylated regulatory DNA sequences defined by SEQ ID NOs. 1 to 6 this correlates with a positive identification of aggressive (high-risk) prostate cancer with a 79% sensitivity and an 82% specificity. The combination of the assay result of only two of these methylated regulatory DNA sequences, namely SEQ ID NO. 1 and SEQ ID NO. 3 (corresponding to the genes GSTP1 and IGFBP3, respectively) in this present analysis correlated with a positive detection of aggressive (high-risk or metastatic) prostate cancer with a sensitivity score of 82% and a specificity score of 82%.

In a preferred embodiment of the invention, the method comprises a step of assaying a biological sample from the individual for the presence of methylated regulatory DNA sequences defined by SEQ ID NO. 1 (GSTP1), and SEQ ID NO. 3 (IGFBP3), in combination with two or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2). The method may also comprise assaying a biological sample from the individual for the presence of methylated regulatory DNA sequences defined by SEQ ID NO. 1 (GSTP1), and SEQ ID NO. 3 (IGFBP3), optionally in combination with one or more methylated regulatory DNA sequences of genes selected from the group comprising: SEQ ID NO 2 (SFRP2), SEQ ID NO. 4 (IGFBP7), SEQ ID NO. 5 (APC), SEQ ID NO. 6 (PTGS2), SEQ ID NO. 7 (LXN), SEQ ID NO. 8 (MAGPIE-1B), SEQ ID NO. 9 (DNAH10), SEQ ID NO. 10 (ZMIZ1), SEQ ID NO. 11 (CENPV), and SEQ ID NO. 12 (OR2L13). The method may also comprise assaying a biological sample from the individual for the presence of methylated regulatory DNA sequences defined by SEQ ID NO. 1 (GSTP1), and SEQ ID NO. 3 (IGFBP3), optionally in combination with one or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2), SEQ ID NO. 7 (LXN), SEQ ID NO. 8 (MAGPIE-1B), SEQ ID NO. 9 (DNAH10), SEQ ID NO. 10 (ZMIZ1), SEQ ID NO. 11 (CENPV), and SEQ ID NO. 12 (OR2L13), SEQ ID NO. 13 (MTMR8), SEQ ID NO. 14 (F3), SEQ ID NO. 15 (CDH8), and SEQ ID NO. 16 (GALNTL6).

The method may also comprise assaying a biological sample from the individual for the presence of methylated regulatory DNA sequences defined by SEQ ID NO. 1 (GSTP1), and SEQ ID NO. 3 (IGFBP3), optionally in combination with one or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2), SEQ ID NO. 7 (LXN), SEQ ID NO. 8 (MAGPIE-1B), SEQ ID NO. 9 (DNAH10), SEQ ID NO. 10 (ZMIZ1), SEQ ID NO. 11 (CENPV), and SEQ ID NO. 12 (OR2L13) and SEQ ID NO. 14 (F3).

Preferably, the biological sample is assayed for the presence of PSA in combination with the methylated regulatory DNA sequences of any one or all of SEQUENCE ID NO's 1 to 6. Preferably, the biological sample is assayed for the presence of PSA in combination with the methylated regulatory DNA sequences of any one or all of SEQUENCE ID NO's 7 to 12. Preferably, the biological sample is assayed for the presence of PSA in combination with the methylated regulatory DNA sequences of any one or all of SEQUENCE ID NO's 7 to 12 and 14. Preferably, the biological sample is assayed for the presence of PSA in combination with the methylated regulatory DNA sequences of any one or all of SEQUENCE ID NO's 13 to 16.

The invention also relates to a kit for assessing prostate cancer status in an individual, comprising components for detecting and/or measuring the level of a methylated regulatory DNA sequence of at least four selected from the group comprising: SEQUENCE ID No's 1 to 6.

The kit preferably comprises a pair of forward and reverse oligonucleotide primers (SEQ ID NOs. 22 to 55) designed to specifically hybridise with bisulfite modified hypermethylated DNA sequences at the regulatory regions of each specific gene as defined by SEQ ID NOs 1 to 6; a fluorescently labelled oligonucleotide probe designed to specifically hybridise with bisulfite modified hypermethylated DNA sequences at the regulatory region of each specific gene (SEQ ID NO. 56 to 72), a set of forward and reverse oligonucleotide primers and a fluorescently labelled probe to specifically hybridise with bisulfite modified DNA contained as part of the human ACTB gene, regardless of DNA methylation patterns of this gene (Positive control 1), a qRT-PCR assay for the KLK3 gene (Positive control 2) to control for the presence of prostate-derived nucleic acids in the bio-specimen, and a gBlock® synthetic gene fragments for construction of standard curves (SEQ ID NO. 19, 20 or 21), necessary for quantification of methylation levels at individual DNA sequences contained within the panel.

As indicated above, the methods, assays and kits of the invention employ biomarkers (methylated regulatory DNA sequences of specific genes or oligonucleotides specific to those regulatory DNA sequences of those genes) as a means of assessing the risk of an aggressive or metastatic prostate cancer in an individual. In one preferred embodiment of the invention, the methods, assays, and kits may be employed as a clinical screening tool to assist in the identification of individuals with an aggressive form of or a high risk metastatic prostate cancer, especially symptomatic individuals, who should be subjected to more invasive investigations, such as a prostate biopsy. In this regard, it should be noted that many patients who present with symptoms of prostate cancer (*i.e.* the need to urinate frequently, difficulty in starting urination, weak or interrupted flow of urine, painful/burning urination; blood in the urine *etc.*) can turn out to be negative for prostate cancer, yet still have to undergo a prostate biopsy to reach that diagnosis. In this regard, the present invention provides a useful clinical decision making tool which can assist a clinician in identifying those symptomatic patients that are most at risk of having the cancer, thereby potentially reducing the numbers of patients who have to undergo a prostate biopsy needlessly.

Thus, in one embodiment, there is a method of determining prostate cancer status in an individual, the method comprising a step of assaying a biological sample from the individual for a combination of methylated DNA regulatory sequences selected from SEQUENCE ID NO's: 1 to 16, which are the DNA regulatory sequences *GSTP1, SFRP2, IGFBP3, IGFBP7, APC, PTGS-2, LXN, MAGPIE-1B, DNAH10, ZMIZ1, CENPV, OR2L13, MTMR8, F3, CDH8* and *GALNTL6,* respectively, the combination of methylated DNA regulatory sequences being chosen such that detection of at least one or more of the methylated DNA regulatory sequences in the individual correlates to at least a 50% risk of the individual being positive for aggressive or high risk metastatic prostate cancer. Typically, the combination of methylated DNA regulatory sequences is chosen such that detection of at least one or more of the methylated DNA regulatory sequences in the individual correlates to at least a 60% risk of the individual being positive for aggressive or high risk metastatic prostate cancer. Suitably, the combination of methylated DNA regulatory sequences is chosen such that detection of one or more of the methylated DNA regulatory sequences in the individual correlates to at least a 70% risk of the individual being positive for aggressive or high risk metastatic prostate cancer. Ideally, the combination of methylated DNA regulatory sequences is chosen such that detection of one or more of the methylated DNA regulatory sequences in the individual correlates to at least an 80% risk of the individual being positive for aggressive or high risk metastatic prostate cancer. Preferably, the detection of one or more methylated DNA regulatory sequences are combined with PSA.

Typically, the combination will comprise at least one methylated DNA regulatory sequences, at least two methylated DNA regulatory sequences, at least three methylated DNA regulatory sequences, preferably at least four methylated DNA regulatory sequences, more preferable would be at least five methylated DNA regulatory sequences, more preferably still at least six methylated DNA regulatory sequences, and ideally between at least seven, eight, nine, ten, eleven, twelve, thirteen, fourteen, fifteen, or sixteen methylated DNA regulatory sequences. Preferably, the at least one, two, three, four, five, six, seven, eight, nine, ten, eleven, twelve, thirteen, fourteen, fifteen, or sixteen methylated DNA regulatory sequences selected are combined with PSA. Preferably, the methylated DNA regulatory sequences are selected from SEQUENCE ID NO's: 1 to 16, or SEQ ID NO's 1 to 12 and 14.

Typically, the biological fluid is urine, or a derivative of urine following centrifugation or filtration, such as urine cell sediment.

In one aspect of the invention, there is provided, as set out in the appended claims, a method of determining the risk of aggressive prostate cancer in an individual diagnosed with prostate cancer, the method comprising a step of assaying a biological sample obtained from the individual for the presence of at last four methylated regulatory DNA sequence selected from group comprising: SEQ ID No's 1 to 6 and calculating a normalized index of methylation (NIM) score and correlating the presence or absence of the methylated regulatory DNA sequence and the NIM score with aggressive prostate cancer.

In one embodiment, detection of least four, five or six methylated regulatory DNA sequence from the group comprising SEQ ID NOs 1 to 6, correlates with the presence of aggressive prostate cancer.

Preferably, detection of six methylated regulatory DNA sequences as defined by SEQ ID NOs 1 to 6, or variants thereof, and having a sensitivity of at least 80% correlates with the presence of an aggressive prostate cancer.

In one embodiment, the method further comprises detecting the presence of PSA in the biological sample.

In one embodiment, the sample is urine or a urine derivative from the individual.

In one embodiment, there is provided a method of identifying an aggressive prostate cancer in an individual, the method comprising the step of assaying a biological sample obtained from the individual for the presence of a methylated regulatory DNA sequence selected from group comprising: SEQ ID No's 1 to 6 and at least one sequence selected from SEQ ID NOs 17 and 18, and calculating a normalized index of methylation (NIM) score, wherein detection of at least four of the methylated regulatory DNA sequences from SEQ ID NOs 1 to 6 and one sequence from SEQ ID NOs 17 and 18 in a sample and the calculated NIM score is indicative of the presence of an aggressive prostate cancer, and wherein the sensitivity of the assay for detecting the at least four methylated regulatory DNA sequences from SEQ ID NOs 1 to 6 is at least 80%.

In one embodiment, detecting at least six methylated DNA regulatory sequences as defined by SEQ ID NOs 1 to 12 and 14 correlates with the presence of an aggressive prostate cancer, at high-risk of metastasising. Preferably, detecting at least six methylated DNA regulatory sequences as defined by SEQ ID NOs 1 to 12 and 14, and having a sensitivity of at least 80%, correlates with the presence of an aggressive prostate cancer.

In one embodiment, the at least one sequence selected from SEQ ID NOs 17 and 18 is SEQ ID NO: 18, prostate-specific antigen (PSA).

Preferably, detection of at least six methylated DNA regulatory sequences as defined by SEQ ID NOs 1 to 6 and having a sensitivity of at least 80% correlates with the presence of an aggressive prostate cancer.

In one embodiment, there is provided a kit for detecting the presence of prostate cancer in a sample from an individual, the kit comprising a control oligonucleotide as defined by SEQ ID NO 19, 20 or 21, or a variant thereof, and a set of oligonucleotides for detecting SEQ ID NOs 1 to 6 wherein detection of at least four sequences from SEQ ID NOs 1 to 6 in the sample is indicative of the presence of prostate cancer, and wherein the sensitivity of the assay for detecting the at least four sequences from SEQ ID NOs 1 to 6 is at least 80%.

In one embodiment, the prostate cancer is an aggressive prostate cancer.

In one embodiment, the kit further comprises an oligonucleotide for detecting the presence of PSA.

In one embodiment, the set of oligonucleotides is defined by SEQ ID NOs. 22 to 72.

In one embodiment, the kit further comprises a support having at least one oligonucleotide selected from group SEQ ID No's 1 to 16 anchored thereon.

In one embodiment, the kit comprises a support having three, four, five, six, seven, eight, nine, ten, eleven, twelve, thirteen, fourteen, fifteen, or sixteen oligonucleotides anchored thereon selected from SEQ ID NOs. 1 to 16.

In one embodiment, the kit comprises a support having three, four, five, six, seven, eight, nine, ten, eleven, twelve or thirteen oligonucleotides anchored thereon selected from SEQ ID NOs. 1 to 12 and 14.

In one embodiment, there is a method of determining the risk of aggressive prostate cancer in an individual, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with aggressive prostate cancer.

In one embodiment, detection of least three methylated regulatory DNA sequence from the group comprising SEQ ID NOs 1 to 16, or variants thereof, correlates with the presence of an aggressive prostate cancer.

Preferably, detection of SEQ ID NO. 1 and SEQ ID NO. 3, or variants thereof, together with at least one further sequence selected from SEQ ID NOs. 2, 4, 5 and 6, or variants thereof, correlates with the presence of an aggressive prostate cancer.

In one embodiment, detection of least four, five or six methylated regulatory DNA sequence from the group comprising SEQ ID NOs 1 to 16, or variants thereof, correlates with the presence of aggressive prostate cancer.

Preferably, detection of six methylated regulatory DNA sequences as defined by SEQ ID NOs 1 to 6, or variants thereof, and having a sensitivity of at least 80% correlates with the presence of an aggressive prostate cancer.

In one embodiment, there is a method of detecting the presence of prostate cancer in an individual, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with a reference abundance marker indicative of prostate cancer.

In one embodiment, there is a method of determining whether an individual requires an invasive trans-rectal biopsy to confirm diagnosis of prostate cancer by histological review of a biopsy specimen, the method comprising a step of assaying a biological sample obtained from the individual for the presence of at last one methylated regulatory DNA sequence selected from group comprising: SEQ ID No's 1 to 16, or variants thereof, and in which the presence of the methylated regulatory DNA sequence determines that the individual requires an invasive trans-rectal biopsy to confirm diagnosis of prostate cancer by histological review of a biopsy specimen.

In one embodiment, the biological sample is urine or a urine derivative from the individual.

In one embodiment, the method comprises detection of six methylated regulatory DNA sequences as defined by SEQ ID NOs 1 to 12 and 14 correlates with the presence of an aggressive prostate cancer. Preferably, detection of six methylated regulatory DNA sequences as defined by SEQ ID NOs 1 to 12 and 14, and having a sensitivity of at least 80%, correlates with the presence of an aggressive prostate cancer.

In one embodiment, the assay comprises detection of six methylated regulatory DNA sequences as defined by SEQ ID NOs 1 to 12 and 14, and having a sensitivity of at least 80%, correlates with the presence of an aggressive prostate cancer.

In one embodiment, the methods and assays described above further comprise the step of applying a NIM threshold of 0.73 for discriminating biopsy positive from biopsy negative samples. In one embodiment, the methods and assays described above further comprise the step of applying a NIM threshold of 1.25 for detecting high-risk or high grade (aggressive) prostate cancer from the samples being assayed. The NIM equation normalises for the amount if input bisulfite modified DNA present in the sample and calculates the proportion of the target sequence which is methylated relative to a 100% fully methylated DNA sequence.

In this specification, the term "biological sample" or "biological fluid" may be a sample obtained from an individual such as, for example, urine or urine cell-sediment, blood or a prostate tissue sample from a biopsy or a radical prostatectomy. In many cases, the individual will be a person suspected of having prostate cancer, or pre-disposed to developing prostate cancer as determined by other phenotypic, genotypic or hereditary traits.

In this specification, the term "prostate cancer status" when used with reference to an individual primarily refers to the risk of the individual having the cancer. Depending on the number of biomarkers detected in the individual, the assay and methods of the invention will assist a clinician is determining the risk that the individual is positive for prostate cancer. Thus, in one embodiment, the methods, assays and kits of the invention provide a means for screening male patients to identify those patients that should undergo further investigative procedures, such as a biopsy. However, the term also encompasses prognostic evaluation of the cancer, identification of predisposition to developing the cancer, staging of the cancer, and evaluation or monitoring of the progress of the cancer, in the individual. The latter evaluation is typically employed as a means of monitoring the effectiveness of a treatment for the cancer.

A "variant" of one of SEQUENCE ID No's 1 to 16 shall be taken to mean at least 70% sequence identity, preferably at least 80% sequence identity, more preferably at least 90% sequence identity, and ideally at least 95%, 96%, 97%, 98% or 99% sequence identity with the native sequence.

mRNA expression of the KLK3 gene (positive control 2 - SEQ ID NO. 18) may be measured by any suitable method including, but not limited to, a Northern Blot or detection by hybridisation to a oligonucleotide probe. A variety of hybridization assays using a variety of technologies for hybridization and detection are available. For example, a TaqMan assay (PE Biosystems, Foster City, CA; See e.g., U.S. Patent Nos. 5,962,233 and 5,538,848,) is utilized. The assay is performed during a PCR reaction. The TaqMan assay exploits the 5'-3' exonuclease activity of the AMPLITAQ GOLD DNA polymerase. A probe consisting of an oligonucleotide with a 5 '-reporter dye (e.g., a fluorescent dye) and a 3 '-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorometer.

DNA methylation may be measured by any suitable method, such as quantitative methylation specific PCR (PMID: 10734209).

In other embodiments, reverse-transcriptase PCR (RT-PCR) is used to detect the expression of RNA where RNA is enzymatically converted to complementary DNA or "cDNA" using a reverse transcriptase enzyme. The cDNA is then used as a template for a PCR reaction. PCR products can be detected by any suitable method, including but not limited to, gel electrophoresis and staining with a DNA specific stain or hybridization to a labelled probe. In some embodiments, the quantitative reverse transcriptase PCR with standardized mixtures of competitive templates method described in U.S. Patents 5,639,606, 5,643,765, and 5,876,978 is utilized.

In the specification, the term "high-risk prostate cancer", "high-risk disease" or "aggressive prostate cancer" or "metastatic prostate cancer" should be understood mean a prostate cancer that is categorised by the D'Amico Risk Stratification criteria. The D'Amico criteria are used to define low, intermediate and high-risk prostate cancer. For example, (i) Low risk: having a PSA less than or equal to 10, a Gleason score less than or equal to 6, or are in clinical stage T1-2a; (ii) Intermediate risk: having a PSA between 10 and 20, a Gleason score of 7, or are in clinical stage T2b; and (iii) High-risk: having a PSA more than 20, a Gleason score equal or larger than 8, or are in clinical stage T2c-3a. The terms high-risk, aggressive and metastatic can be used interchangeably. The terms high-risk and aggressive describe a cancer of high tumour grade (according to the Gleason scale, >=8) and a highly likelihood of metastasising.

In the specification, the term "gBlock®" should be understood to mean a doublestranded DNA molecule of 125-2000 bp in length. In this instance, the gBlock® is defined by SEQ ID NO. 19 and contains sequences for (A) an internal control ACTB, and the genes (B) GSTP1 (C), SFRP2, (D) IGFBP3, (E) IGFBP7, (F) APC and (G) PTGS2. The gBock® defined by SEQ ID NO: 20 was designed to contain the nucleotide sequences of bisulfite converted fully methylated internal control sequence *(ACTB)* and seven DNA regulatory sequences (*LXN, MAGPIE-1B, DNAH10, ZMIZ1, CENPV* and *OR2L13).* The gBlock® defined by SEQ ID NO. 21 was designed to contain the nucleotide sequences of bisulfite converted fully methylated internal control sequence *(ACTB)* and four DNA regulatory sequences (*MTMR8, F3, CDH8 and GALNTL6).*

Some of the uses of the invention include:

- To test for the presence of prostate cancer.
- Use as a novel screening test for any male at risk of having prostate cancer.
- Use as a non-invasive test using urine to determine which male requires an invasive trans-rectal biopsy to confirm a diagnosis of prostate cancer by histological review of a biopsy specimen.
- The test can be carried out on any biological sample that harbours prostate DNA, including blood plasma/serum, prostate tissue and metastatic lesions, either visceral or bone.

Some of the advantages of the invention is to:

- Reduce/eliminate unnecessary invasive biopsies in men who don't need them;
- Identify which men require a trans-rectal prostate biopsy
- Alleviate over-treatment of low-risk disease;
- Inform the clinician about the molecular biology of the disease; and
- Aid risk-stratification for selection of subsequent treatments/active surveillance.

## Brief Description of the Figures

The invention will be more clearly understood from the following description of an embodiment thereof, given by way of example only, with reference to the accompanying drawings, in which:-

**Figure 1** illustrates Feasibility Study data (n=156). Results on a panel of 156 pre-biopsy urine samples, shown by biopsy outcome. Positive-biopsy men are further categorised into low (LR), intermediate (IR) and high (HR) risk groups using the CAPRA score. Each row represents a gene, each column represents a patient. Methylation is measured as a continuous variable from 0-1000 (Normalised Index of Methylation, NIM). Black squares indicate high methylation with a normalised index of methylation (NIM)>1, white squares indicate NIM=0 and shades of grey indicate intermediate NIM.

**Figure 2** illustrates results reduced into categorical results: men with methylation of at least one gene and men with an NIM>1 in any one gene.

**Figure 3** illustrates ROC curves for (A) PSA alone, (B)-(C) using the invention, and (D) the test of the invention and PSA>4 ng/ml, which achieve an AUC of 0.54, 0.87 (average) and 0.96, respectively.

**Figure 4** illustrates that the positive predictive value of invention which indicates its utility for reducing number of unnecessary biopsies by selectively detecting aggressive PCa.

**Figure 5** illustrates standard curves constructed over a 6-log range using 5 independent qMSP measurements of a gBlock® fragment containing sequences for (A) internal control, (B) GSTP1 (C), SFRP2, (D) IGFBP3, (E) IGFBP7, (F) APC and (G) PTGS2 (SEQ ID NO. 19). Each qMSP assay has a slope of -3.3 (+/- 10%) and an $R^2$ >0.99, indicating a PCR efficiency close to 100%.

**Figure 6** illustrates graphs showing quantitative methylation-specific PCR data for SEQ ID NOs 7-16 in a radical prostatectomy cohort reveal quantitatively higher levels of DNA methylation in aggressive tumours (PC-A) compared with significant (PC-S) and indolent (PC-I) tumours and benign tissue.

**Figure 7** illustrates graphs showing descriptive statistics of the study cohort used in epiCaPture analysis with an increased cohort size from 156 men to 283 men. The data presented includes the original cohort of patients. A) Age, B) PSA levels of men (Whiskers indicate the minimum and maximum levels and the mean is indicated by a horizontal line) and statistics of the biopsy-positive cohort used in epiCaPture analysis. Patients were stratified according to C) D'Amico risk group and D) tumour grade (Gleason score, GS).

**Figure 8** illustrates a heat map of epiCaPture NIM scores for increased cohort of 283 men. Positive-biopsy men are further categorised by Gleason score. Each row represents a gene, each column represents a patient. Methylation is measured as a continuous variable from 0-1000 (Normalised Index of Methylation, NIM). White squares represents absence of methylation (NIM=0), with increasing shades of grey and black representing quantitatively higher amounts of methylation for a gene.

**Figure 9** is a graph illustrating performance of epiCaPture versus predicate at non-invasive detection of high-risk prostate cancer.

**Figure 10** are graphs illustrating *GSTP1* methylation as detected in three independent cohorts of radical prostatectomy samples (A) a cohort of 44 men studied by Infinium HM450k methylation Beadchip (Table 11); (B) cohort of 125 men studied by quantitative PCR (Table 12); and (C) cohort of 178 men extracted from The Cancer Genome Atlas, for whom Infinium HM450k methylation Beadchip are publically available (Table 13). Panel (D) shows the methylation values detected in the urine samples from men undergoing trus-biopsy (n=283). In all cohorts, significantly higher levels of methylation are detected in the high-risk and high grade disease.

**Figure 11** are graphs illustrating *SFRP2* methylation as detected in A) prostate tissues and B) urine samples from men undergoing TRUS-biopsy. In both cohorts, significantly higher levels of methylation are detected in the high-risk and high grade disease.

**Figure 12** are graphs illustrating *IGFBP3* methylation as detected in (A) prostate tissues from men undergoing radical prostatectomy (Perry et al, British Journal of Cancer, 2007). Abbreviations: HGPIN: high grade prostatic intraepithelial neoplasia, HB: histologically benign and BPH: benign prostatic hyperplasia. (B) urine samples from men undergoing trus-biopsy, n=283. In both prostate tissue and urine, significantly higher levels of methylation are detected in the high-risk and high grade disease patients.

**Figure 13** are graphs illustrating *IGFBP7* methylation as detected in (A) prostate tissues from men undergoing radical prostatectomy (Sullivan *et al*, Journal of Urology, 2012). Abbreviations: HGPIN: high grade prostatic intraepithelial neoplasia, HB: histologically benign and BPH: benign prostatic hyperplasia. (B) urine samples from men undergoing trus-biopsy, n=283. In both prostate tissue and urine, significantly higher levels of methylation are detected in the high-risk and high grade disease patients.

**Figure 14** are graphs illustrating *APC* methylation as detected in (A) prostate tissues from men undergoing radical prostatectomy (Murphy et al, Epigenetic Diagnosis and Therapy, 2015). Abbreviations: HGPIN: high grade prostatic intraepithelial neoplasia, TA: tumour associated benign. (B) urine samples from men undergoing trus-biopsy, n=283. In both prostate tissue and urine, significantly higher levels of methylation are detected in the high-risk and high grade disease patients.

**Figure 15** are graphs illustrating supporting data for *LXN.* DNA methylation was measured in three independent cohorts of radical prostatectomy samples (Tables 11 to 13), and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 16** are graphs illustrating supporting data for *MAGPIE-1B.* DNA methylation was measured in three independent cohorts of radical prostatectomy samples (Tables 11 to 13), and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 17** are graphs illustrating supporting data for *DNAH10.* DNA methylation was measured in three independent cohorts of radical prostatectomy samples (Tables 11 to 13), and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 18** are graphs illustrating supporting data for *ZMIZ1.* DNA methylation was measured in three independent cohorts of radical prostatectomy samples, and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 19** are graphs illustrating supporting data for *CENPV.* DNA methylation was measured in three independent cohorts of radical prostatectomy samples, and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 20** are graphs illustrating supporting data for *OR2L13.* DNA methylation was measured in three independent cohorts of radical prostatectomy samples, and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A and C) and quantitative PCR (B). For simplicity, significance values are only shown for comparisons with aggressive prostate cancer.

**Figure 21** are graphs illustrating supporting data for (A-C) *F3.* DNA methylation was measured in three independent cohorts of radical prostatectomy samples, and in each case is significantly higher in high-risk/aggressive prostate cancer compared benign prostate tissue and/or low-risk or indolent prostate cancer. Two methodologies were used to quantitatively measure DNA methylation: Infinium HM450K BeadChip (A, and C) and quantitative PCR (B). For simplicity, significance values are only shown for comparisons with aggressive (high-risk) prostate cancer

## Detailed Description

*Materials and Methods*

Statistical methods:

Logistic regression is a standard method for modelling the relationship between a binary variable, in this case high-risk versus low-risk prostate cancer, and a set of continuous or categorical variables. For this analysis, the variables used for prediction consist of gene methylation values, as well as patient variables age and PSA. Mathematically this relationship is expressed as

$$\log\left(\frac{p_i}{1-p_i}\right) = \beta_1 X_{1i} + \beta_2 X_{2i} + \cdots + \beta_m X_{mi} \qquad (1)$$

where $p_i$ is the probability that the nth patient is high-risk based on their methylation profile and clinical characteristics, which are represented by the $X_{mi}$'s. The $\beta_m$ coefficients give the effect that each incremental change in methylation, age or PSA has on the log-odds of the patient being high risk of prostate metastasis.

Due to the cost of collection of biomarkers, and the general principle that simpler models lead to more robust predictions, one aim of the analysis is to choose the smallest number of predictor variables, the $X_m$'s, which will yield the best performing prediction model.

A LASSO[1] logistic regression (discussed below), along with a standard logistic regression incorporating six genes were fit to the data. Logistic regression models for each of the separate genes were also fitted for comparison. All models were trained using repeated 5-fold cross-validation with bootstrap resampling. The optimal cut-off value for prediction was then chosen using the entire data set.

A common problem with building a prediction model on the entire dataset is that the model will tend to over fit the current data set and will then underperform when new data is predicted from the model. In general, more complex models will tend to adapt to the training data and will not generalise as well to new data. Therefore sparser models are preferred.

In an ideal case a model is fitted to some training data and then its performance is estimated on an independent test set. A model can be selected by choosing the model that performs best on the test dataset which consists of new unseen observations. For small datasets, a single split of the data into testing and training sets is often not possible.

Cross-validation is a method for performing multiple random training-test splits of a dataset. The process is as follows:

1. Split the data into K equally sized portions (K is usually chosen to be between 5 and 10);
2. Leave aside one of the K portions and train the model(s) on the all of the remaining K-1 portions together;
3. Test the performance of the model(s) on the portion of the data that was set aside in step 2; and
4. Repeat iteratively leaving out each of the K portions in succession.

This method gives a more accurate assessment of the out-of-sample prediction performance of the models than simply fitting the model to the entire dataset. To further account for uncertainty in the cross-validation process, the dataset is bootstrapped, *i.e.* resample the entire dataset with replacement, and perform cross-validation on each bootstrap iteration. For the analysis here, K= 5 and 2000 bootstrap iterations were used.

All of the data is used in the model-building and assessment stages. The model building process used aims to mitigate against any optimistic bias. Due to the relatively small number of high-risk cases, splitting the data is not an efficient option. Ideally a test or hold-out set of data which were not used in the training step should be used on which to test performance. This should be done at a later stage using an independently collected test data set.

The LASSO is a penalised regression method for building prediction models which mitigates against over-fitting on a data set. The LASSO is a method for both model selection and estimation. In standard logistic regression, the model parameters are fitted using iterative maximum likelihood. This estimates the parameters which fit the data the best, and as such can over-fit to the training data. Penalised regression methods add a penalty term to the estimation equation which penalises large values of the coefficients. This is a form of shrinkage which can yield more robust results. In the case of LASSO, the penalty term shrinks some coefficients to zero, acting as a form of variable selection.

The strength of penalisation is determined by a parameter, $\lambda$. The optimal lambda value is found by running a further cross-validation iteration within each iteration of the outer cross-validation loop.

For the final assessment of the LASSO model, models chosen in the resampling and cross-validation iterations were searched and selected as the final model, the most frequently occurring model. The performance assessments and model parameters were then based on the iterations where this model occurred. Regression coefficients were then obtained by averaging over these iterations.

<u>Samples</u>

The epiCaPture test (method described herein) is performed on urine or urine cell-sediment. The urine cell-sediment is obtained by centrifugation (10,000xg for 10 minutes) of a first-void urine sample (up to 50ml) following a digital rectal examination (DRE). The DRE consists of three strokes per lobe of the prostate gland. Enough pressure is applied to the prostate to depress the surface approximately 1cm, from the base toe the apex and from the lateral to the median line for each lobe. Total nucleic acid is extracted from the cell sediment using a standard silica-membrane based extraction protocol (using a Qiagen total nucleic acid isolation kit, or similar commercially available product). Purified DNA (100ng) is subject to bisulfite conversion (using a Qiagen epitect kit, or similar commercially available product).

Expression of the *KLK3* gene is measured by qRT-PCR using a commercially available primer and probe assay (Assay ID Hs.PT.58.38546086 available from Integrated DNA Technologies). Positive expression of the *KLK3* gene relative to a housekeeper gene (ACTB) indicates the presence of prostate cells in the urine sediment and validity of the urine sample for epiCaPture analysis.

A 648 bp synthetic gBlock® DNA sequence (IDT - SEQ ID NO: 19) was designed to contain the nucleotide sequences of bisulfite converted fully methylated internal control sequence *(ACTB)* and six DNA regulatory sequences (*GSTP1, SFRP2, IGFBP3, IGFBP7, APC, PTGS2):*

AAAGGTGGAGGTAGTTAGGGTTTATTTGTATATTGATTTGAGATTAGTTGAATAAA
AGTGTATATTTTAAAAATGAGGTTAAGTGTGATTTTGTGGTGTGGAAAGTTGCGCG
GCGATTTCGGGGATTTTAGGGCGTTTTTTTGCGGTCGACGTTCGGGGTGTAGCGGTC
GTCGGGGTTGGGGTCGGCGGGAGTTCGAAAAGTTTTTCGGAGTTGCGCGCGGGTTT
GTAGCGTTTCGTTCGCGTTGTTTTTTCGGTGTTTCGTTTTTTCGCGTTTTAGTCGTCG
GTTGTTAGTTTTTCGGGGTTTCGAGTCGTATTTAGCGAAGAGAGCAAATTTTTTCGA
TATCGGTTCGTCGTAGGGAGATTTTATTTCGAGAGCGGAAGGGGTAAGGGCGGCGG
GGTTAAGGAGATCAAAAAGCGGGCGTGAGATCGAGCGTTTATGGGTCGGTTACGTC
GGGTGTTCGTTTATTTTTCGACGTTAGTAGGAGCGCGAAATTATATGTCGGTTACGT
GCGTTTATATTTAGTTAATCGGCGGGTTTTCGACGGGAATGGGGAGCGTTTTGGTTC
AAACGGAAGCGTTCGGGTAAAGATTGCGAAGAAGAAAAGATATTTGGCGGAAATT
TGTGCGTTTGGGGCGGTGGAATTCAAA

An 885 bp synthetic gBlock® DNA sequence (IDT - SEQ ID NO: 20) was designed to contain the nucleotide sequences of bisulfite converted fully methylated internal control sequence *(ACTB)* and six DNA regulatory sequences (*LXN, MAGPIE-1B, DNAH10, ZMIZ1, CENPV and OR2L13)*:

AAAGGTGGAGGTAGTTAGGGTTTATTTGTATATTGATTTGAGATTAGTTGAATAAA
AGTGTATATTTTAAAAATGAGGTTAAGTGTGATTTTGTGGTGTGGAAATGGGTTATT
TTGGTTTAACGGGATTAGTAGTAGAGCGTCGTTCGTTTTGTTTGTTGTTGGGTTCGG
TTGTCGAGGCGGAAAAGTCGTAAGAAATTTGTTTTTGGTTTTTGTAGGCGTTTGGGT
TGTTTTATTTGAGAGTTGCGTAGGGCGGTTTGGCGGTGGTTGTTGTTTATATAATTC
GAAACGTCGAGGTGTTGTGATTTTCGTTTCGTTAATTTTTTAGTTTTAGTTTTATTTG
TAAGGTGGGCGGGTTGTTTGTAAATCGGTCGGCGTGGGGTGGGGTCGTATTTTCGG
TTGTAGCGGTTAAAGGGTTTCGTCGTCGTTTTCGTTCGGAGGTTGGAGTGTTGTTCG
TCGGGTCGTGCGTTCGTTCGGTAGCGGCGTGTATTAGTATTATAAACGTTGGGACGT
ATTTGTTTGTTTAGTTTCGTAGGTAGAGGGGTTGCGTTTGGGTTTACGTTCGCGATA
TTTAGAATTTATTCGTATTTGCGAAGGCGAAAATACGTTTTTGTCGGTCGTTTAGTTT
TTTGTAGGTGTAAGGGCGGATGTTTTAGCGATTACGGGAGTCGGGTTGGGGAGGTT
GGTGGGGGGCGGGGGGAGTTTTAAATTTAGGTTCGTTTAGTTATAGGCGTTTAGGTT
TAGTCGGAAATTGTCGGAGGACGCGTTGTTGCGAGATTAGTCGCGGCGTTTTTGGT

AGTAGTGGGCGTGTTTGCGGGTTTAGGAGGGTTTTTTTTCGCGATCGTCGATTACG
ATGAGAGCGTGAAGATTTTTTCGAAAGGAAAA

A 643 bp synthetic gBlock® DNA sequence (IDT - SEQ ID NO: 21) was designed to contain the nucleotide sequences of bisulfite converted fully methylated internal control sequence *(ACTB)* and four DNA regulatory sequences (*MTMR8, F3, CDH8 and GALNTL6):*

AAAGGGCGGTTTTGGTTTAAATTTTCGTTATTTGATTTTCGGCGAAAGTTTTTATCGC
GATATTTTGATCGTAGTCGTTTTCGTTAAAAAAGGTGGAGGTAGTTAGGGTTTATTT
GTATATTGATTTGAGATTAGTTGAATAAAAGTGTATATTTTAAAAATGAGGTTAAGT
GTGATTTTGTGGTGTGGAAATTCGGTTTATTACGGCGGTTATTTTTCGGGTAGTGAC
GACGATCGAGACGGTGAGGGCGGTTATCGTTGGGGAGGGAGGTTCGGGTTTAGGTT
TGGAAGTAAAACGTGTGGTTTGTTATATCGTCGGTTGTATTGGATTAGGATTATTTT
TTATGAAGGTTTGTTTTGTTAGTACGTAGTAGGTTTTAGTTTTTACGTCGTTTCGAAT
ATTTCGTAGAAATACGGGGTATGTATAACGAAACGGATTTCGTTTATGTTTTTCGGA
GTTATAGGGTTTGGTGCGGAGACGTAGGGCGGGCGCGTTGGGTTTTGGGTGTTCGT
AATTTAAAGTGTAGTTGGTGTAAACGGGTCGTTTTTATTTTTCGTAGTCGTCGCGTTT
CGGTTCGTCGCGTTTTCGTCGGTATTATGGAGGAATTTTTGGGATAATCGTTTGTAG
TCGGCGATTGGGAAA

**Table 1**: The gBlock® (SEQ ID NO. 19) was used to construct to-fold serial dilutions over a 6-log template concentration range to determine the dynamic range and PCR efficiency of each epiCaPture assay:

| Standard | Copy number | Vol. of gBlock (μl) | Vol. of molecular grade $H_2O$ (μl) | DNA conc. (pg/μl) |
|---|---|---|---|---|
| 1 | 1,000,000 | 10 (WS) | 173.8 | 0.544 |
| 2 | 100,000 | 10 (1) | 90 | 0.0544 |
| 3 | 10,000 | 10 (2) | 90 | 0.00544 |
| 4 | 1,000 | 10 (3) | 90 | 0.000544 |
| 5 | 100 | 10 (4) | 90 | 0.0000544 |
| 6 | 10 | 10 (5) | 90 | 0.00000544 |

Quantitative methylation specific PCR (qMSP) is performed, as previously described [3-5]. The PCR efficiency of each of the assays (internal control and 6 targets) was rigorously evaluated by performing 5 independent replicates (each with 3 technical replicates) over a 6-log template concentration range (Figure 5). Bisulfite treated DNA is amplified in parallel TaqMan® PCR reactions performed with oligonucleotides specific for each of the target methylated DNA regulatory sequences (SEQ ID NOs 1 to 6) and the endogenous control gene *ACTB* (SEQ ID NO: 17). Samples are considered positively amplified when a comparative threshold cycle ($C_T$) of <50 was detected in at least two out of three replicates. A normalized index of methylation (NIM) was calculated, as previously described[6], to determine the ratio of the normalized amount of methylated target to the normalized amount of *ACTB* in any given sample, by applying the formula:

$$\text{NIM} = [(TARGET_{\text{sample}}/TARGET_{\text{MC}})/(ACTB_{\text{ sample}}/ACTB_{\text{ MC}})] \times 1000 \qquad (2)$$

Where $TARGET_{\text{sample}}$ is the quantity of fully methylated copies of each of the sequences being sampled in any individual sample, $\text{TARGET}_{\text{MC}}$ is the quantity of fully methylated copies of each of the sequences being sampled in a commercially available fully methylated bisulfite converted human DNA sample (Qiagen product number 59655), *ACTB* sample is the quantity of bisulfite modified templates in any individual sample and $ACTB_{\text{MC}}$ is the quantity of bisulfite modified templates in the universally methylated control DNA.

All genomic sequences for *GSTP1*, *SFRP2, IGFBP3, IGFBP7, APC, PTGS-2, LXN, MAGPIE-1B, DNAH10, ZMIZ1, CENPV, OR2L13, and F3* were obtained from the UCSC Human Genome Browser (http://genome-euro.ucsc.edu).

*Results*

Results from the study on 156 men (Figures 3(A) to 3(D)) demonstrate that the invention can non-invasively discriminate high-risk (metastatic) PCa from low-risk (less chance of metastasis) disease and benign enlargement of the prostate (AUC=0.86). In this cohort, a high score (NIM>1) had 100% specificity for PCa, and greatly outperformed PSA, which yielded a PCa-specificity of only 11.63%.

**Table 2** is data relating to the cohort of 156 TRUS-biopsy patients. The exclusion criteria for the cohort were (1) metastases on an MRI and/or a bone scan and (2) not post-DRE.

| | **Biopsy positive** | **Biopsy negative** | **P value** |
|---|---|---|---|
| N | 108 | 48 | |
| **Age (years)** | | | |
| Mean | 69.95 | 64.85 | 0.008 |
| Median | 69.50 | 66 | |
| Range | 53-85 | 42-82 | |
| **PSA (ng/ml)** | | | |
| Mean | 16.53 | 7.06 | <0.0001 |

(continued)

| PSA (ng/ml) | | | |
|---|---|---|---|
| Median | 10 | 6.10 | |
| Range | 4.1-95.9 | 0.2-30.30 | |
| **Risk group** | | | |
| LR | 14 | | |
| IR | 58 | | |
| HR | 36 | | |

Urinary detection of prostate cancer

For non-invasively distinguishing men who have prostate cancer form those who do not (or more strictly speaking, men with a positive biopsy from men with a negative biopsy), the best combination of biomarkers is *GSTP1* used in conjunction with *PSA*. This is calculated using a LASSO model (Table 3, Figure 3A). This achieved a positive predictive value (PPV) of 92%, with a negative predictive value (NPV) of 52%, with a sensitivity and specificity for prostate cancer of 81% and 77%, respectively. The combination of six methylated DNA regulatory sequences (as defined by SEQ ID NOs 1 to 6) in the method described herein also performs well at non-invasive detecting prostate cancer: PPV = 92%, NPV = 51%, sensitivity = 60% and specificity = 89%.

| **Table 3: Biopsy positive versus biopsy negative** | | | | | |
|---|---|---|---|---|---|
| SEQ (Gene) | AUC | Sensitivity | Specificity | PPV | NPV |
| **1 (GSTP1)** | 0.72 (0.66 - 0.77) | 0.44 | 0.98 | 0.98 | 0.45 |
| **2 (SFRP2)** | 0.66 (0.60 - 0.72) | 0.37 | 0.94 | 0.93 | 0.41 |
| **3 (IGFBP3)** | 0.65 (0.58 - 0.73) | 0.41 | 0.89 | 0.89 | 0.41 |
| **4 (IGFBP7)** | 0.64 (0.58 - 0.70) | 0.29 | 1 | 1 | 0.40 |
| **5 (APC)** | 0.68 (0.60 - 0.75) | 0.49 | 0.87 | 0.89 | 0.44 |
| **6 (PTGS2)** | 0.63 (0.55 - 0.72) | 0.34 | 0.98 | 0.97 | 0.41 |
| **PSA** | 0.76 (0.67 - 0.84) | 0.88 | 0.51 | 0.80 | 0.67 |
| **All** | 0.75 (0.68 - 0.81) | 0.60 | 0.89 | 0.92 | 0.51 |
| **LASSO (1 (GSTP1) + PSA)** | 0.83 (0.74 - 0.89) | 0.81 | 0.77 | 0.92 | 0.50 |

Urinary detection of high-risk prostate cancer

However, as stated already, the dilemma for prostate cancer detection is not in the ability to detect the entire spectrum of disease, for which PSA is already adequately doing, but to specifically detect high-risk disease with high likelihood to metastasise. For predicting high-risk prostate cancer according to D'Amico criteria[2], the LASSO, which is the selection method used here, determines that GSTP1 and IGFBP3 are the best fit (Table 4, Figure 3B). This combination delivers a PPV 56% of and NPV of 94% for high-risk disease, with a sensitivity and specificity both at 82%. The combination of all 6 genes, performs slightly less well, delivering a sensitivity of 52% and a specificity of 92%, for high risk disease. The method described herein (and derivations of it) outperforms current clinical practice (PSA), which in this cohort was found to have a sensitivity of 100% and specificity of only 21% (at the 4ng/ml cut-off) for high-risk disease.

| **Table 4. Detection of high-risk disease** | | | | | |
|---|---|---|---|---|---|
| SEQ (Gene) | AUC | Sensitivity | Specificity | PPV | NPV |
| **1 (GSTP1)** | 0.78 (0.68 - 0.87) | 0.70 | 0.87 | 0.61 | 0.92 |
| **2 (SFRP2)** | 0.77 (0.68 - 0.86) | 0.67 | 0.85 | 0.55 | 0.90 |

(continued)

| Table 4. Detection of high-risk disease | | | | | |
|---|---|---|---|---|---|
| **SEQ (Gene)** | AUC | Sensitivity | Specificity | PPV | NPV |
| **3 (IGFBP3)** | 0.76 (0.66 - 0.86) | 0.61 | 0.82 | 0.49 | 0.88 |
| **4 (IGFBP7)** | 0.77 (0.67 - 0.86) | 0.58 | 0.91 | 0.63 | 0.88 |
| **5 (APC)** | 0.76 (0.66 - 0.86) | 0.67 | 0.84 | 0.54 | 0.90 |
| **6 (PTGS2)** | 0.71 (0.60 - 0.82) | 0.52 | 0.92 | 0.63 | 0.87 |
| **All six** | 0.84 (0.75 - 0.93) | 0.79 | 0.82 | 0.55 | 0.93 |
| **LASSO (1 (GSTP1) + 3 (IGFBP3))** | 0.83 (0.75 - 0.92) | 0.82 | 0.82 | 0.56 | 0.94 |

Urinary detection of high-grade prostate cancer.

The Gleason grading system is the strongest prognostic indicator for prostate cancer. It is a histological grading system based on the glandular pattern of the tumour. A Gleason score is obtained by the addition of the primary and secondary grades. The presence of Gleason grade 4 or higher, or a Gleason score of 7 or higher predicts a poor prognosis.

For predicting tumours with a high Gleason score (>=8), the combination of all 6 biomarkers outlined above outperforms all biomarkers assessed individually (Table 5, Figure 3C), with a PPV of 48%, a NPV of 96% and a sensitivity and specificity of 76% and 87%, respectively. Combining all six markers with PSA gives some improvement again, with a sensitivity and specificity of 86% and 82%.

| Table 5: Detection of high-grade disease | | | | | |
|---|---|---|---|---|---|
| **SEQ (Gene)** | AUC | Sensitivity | Specificity | PPV | NPV |
| **1 (GSTP1)** | 0.74 (0.63 - 0.86) | 0.67 | 0.82 | 0.38 | 0.94 |
| **2 (SFRP2)** | 0.78 (0.67 - 0.89) | 0.67 | 0.88 | 0.48 | 0.94 |
| **3 (IGFBP3)** | 0.78 (0.67 - 0.90) | 0.67 | 0.82 | 0.38 | 0.94 |
| **4 (IGFBP7)** | 0.77 (0.66 - 0.89) | 0.71 | 0.78 | 0.35 | 0.94 |
| **5 (APC)** | 0.75 (0.62 - 0.87) | 0.71 | 0.80 | 0.38 | 0.94 |
| **6 (PTGS2)** | 0.68 (0.54 - 0.83) | 0.57 | 0.89 | 0.46 | 0.93 |
| **PSA** | 0.79 (0.71 - 0.87) | 0.95 | 0.63 | 0.29 | 0.99 |
| **All 6** | 0.83 (0.73 - 0.94) | 0.76 | 0.87 | 0.48 | 0.96 |
| **All 6 + PSA** | 0.86 (0.76 - 0.96) | 0.86 | 0.82 | 0.44 | 0.97 |
| **LASSO (3 (IGFBP3))** | 0.78 (0.67 - 0.90) | 0.67 | 0.82 | 0.38 | 0.94 |

*Supplementary Data*

The cohort size was increased from 156 men to 283 men.

**Table 6** is data relating to the cohort of 283 TRUS-biopsy patients. The exclusion criteria for the cohort were (1) metastases on an MRI and/or a bone scan and (2) not post-DRE.

| | **Biopsy negative** | **Biopsy positive** | ***P* value** |
|---|---|---|---|
| **n (283)** | **135** | **148** | |
| **Age (years)** | | | |
| mean | 64.43 | 68.44 | <0.0001 |
| median | 65 | 68 | <0.0001 |
| range | 42-83 | 47-85 | |

(continued)

| | | | |
|---|---|---|---|
| **PSA (ng/ml)** | | | |
| mean | 6.59 | 11.78 | <0.0001 |
| median | 6.05 | 8.90 | <0.0001 |
| range | 0.2-63.80 | 0.6-144 | |
| **D'Amico Risk Group** | | | |
| LR | | 29 | |
| IR | | 73 | |
| HR | | 48 | |
| **Gleason Score** | | | |
| 6 | | 41 | |
| 7 | | 74 | |
| 8 | | 11 | |
| 9 | | 21 | |
| 10 | | 1 | |

epiCaPture was performed on the cohort of 283 men, consisting of 135 biopsy-positive men and 148 biopsy-negative men. The age and PSA characteristics of the cohort are presented in Table 6. Although the biopsy-positive group were significantly older and had a significantly higher median PSA level (8.90 versus 6.05), there is considerable overlap in the range of ages and PSA levels for both groups (Figure 7A and 7B). Indeed, the mean and median PSA levels for the biopsy-negative group are above the 4ng/ml threshold widely used for indicating need for prostate-biopsy. The biopsy-positive cohort were considered in terms of risk-group stratification (according to the D'Amico criteria), which encompasses tumour grade (Gleason score), PSA level and clinical stage) and tumour grade stratification (Table 6, Figures 7C and 7D).

Each of the 6 gene panel was analysed individually in each patient, and a normalised index of methylation (NIM) score was generated for each gene (Figure 8). Different approaches were studied to determine the best performing method to (1) discriminate biopsy positive from biopsy negative and (2) selectively detect high-risk and high-grade disease. The performance of individual genes versus different combinations was studied using LASSO and tree mathematical models. In each instance, the performance of an NIM threshold (equations 3 to 5 below) produced the best performance indices (positive and negative predictive power) (Table 7-9). The NIM equation normalises for the amount if input bisulfite modified DNA present in the sample and calculates the proportion of the target sequence which is methylated relative to a 100% fully methylated DNA sequence.

$$NIM = [(TARGET_{sample}/TARGET_{MC})/(ACTB_{sample}/ACTB_{MC})] \times 1000 \ldots\ldots(3)$$

NIM threshold for discriminating biopsy positive from biopsy negative was determined as 0.73:

$$\text{NIM SUM (POSITIVE)}: (NIM_{Gene1}+NIM_{Gene2}+NIM_{Gene3}+NIM_{Gene4}+NIM_{Gene\ 5}+NIM_{Gene6}) > 0.73 \ldots\ldots (4)$$

Data from the 283 men show that for the 6 gene panel, the NIM threshold for detecting high-risk/high-grade disease was determined as 1.25 across the 6 gene panel.

$$\text{NIM SUM (HIGH RISK)}: (NIM_{Gene1}+NIM_{Gene2}+NIM_{Gene3}+NIM_{Gene4}+NIM_{Gene5}+NIM_{Gene6}) > 1.25 \ldots.. (5)$$

Detection of high-grade Prostate Cancer

By applying this model (NIM_SUM >1.25), epiCaPture has a comparable sensitivity for high-grade prostate cancer (>=Gleason score 8) compared with the predicate test, PSA (Table 9, Figure 9). In this cohort of men, epiCaPture detected 84.85% of men with high-grade disease, as compared with 90.91% detected by PSA. The specificity and negative predictive value (Table 9, Table 10) of epiCaPture is far superior to PSA. Almost 98% of men with a negative biopsy tested negative for epiCaPture. Comparably, only 24.44% of the 135 men with a negative biopsy did not have an elevated PSA. This high false-positive rate (76%) of PSA is the reason why so many men undergo unnecessary biopsy.

**Table 7. epiCaPture performance characteristics: biopsy positive versus biopsy negative**

| **SEQ ID NO (Gene)** | AUC | Sensitivity | Specificity | PPV | NPV |
|---|---|---|---|---|---|
| 1 (GSTP1) | 0.69 | 0.39 | 0.98 | 0.95 | 0.59 |
| 2 (SFRP2) | 0.64 | 0.35 | 0.93 | 0.84 | 0.57 |
| 3 (IGFBP3) | 0.65 | 0.35 | 0.93 | 0.84 | 0.57 |
| 4 (IGFBP7) | 0.66 | 0.33 | 0.97 | 0.92 | 0.57 |
| 5 (APC) | 0.69 | 0.43 | 0.93 | 0.86 | 0.60 |
| 6 (PTGS2) | 0.66 | 0.40 | 0.93 | 0.87 | 0.59 |
| Best 4 (1,2, 3, 4) | 0.75 | 0.45 | 1.00 | 1.00 | 0.63 |
| Best 5 (1, 3, 4, 5, 6) | 0.76 | 0.49 | 0.99 | 0.97 | 0.64 |
| All 6 | 0.77 | 0.50 | 0.98 | 0.96 | 0.64 |
| NIM Sum 4 (1,3,4,5) | | 0.45 | 0.99 | 0.99 | 0.62 |
| NIM Sum 5 (1,2,4,5,6) | | 0.46 | 0.99 | 0.97 | 0.62 |
| **NIM Sum > 0.73** | | **0.55** | **0.93** | **0.9** | **0.65** |
| **Tree (G1 + G3)** | | 0.76 | 0.9 | 0.5 | 0.97 |
| **LASSO (G1 + G3 +G4 + G5)** | 0.76 | 0.56 | 0.9 | 0.86 | 0.65 |

Individual genes (targets) varied in their ability to discriminate presence of prostate cancer (biopsy-positive) from absence (biopsy-negative), ranging from a sensitivity of 33% (Gene 4) to 40% (Gene 6) (Table 7). Increasing the number of markers, for example, the best 4 or 5 or all 6, improved the sensitivity of urinary detection of prostate cancer to 45%, 49% and 50%, respectively.

However, summing the NIM across the gene panel and applying an NIM sum threshold of >0.73 improved the sensitivity to 55% of men with prostate cancer. The positive and negative predictive values for prostate cancer by applying an NIM sum threshold >0.73 are 90% and 65%, respectively.

**Table 8. epiCaPture performance characteristics: detection of high risk disease**

| **SEQ ID NO (Gene)** | AUC | Sensitivity | Specificity | PPV | NPV |
|---|---|---|---|---|---|
| 1 (GSTP1) | 0.79 | 0.63 | 0.92 | 0.62 | 0.93 |
| 2 (SFRP2) | 0.78 | 0.59 | 0.93 | 0.61 | 0.92 |
| 3 (IGFBP3) | 0.78 | 0.46 | 0.97 | 0.78 | 0.90 |
| 4 (IGFBP7) | 0.79 | 0.57 | 0.93 | 0.62 | 0.92 |
| 5 (APC) | 0.78 | 0.61 | 0.92 | 0.60 | 0.92 |
| 6 (PTGS2) | 0.74 | 0.48 | 0.95 | 0.63 | 0.90 |
| Best 4 (2,3,5,6) | 0.81 | 0.87 | 0.63 | 0.31 | 0.96 |
| Best 5 (1, 2,3,4,6) | 0.80 | 0.87 | 0.52 | 0.26 | 0.95 |
| All 6 | 0.78 | 0.89 | 0.13 | 0.17 | 0.86 |
| NIM Sum 4 (1,3,5,6) | | 0.80 | 0.88 | 0.56 | 0.96 |
| NIM Sum 5 (1,2,3,4,5) | | 0.83 | 0.85 | 0.52 | 0.96 |
| **NIM Sum >1.25** | | **0.83** | **0.85** | **0.52** | **0.96** |

(continued)

| Table 8. epiCaPture performance characteristics: detection of high risk disease | | | | | |
|---|---|---|---|---|---|
| **SEQ ID NO (Gene)** | AUC | Sensitivity | Specificity | PPV | NPV |
| **Tree (G3 + G5)** | | 0.74 | 0.91 | 0.61 | 0.95 |
| **LASSO (G3 + G4 + G5 + G6)** | 0.86 | 0.89 | 0.70 | 0.36 | 0.97 |

Individual genes (targets) varied in their ability to detect high-risk prostate cancers, ranging from a sensitivity of 46% (Gene 3) to 63% (Gene 1) (Table 8). Increasing the number of markers, for example, the best 4 or 5 or all 6, does not markedly improve the accuracy of detecting high-risk prostate cancer, over individual markers, which can be attributed to the molecular heterogeneity of prostate cancer.

However, summing the NIM across the panel of best 4 or best 5 or applying an NIM sum threshold of 1.25 improved the sensitivity to 80% and 83%, respectively. The positive and negative predictive value for high-risk prostate cancer by applying an NIM sum threshold >1.25 are 52% and 96%, respectively.

| Table 9. epiCaPture performance characteristics: detection of high -grade disease | | | | | |
|---|---|---|---|---|---|
| **SEQ ID NO (Gene)** | AUC | Sensitivity | Specificity | PPV | NPV |
| 1 (GSTP1) | 0.773091 | 0.606061 | 0.892 | 0.425532 | 0.944915 |
| 2 (SFRP2) | 0.79297 | 0.636364 | 0.92 | 0.512195 | 0.950413 |
| 3 (IGFBP3) | 0.790545 | 0.515152 | 0.968 | 0.68 | 0.937984 |
| 4 (IGFBP7) | 0.803212 | 0.606061 | 0.912 | 0.47619 | 0.946058 |
| 5 (APC) | 0.783212 | 0.636364 | 0.896 | 0.446809 | 0.949153 |
| 6 (PTGS2) | 0.728424 | 0.515152 | 0.932 | 0.5 | 0.935743 |
| Best 4 (3, 4, 5, 6) | 0.82 | 0.88 | 0.62 | 0.24 | 0.98 |
| Best 5 (2, 3, 4, 5, 6) | 0.82 | 0.88 | 0.51 | 0.19 | 0.97 |
| All 6 | 0.76 | 0.88 | 0.08 | 0.11 | 0.83 |
| NIM Sum 4 (2,3,4,5) | | 0.82 | 0.87 | 0.45 | 0.97 |
| NIM Sum 5 (2, 3, 4, 5, 6) | | 0.82 | 0.85 | 0.42 | 0.97 |
| **NIM Sum >1.25** | | **0.85** | **0.82** | **0.38** | **0.98** |
| **Tree (G3 + G6)** | | 0.73 | 0.92 | 0.55 | 0.96 |
| **LASSO (G3 + G5 + G6)** | 0.83 | 0.78 | 0.82 | 0.36 | 0.97 |

Individual genes also varied in their ability to detect high-grade (Gleason score >=8) prostate cancers, ranging from a sensitivity of 52% (Gene 3 and 6) to 64% (Gene 2 and 5) (Table 9). Increasing the number of markers, for example, the best 4 or 5 or all 6, does not markedly improve the accuracy of detecting high-grade prostate cancer, over individual markers, which can be attributed to the molecular heterogeneity of prostate cancer.

However, summing the NIM across the panel of best 4 or best 5 or applying an NIM sum threshold of 1.25 improved the sensitivity to 82% and 85%, respectively. The positive and negative predictive values for high-grade prostate cancer by applying an NIM sum threshold >1.25 are 38% and 98%, respectively.

By applying this model (NIM_SUM>1.25), epiCaPture has a comparable sensitivity for high-grade prostate cancer (>=Gleason score 8) compared with the predicate test, PSA (Table 10, Figure 9). In this cohort of men, epiCaPture detected 84.85% of men with high-grade disease, as compared with 90.91% detected by PSA. The specificity and negative predictive value (Table 8, Table 9) of epiCaPture is far superior to PSA. Almost 98% of men with a negative biopsy tested negative for epiCaPture. Comparably, only 24.44% of the 135 men with a negative biopsy did not have an elevated PSA. This high false-positive rate (76%) of PSA is the reason why so many men undergo unnecessary biopsy.

| Table 10. Relative Sensitivity & Specificity | | | |
|---|---|---|---|
| | SENSITIVITY | | |
| | n | epiCaPture high n (%) | PSA >= 4 ng/ml n (%) |
| **Biopsy positive**<br>False-negative rate | 148 | 70 (47.30)<br>0.53 | 136 (91.89)<br>0.08 |
| **Gleason >=8**<br>False-negative rate | 33 | 28 (84.85)<br>0.15 | 30 (90.91)<br>0.09 |
| | SPECIFICITY | | |
| | n | epiCaPture negative n (%) | PSA < 4 ng/ml n (%) |
| **Biopsy negative**<br>False-positive rate | 135 | 132 (97.78)<br>0.02 | 33 (24.44)<br>0.76 |

Quantitative analysis of DNA methylation at six gene loci in prostate tissues and urine samples indicates that high levels of methylation detected in high-risk tumour tissues can be measured in urine as a surrogate. Examples of this are shown for five of the six gene panel, Target 1 (GSTP1; Figure 10), Target 2 (SFRP2; Figure 11), Target 3 (IGFBP3; Figure 12), Target 4 (IGFBP7; Figure 13) and Target 5 (APC; Figure 14).

Quantitative analysis of DNA methylation at the seven of the remaining ten gene loci that were analysed in prostate tissues indicates that high levels of methylation detected in high-risk tumour tissues can be measured. The genes were analysed on 3 independent cohorts of prostate tissue samples and all show consistent patterns of significant methylation in high-risk/aggressive prostate cancer. Examples of this are shown for Target 7 (LXN; Figure 15), Target 8 (MAGPIE-1B; Figure 16), Target 9 (DNAH10; Figure 17), Target 10 (ZMIZ1; Figure 18), Target 11 (CENPV; Figure 19), Target 12 (OR2L13; Figure 20) and Target 13 (F3; Figure 21). The details of the three different cohorts used for the study relating to those genes listed above are provided below.

*Cohort A*

Benign prostate tissue was obtained from radical cystoprostatectomy or trans-urethral resection of the prostate, from men with no clinical or histopathological evidence of prostate cancer. Precursor lesions proliferative inflammatory atrophy (PIA) and high grade prostatic intra-epithelial neoplasia (HGPIN), (HGPIN) and primary tumours (indolent (PCI) and aggressive (PCA)) were all obtained from radical prostatectomy specimens. PCI was defined as Gleason 6, pT2 disease, with a pre-operative PSA <10 ng/ml and no evidence of biochemical or clinical recurrence (5-year follow-up). PCA was defined as primary Gleason ≥4, pT3 disease, with evidence of biochemical or clinical recurrence. Metastatic lesions were obtained from visceral metastases (liver and or lymph node), obtained during rapid autopsy. All patient samples were obtained retrospectively with ethical approval granted by the associated institutions: benign (St. James's Hospital (SJH), Ireland; Adelaide and Meath Hospital incorporating the National Children's Hospital (AMNCH), Ireland); PIA (SJH); HGPIN (AMNCH); PCI (SJH; Mater Misericordiae (MM), Ireland; Beaumont Hospital (BH), Ireland); PCA (SJH; MM; BH); PCM (University of Washington, USA).

In each case, H&E slides were reviewed by a consultant pathologist, who identified and marked the relevant target areas. Six serial 8μm sections were cut from the respective formalin fixed paraffin embedded (FFPE) blocks and mounted onto PEN membrane glass slides (Life Technologies) for laser capture microdissection (LCM). The sixth section was H&E stained and reviewed to ensure a consistent percentage of target cells. LCM was performed to enrich for target epithelia as previously described, using the Arcturus XT system (Life Technologies). DNA and total RNA were isolated from LCM caps (harboring microdissected tissue) in parallel, using the QIAamp DNA micro kit (Qiagen) and RecoverAll Total Nucleic Acids isolation kit (Ambion), respectively.

**Table 11 - Clinicopathologic data for Cohort A**

| | Benign* | PIA* | HGPIN* | PCI* | PCA* | PCM |
|---|---|---|---|---|---|---|
| **Number of cases** | 10 | 7 | 6 | 7 | 8 | 6 |
| **Mean age (years)** | 66.10 | 61.30 | 61.50 | 57.50 | 58.75 | 72.83 |

(continued)

| | Benign* | PIA* | HGPIN* | PCI* | PCA* | PCM |
|---|---|---|---|---|---|---|
| median | 64.50 | 62.00 | 62.00 | 58.00 | 59.50 | 74.00 |
| range | 48-79 | 49-68 | 56-66 | 50-66 | 46-69 | 60-81 |
| Mean PSA (ng/ml) | NA | 7.74 | 8.05 | 5.23 | 8.00 | 62.48 |
| median | | 8.94 | 7.90 | 5.40 | 7.25 | 51.95 |
| range | | 3.18-9.89 | 5-11.60 | 3.60-7.10 | 4.50-13.60 | 41-105 |
| Gleason score (n) | | | | | | |
| 6 | - | 0 | 4 | 7 | 0 | 0 |
| 7 (3+4) | - | 4 | 0 | 0 | 0 | 0 |
| 7 (4+3) | - | 3 | 1 | 0 | 5 | 0 |
| 8 | - | 0 | 0 | 0 | 3 | 2 |
| 9 | - | 0 | 1 | 0 | 0 | 3 |
| 10 | - | 0 | 0 | 0 | 0 | 1 |
| TNM stage (n) | | | | | | |
| pT2a | - | 1 | 0 | 0 | 0 | 0 |
| pT2b | - | 0 | 1 | 0 | 0 | 0 |
| pT2c | - | 5 | 4 | 7 | 0 | 0 |
| pT3a | - | 1 | 1 | 0 | 4 | 0 |
| pT3b | - | 0 | 0 | 0 | 4 | 0 |
| pT3c | - | 0 | 0 | 0 | 0 | 0 |
| pT4 | - | 0 | 0 | 0 | 0 | 6 |
| BCR (n) | - | NA | NA | 0 | 8 | 6 |
| Abbreviations: PIA: proliferative inflammatory atrophy, HGPIN: high-grade prostatic intraepithelial neoplasia, PCI: indolent prostate cancer, PCA: aggressive prostate cancer, PCM: metastatic prostate cancer, BCR: biochemical recurrence, NA = not available. *cohorts are age-matched. | | | | | | |

*Cohort B*

A retrospective cohort of radical prostatectomy cases was used to validate potentially prognostic differentially methylated regions identified in cohort 1. All patient samples were obtained retrospectively with ethical approval granted by the associated institutions: benign (SJH, AMNCH) and tumor (SJH, MM and BH). Tumor samples were assigned as low-risk (Gleason score 3+3, pT2; n=23); significant (Gleason score 7, pT2; n=42); or high-risk (Gleason score ≥ 4+3, pT3; n=39), based on histopathological review of radical prostatectomy specimens. For control purposes, histologically benign prostate tissues (n=21) were procured from radical prostatectomy or trans-urethral resection of the prostate. Tumor and benign foci were marked by a consultant histopathologist (SPF, BL) and targeted macro-dissection with a scalpel was carried out on four serial 5μm sections. DNA and total RNA were isolated using the RecoverAll Total Nucleic Acids Isolation kit (Ambion).

**Table 12 - Clinicopathologic data for Cohort B**

| | Benign* | Tumor | | |
|---|---|---|---|---|
| | | low-risk | significant | high-risk |
| Number of cases | 21 | 23 | 42 | 39 |

(continued)

| | Benign* | Tumor | | |
|---|---|---|---|---|
| | | low-risk | significant | high-risk |
| **Mean age (years)** | 65.9 | 59.7 | 60 | 62 |
| **range** | 44-87 | 49-70 | 48-73 | 49-74 |
| **Mean PSA (ng/ml)** | 5.5 | 6.6 | 6.8 | 8.2 |
| **range** | 0.42-10.4 | 1.2-12.3 | 2.4-14.7 | 3.1-18.7 |
| **Gleason score (n)** | | | | |
| **≤6** | - | 23 | 1 | 0 |
| **7 (3+4)** | - | 0 | 37 | 17 |
| **7 (4+3)** | - | 0 | 4 | 14 |
| **≥8** | - | 0 | 0 | 8 |
| **TNM stage (n)** | | | | |
| **pT2** | - | 23 | 42 | 0 |
| **pT3** | - | 0 | 0 | 39 |
| **pT4** | - | 0 | 0 | 0 |

*Cohort C*

In June 2014, The Cancer Genome Atlas (TCGA) database was mined for HM450k data for patient specimens corresponding to low-risk (n=9), significant (n=68) and high-risk (n=67) PCa as defined already for cohort 2. Histologically benign HM450k data were also retrieved (n=29). For each sample, raw *.IDAT files were extracted and processed through an abridged run of RnBeads (including pre-filtering, BMIQ normalization and post-filtering). β-values for probes contained within the 13 potentially prognostic DMRs were extracted and a mean DMR β-value was calculated for each sample. Methylation differences between cohorts were assessed using an unpaired T test with Welch's correction. Significance was ascribed as P<0.05.

**Table 13 - Clinicopathologic data for Cohort C**

| | Benign* | Tumor | | |
|---|---|---|---|---|
| | | low-risk | significant | high-risk |
| **Number of cases** | 34 | 9 | 68 | 67 |
| **Mean age (years)** | 60.8 | 58 | 60.1 | 61.4 |
| **range** | 44-71 | 47-72 | 44-73 | 44-77 |
| **Mean PSA (ng/ml)** | 12.7 | 6.8 | 7.1 | 13.6 |
| **range** | 1.8-87 | 3.6 - 10 | 0.7 - 26.6 | 1.6 - 87 |
| **Gleason score (n)** | | | | |
| **≤6** | - | 9 | 0 | 0 |
| **7 (3+4)** | - | 0 | 56 | 0 |
| **7 (4+3)** | - | 0 | 12 | 35 |
| **≥8** | - | 0 | 0 | 32 |
| **TNM stage (n)** | | | | |
| **pT2** | - | 9 | 68 | 0 |
| **pT3** | - | 0 | 0 | 63 |

(continued)

| | Benign* | Tumor | | |
|---|---|---|---|---|
| | | low-risk | significant | high-risk |
| pT4 | - | 0 | 0 | 4 |

In the specification the terms "comprise, comprises, comprised and comprising" or any variation thereof and the terms "include, includes, included and including" or any variation thereof are considered to be totally interchangeable and they should all be afforded the widest possible interpretation and vice versa.

**References**

1. Tibshirani R. Regression shrinkage and selection via the LASSO. J. Royal. Statist. Soc B., 1996 vol. 58(1): 267-288.
2. Bastian PJ, Boorjian SA, Bossi A, et al. High-risk prostate cancer: from definition to contemporary management. European urology 2012; 61(6): 1096-106.
3. Eads CA DK, Kawakami K, Saltz LB, Blake C, Shibata D, Danenberg PV, Laird PW. MethyLight: a high-throughput assay to measure DNA methylation. Nucleic Acids Research 2000; 28(8): E32.
4. Perry AS, Loftus B, Moroose R, et al. In silico mining identifies IGFBP3 as a novel target of methylation in prostate cancer. British journal of cancer 2007; 96(10): 1587-94.
5. Perry AS, O'Hurley G, Raheem OA, et al. Gene expression and epigenetic discovery screen reveal methylation of SFRP2 in prostate cancer. International journal of cancer Journal international du cancer 2013; 132(8): 1771-80.
6. Yegnasubramanian S, Kowalski J, Gonzalgo ML, et al. Hypermethylation of CpG islands in primary and metastatic human prostate cancer. Cancer Res 2004; 64(6): 1975-86.
7. Sullivan L, Murphy TM, Barrett C, Loftus B, Thornhill J, Hollywood D, Lynch T, Perry AS. IGFBP7 promoter methylation and gene expression analysis in prostate cancer. Journal of Urology (2012) 188(4) 1354-60. PMID: 22906661.
8. Murphy TM, Tuzova AV, O'Rourke CJ, Greene C, Sullivan L, Thornhill J, Barrett C, Loftus B, Lynch T, Perry AS. Multigene Methylation Biomarker Analysis in Prostate Cancer. Epigenetic Diagnosis and Therapy, in press


## Claims

1. A method of determining the presence of high-risk prostate cancer in an individual, the method comprising a step of assaying a biological sample obtained from the individual for the presence of at least four methylated regulatory DNA sequences selected from group comprising: SEQ ID No's 1 to 6 and calculating a normalized index of methylation (NIM) score, wherein the presence of the at least four methylated regulatory DNA sequences and the calculated NIM score indicates a high-risk prostate cancer.

2. A method according to Claim 1, wherein detection of least four, five or six methylated regulatory DNA sequence from the group comprising SEQ ID NOs 1 to 6 and the calculated NIM score indicates the presence of a high-risk prostate cancer.

3. A method according to Claim 1, wherein detection of six methylated regulatory DNA sequences as defined by SEQ ID NOs 1 to 6 and having a sensitivity of at least 80% and the calculated NIM score indicates the presence of high-risk prostate cancer.

4. A method according to Claim 1, wherein detection of six methylated regulatory DNA sequences as defined by SEQ ID NOs 1 to 6 and the calculated NIM score indicates the presence of high-risk prostate cancer.

5. A method according to Claim 4, wherein detection of six methylated regulatory DNA sequences as defined by SEQ ID NOs 1 to 6, and having a sensitivity of at least 80%, and the calculated NIM score indicates the presence of high-risk prostate cancer.

6. A method according to Claim 1 in which the sample is urine or a urine derivative from the individual.

7. A method according to Claim 1 for determining an aggressive prostate cancer in an individual, the method further comprising the step of assaying the biological sample obtained from the individual for the presence of at least one sequence selected from SEQ ID NOs 17 and 18, wherein detection of at least four of the methylated regulatory DNA sequences from SEQ ID NOs 1 to 6 and one sequence from SEQ ID NOs 17 and 18 in a sample and calculating a NIM score indicates the presence of an aggressive prostate cancer, and wherein the sensitivity of the assay for detecting the at least four methylated regulatory DNA sequences from SEQ ID NOs 1 to 6 is at least 80%.

**8.** A method according to Claim 7, in which the at least one sequence selected from SEQ ID NOs 17 and 18, is SEQ ID NO: 18, encoding prostate-specific antigen (PSA).

**9.** A method according to Claim 7 or Claim 8, in which detection of at least six methylated DNA regulatory sequences as defined by SEQ ID NOs 1 to 6, having a sensitivity of at least 80% and the calculated NIM score indicates the presence of an aggressive (metastatic) prostate cancer.

**10.** A kit for detecting the presence of prostate cancer in a sample from an individual, the kit comprising a control oligonucleotide as defined by SEQ ID NO 19, 20 or 21, and a set of oligonucleotides for detecting SEQ ID NOs 1 to 6, wherein detection of at least four sequences from SEQ ID NOs 1 to 6 in the sample and a calculated NIM score indicates the presence of prostate cancer, and wherein the sensitivity of the assay for detecting the at least four sequences from SEQ ID NOs 1 to 6 is at least 80%.

**11.** A kit as claimed in Claim 10, wherein the kit further comprises an oligonucleotide for detecting the presence of PSA.

**12.** A kit as claimed in Claim 10 or Claim 11, wherein the set of oligonucleotides is defined by SEQ ID NOs. 22 to 72.

**13.** A kit as claimed in Claim 10 or Claim 11, wherein the kit further comprises a support having at least one oligonucleotide selected from group SEQ ID No's 1 to 6 anchored thereon.

**14.** A method according to Claim 1 and Claim 7, in which the methylated regulatory DNA sequences selected from SEQ ID NOs 1 to 6 comprise SEQ ID No's 1 and 3.

## Patentansprüche

**1.** Verfahren zur Bestimmung der Gegenwart von Hochrisiko-Prostatakrebs bei einem Individuum, wobei das Verfahren Folgendes umfasst: einen Schritt des Untersuchens einer biologischen Probe, die von dem Individuum erhalten wurde, auf die Gegenwart von mindestens vier methylierten regulatorischen DNA-Sequenzen, die aus der Gruppe ausgewählt sind, die SEQ.-ID Nr. 1 bis 6 umfasst, und Berechnen eines Werts des normalisierten Methylierungsindex (NIM = Normalized Index of Methylation), wobei die Gegenwart der mindestens vier methylierten regulatorischen DNA-Sequenzen und der berechnete NIM-Wert einen Hochrisiko-Prostatakrebs anzeigen.

**2.** Verfahren nach Anspruch 1, wobei der Nachweis von mindestens vier, fünf oder sechs methylierten regulatorischen DNA-Sequenzen aus der Gruppe, die SEQ.-ID Nr. 1 bis 6 umfasst, und der berechnete NIM-Wert die Gegenwart eines Hochrisiko-Prostatakrebses anzeigen.

**3.** Verfahren nach Anspruch 1, wobei der Nachweis von sechs methylierten regulatorischen DNA-Sequenzen, wie durch SEQ.-ID Nr. 1 bis 6 definiert, und der eine Empfindlichkeit von mindestens 80 % aufweist, und der berechnete NIM-Wert die Gegenwart von Hochrisiko-Prostatakrebs anzeigen.

**4.** Verfahren nach Anspruch 1, wobei der Nachweis von sechs methylierten regulatorischen DNA-Sequenzen, wie durch SEQ.-ID Nr. 1 bis 6 definiert, und der berechnete NIM-Wert die Gegenwart von Hochrisiko-Prostatakrebs anzeigen.

**5.** Verfahren nach Anspruch 4, wobei der Nachweis von sechs methylierten regulatorischen DNA-Sequenzen, wie durch SEQ.-ID Nr. 1 bis 6 definiert, und der eine Empfindlichkeit von mindestens 80 % aufweist, und der berechnete NIM-Wert die Gegenwart von Hochrisiko-Prostatakrebs anzeigen.

**6.** Verfahren nach Anspruch 1, in welchem die Probe Urin oder ein Urinderivat von dem Individuum ist.

**7.** Verfahren nach Anspruch 1 für die Bestimmung eines aggressiven Prostatakrebses bei einem Individuum, wobei das Verfahren weiter Folgendes umfasst: den Schritt des Untersuchens der biologischen Probe, die von dem Individuum erhalten wurde, auf die Gegenwart von mindestens einer Sequenz, die aus SEQ.-ID Nr. 17 und 18 ausgewählt ist, wobei der Nachweis von mindestens vier der methylierten regulatorischen DNA-Sequenzen aus SEQ.-ID Nr. 1 bis 6 und einer Sequenz aus SEQ.-ID Nr. 17 und 18 in einer Probe und das Berechnen eines NIM-Werts die Gegenwart eines aggressiven Prostatakrebses anzeigen und wobei die Empfindlichkeit der Untersuchung für den Nachweis der mindestens vier methylierten regulatorischen DNA-Sequenzen aus SEQ.-ID Nr. 1 bis 6 mindestens 80 % beträgt.

**8.** Verfahren nach Anspruch 7, in welchem die mindestens eine Sequenz, die aus SEQ.-ID Nr. 17 und 18 ausgewählt ist, SEQ.-ID Nr. 18 ist, die prostataspezifisches Antigen (PSA) kodiert.

**9.** Verfahren nach Anspruch 7 oder Anspruch 8, in welchem der Nachweis von mindestens sechs methylierten regulatorischen DNA-Sequenzen, wie durch SEQ.-ID Nr. 1 bis 6 definiert, der eine Empfindlichkeit von mindestens 80 % aufweist, und der berechnete NIM-Wert die Gegenwart eines aggressiven (metastasierenden) Prostatakrebs anzeigen.

**10.** Kit für den Nachweis der Gegenwart von Prostatakrebs in einer Probe von einem Individuum, wobei der Kit ein Kontroll-Oligonukleotid, wie durch SEQ.-ID Nr. 19, 20 oder 21 definiert, und einen Satz von Oligonukleotiden für das Nachweisen von SEQ.-ID Nr. 1 bis 6 umfasst, wobei der Nachweis von mindestens vier Sequenzen von SEQ.-ID Nr. 1 bis 6 in der Probe und ein berechneter NIM-Wert die Gegenwart von Prostatakrebs anzeigen und wobei die Empfindlichkeit der Untersuchung für den Nachweis der mindestens vier Sequenzen von SEQ.-ID Nr. 1 bis 6 mindestens 80 % beträgt.

**11.** Kit nach Anspruch 10, wobei der Kit weiter ein Oligonukleotid für den Nachweis der Gegenwart von PSA umfasst.

**12.** Kit nach Anspruch 10 oder Anspruch 11, wobei der Satz von Oligonukleotiden durch SEQ.-ID Nr. 22 bis 72 definiert ist.

**13.** Kit nach Anspruch 10 oder Anspruch 11, wobei der Kit weiter einen Träger umfasst, der daran verankert mindestens ein Oligonukleotid, das aus der Gruppe SEQ.-ID Nr. 1 bis 6 ausgewählt ist, aufweist.

**14.** Verfahren nach Anspruch 1 und Anspruch 7, in welchem die methylierten regulatorischen DNA-Sequenzen, die aus SEQ.-ID Nr. 1 bis 6 ausgewählt sind, SEQ.-ID Nr. 1 und 3 umfassen.

## Revendications

**1.** Méthode destinée à la détermination de la présence d'un cancer de la prostate à risque élevé chez un individu, la méthode comprenant une étape consistant à doser un échantillon biologique obtenu auprès de l'individu pour la présence d'au moins quatre séquences d'ADN régulatrices méthylées choisies dans le groupe comprenant SEQ ID n° 1 à 6 et le calcul d'un score d'indice normalisé de méthylation (INM), où la présence des au moins quatre séquences d'ADN régulatrices méthylées et le score d'INM calculé indiquent un cancer de la prostate à risque élevé.

**2.** Méthode selon la revendication 1, dans laquelle la détection d'au moins quatre, cinq ou six séquences d'ADN régulatrices méthylées dans le groupe comprenant SEQ ID n° 1 à 6 et le score d'INM calculé indiquent la présence d'un cancer de la prostate à risque élevé.

**3.** Méthode selon la revendication 1, dans laquelle la détection de six séquences d'ADN régulatrices méthylées telles que définies par SEQ ID n° 1 à 6 et ayant une sensibilité d'au moins 80% et le score d'INM calculé indiquent la présence d'un cancer de la prostate à risque élevé.

**4.** Méthode selon la revendication 1, dans laquelle la détection de six séquences d'ADN régulatrices méthylées telles que définies par SEQ ID n° 1 à 6 et le score d'INM calculé indiquent la présence d'un cancer de la prostate à risque élevé.

**5.** Méthode selon la revendication 4, dans laquelle la détection de six séquences d'ADN régulatrices méthylées telles que définies par SEQ ID n° 1 à 6, et ayant une sensibilité d'au moins 80%, et le score d'INM calculé indiquent la présence d'un cancer de la prostate à risque élevé.

**6.** Méthode selon la revendication 1, dans laquelle l'échantillon est constitué d'urine ou d'un dérivé d'urine issu(e) de l'individu.

7. Méthode selon la revendication 1, destinée à la détermination d'un cancer de la prostate agressif chez un individu, la méthode comprenant en outre l'étape consistant à doser l'échantillon biologique obtenu auprès de l'individu pour la présence d'au moins une séquence choisie parmi SEQ ID n° 17 et 18, où la détection d'au moins quatre parmi les séquences d'ADN régulatrices méthylées de SEQ ID n° 1 à 6 et d'une séquence choisie parmi SEQ ID n° 17 et 18 dans un échantillon et le calcul d'un score d'INM indiquent la présence d'un cancer de la prostate agressif, et où la sensibilité du dosage pour la détection des au moins quatre séquences d'ADN régulatrices méthylées de SEQ ID n° 1 à 6 est d'au moins 80%.

8. Méthode selon la revendication 7, dans laquelle la au moins une séquence choisie parmi SEQ ID n° 17 et 18, est SEQ ID n° 18, codant pour un antigène prostatique spécifique (PSA).

9. Méthode selon la revendication 7 ou la revendication 8, dans laquelle la détection d'au moins six séquences d'ADN régulatrices méthylées telles que définies par SEQ ID n° 1 à 6, ayant une sensibilité d'au moins 80%, et le score d'INM calculé indiquent la présence d'un cancer de la prostate agressif (métastatique).

10. Kit destiné à la détection de la présence d'un cancer de la prostate dans un échantillon issu d'un individu, le kit comprenant un oligonucléotide témoin tel que défini pas SEQ ID n° 19, 20 ou 21, et un set d'oligonucléotides pour la détection de SEQ ID n° 1 à 6, dans lequel la détection d'au moins quatre séquences de SEQ ID n° 1 à 6 dans l'échantillon et un score d'INM calculé indiquent la présence d'un cancer de la prostate, et où la sensibilité du dosage de détection des au moins quatre séquences de SEQ ID n° 1 à 6 est d'au moins 80%.

11. Kit selon la revendication 10, le kit comprenant en outre un oligonucléotide pour la détection de la présence de PSA.

12. Kit selon la revendication 10 ou la revendication 11, dans lequel le set d'oligonucléotides est défini par SEQ ID n° 22 à 72.

13. Kit selon la revendication 10 ou la revendication 11, le kit comprenant en outre un support ayant au moins un oligonucléotide choisi dans le groupe constitué par SEQ ID n° 1 à 6 ancré sur celui-ci.

14. Méthode selon la revendication 1 et la revendication 7, dans laquelle les séquences d'ADN régulatrices méthylées choisies parmi SEQ ID n° 1 à 6 comprennent SEQ ID n° 1 et 3.

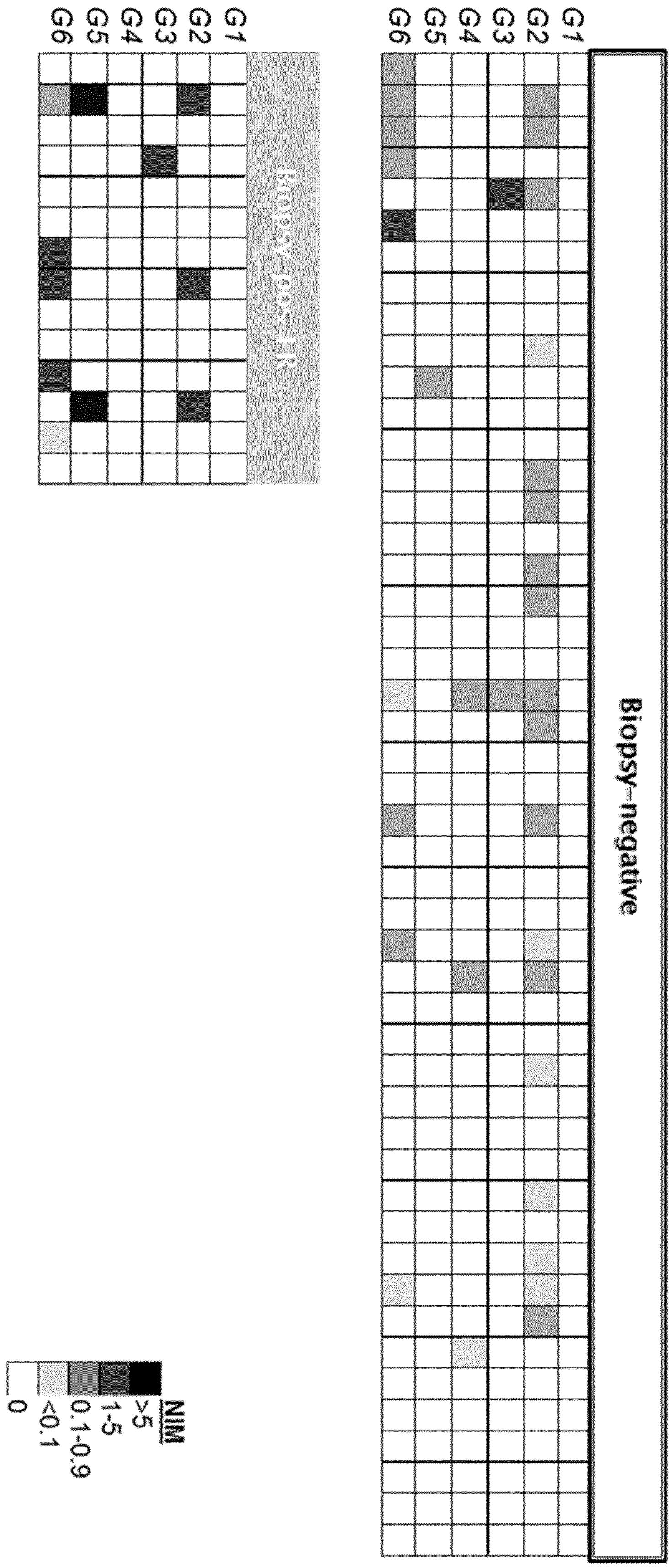
G1
G2
G3
G4
G5
G6
Biopsy–pos LR
Biopsy–negative
NIM
>5
1-5
0.1-0.9
<0.1
0

Figure 1

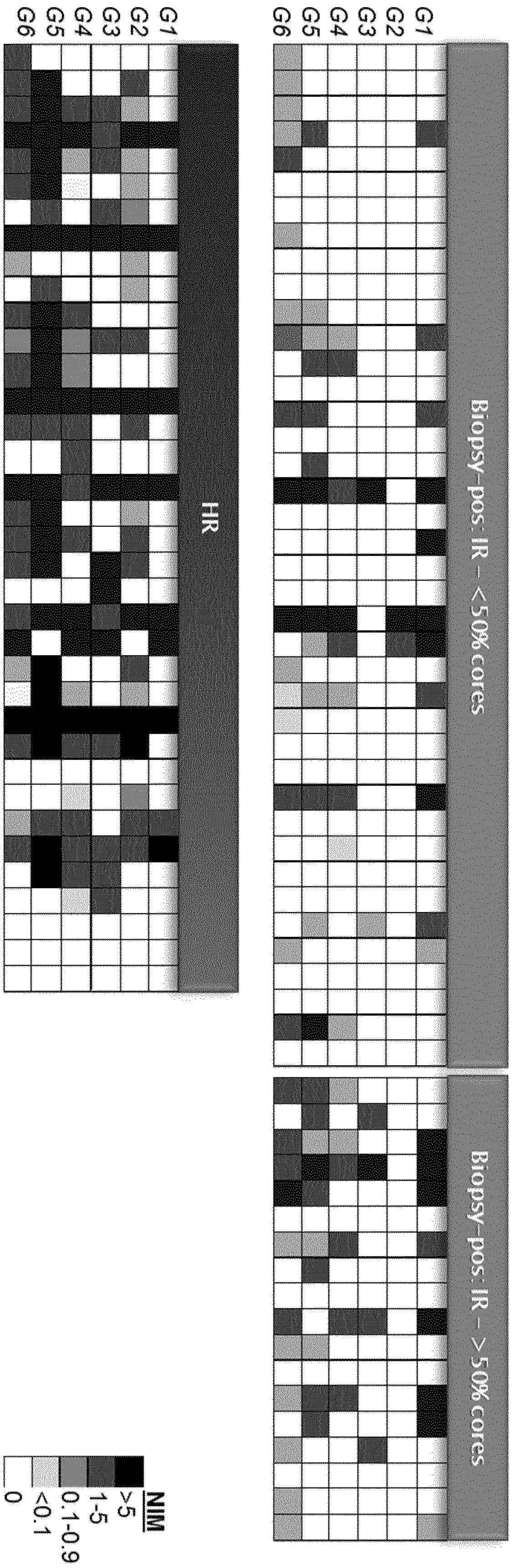


**Figure 1 (continued)**

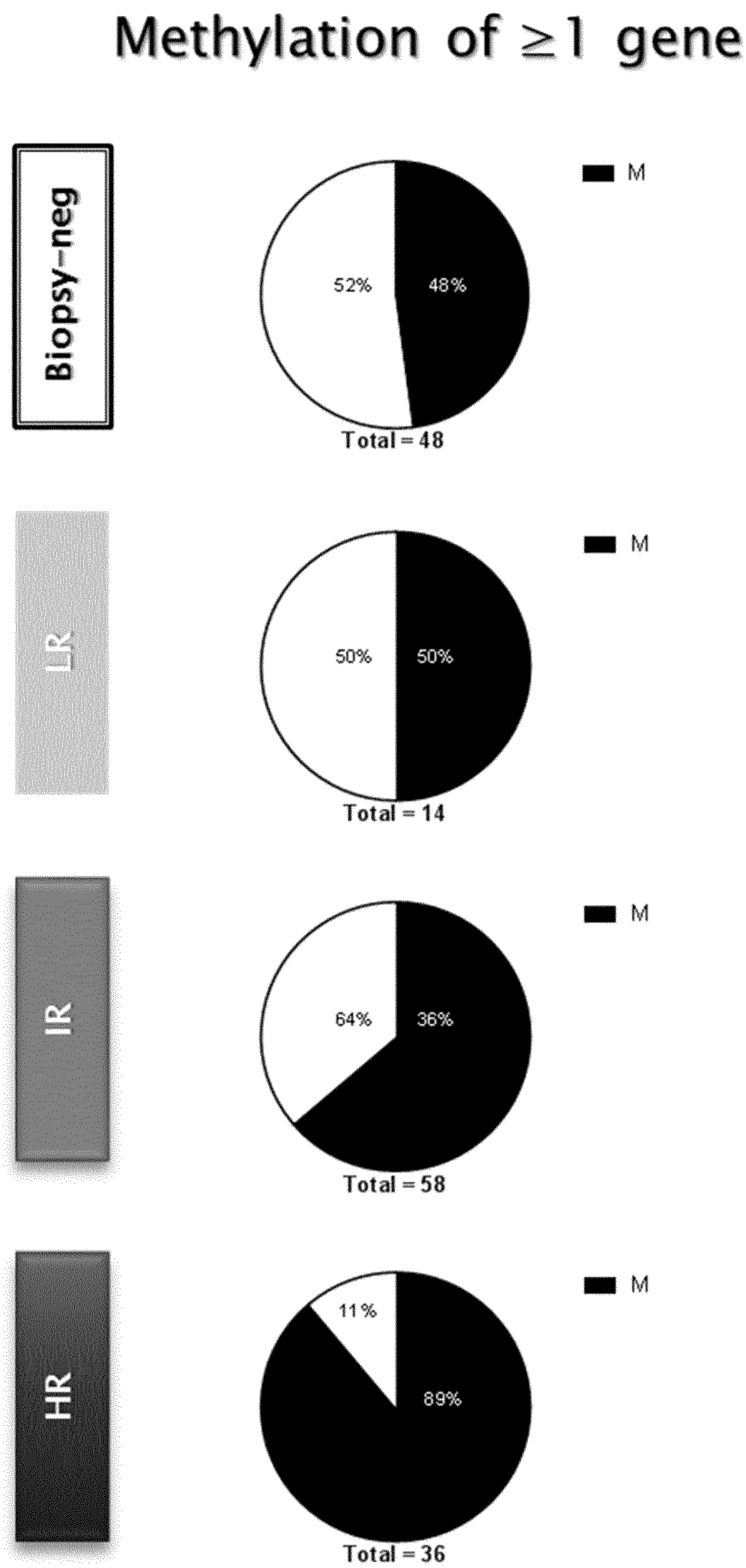
Methylation of ≥1 gene
Biopsy-neg
M
52%
48%
Total = 48
LR
M
50%
50%
Total = 14
IR
M
64%
36%
Total = 58
HR
M
11%
89%
Total = 36

Figure 2

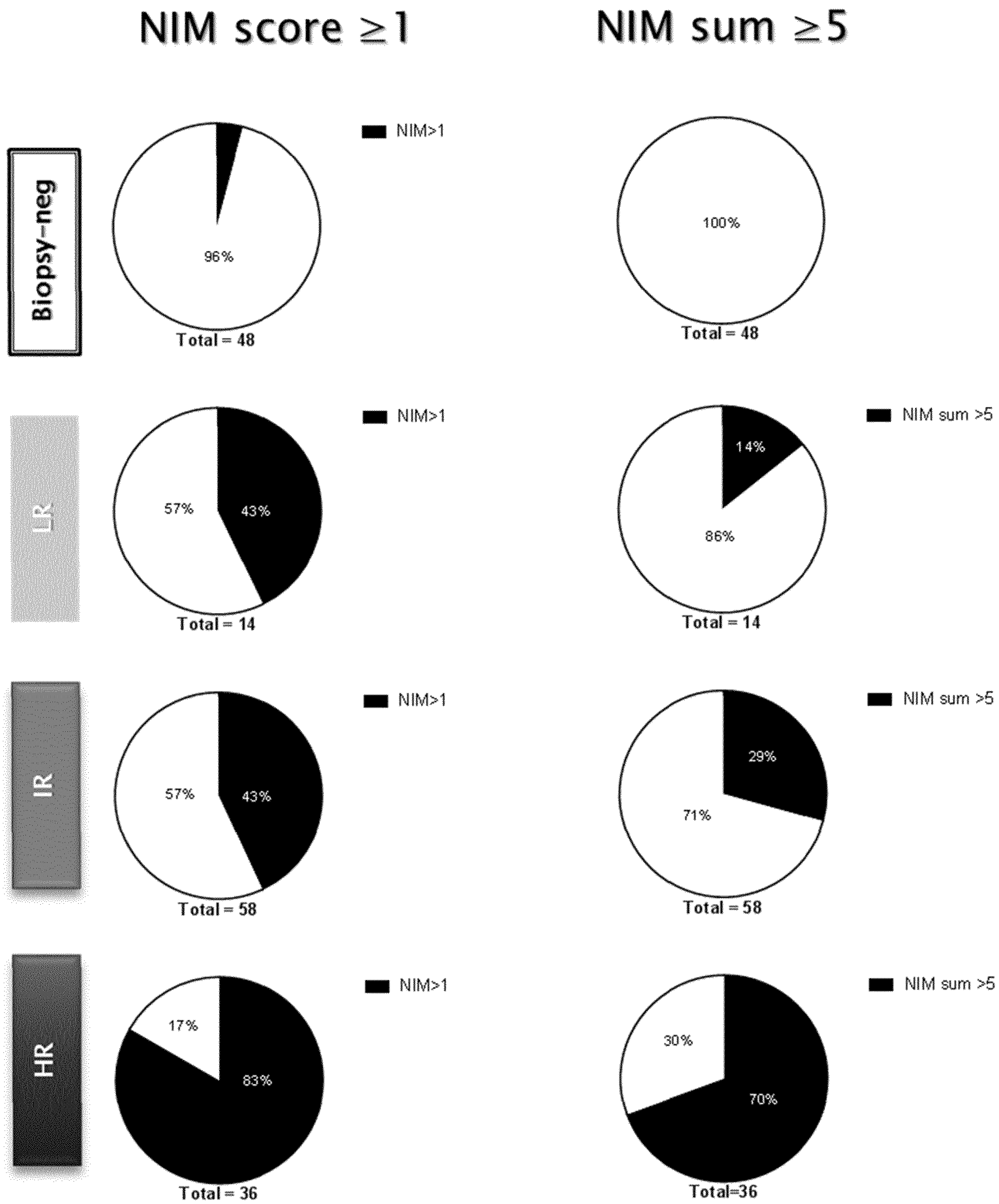


Figure 2 (cont.)

**(A) PSA**

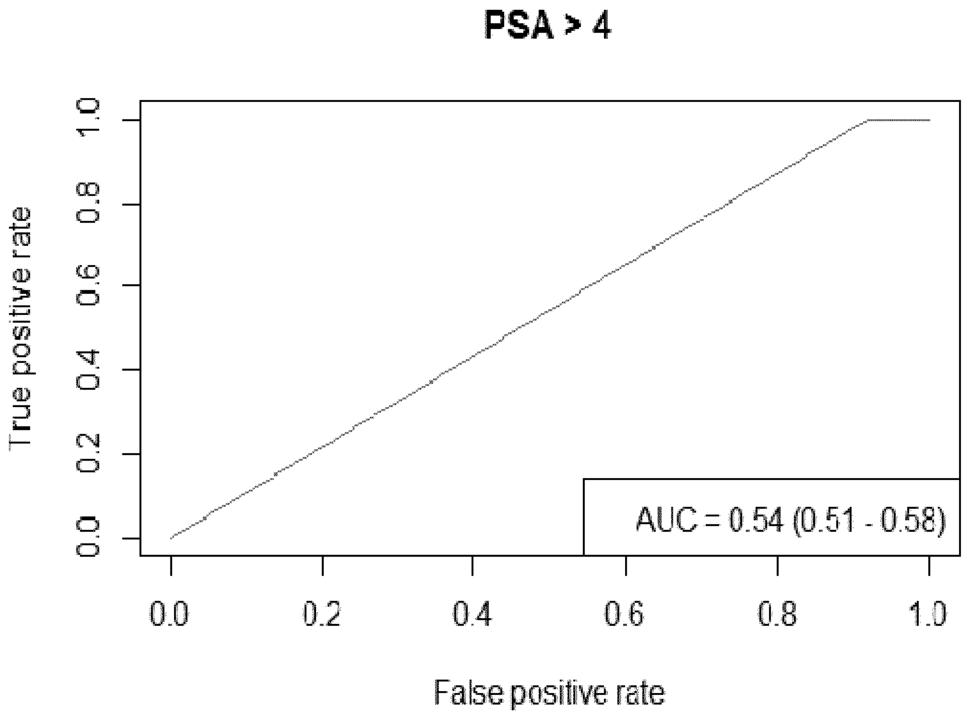


**(B) Individual Methylation Panel**

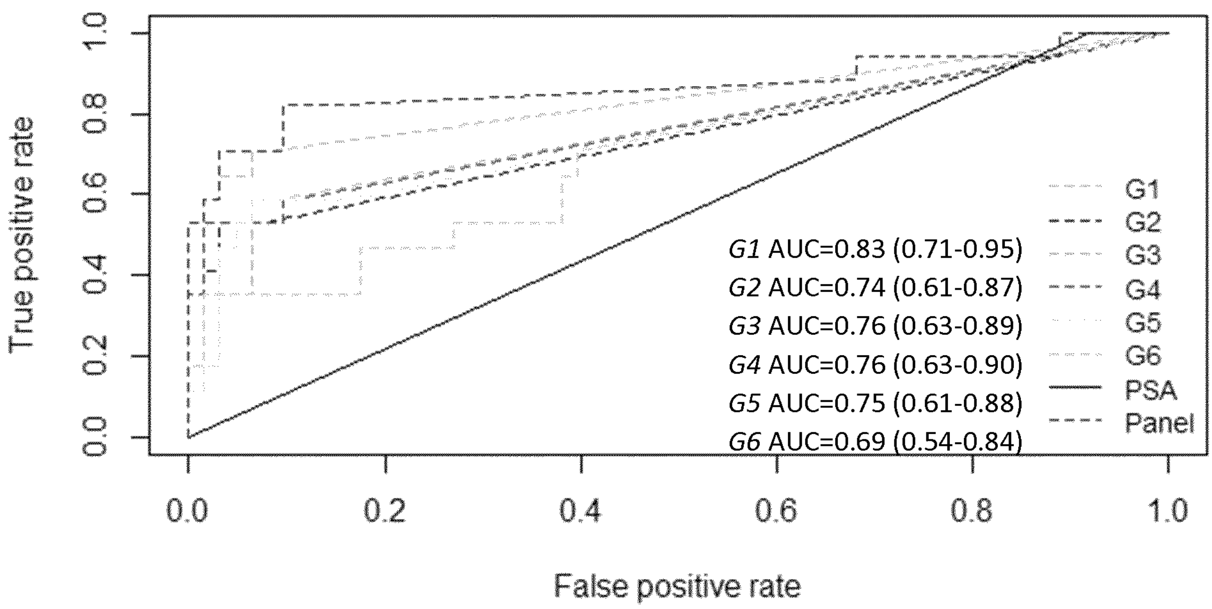


**Figure 3**

**(C) Methylation Panel**

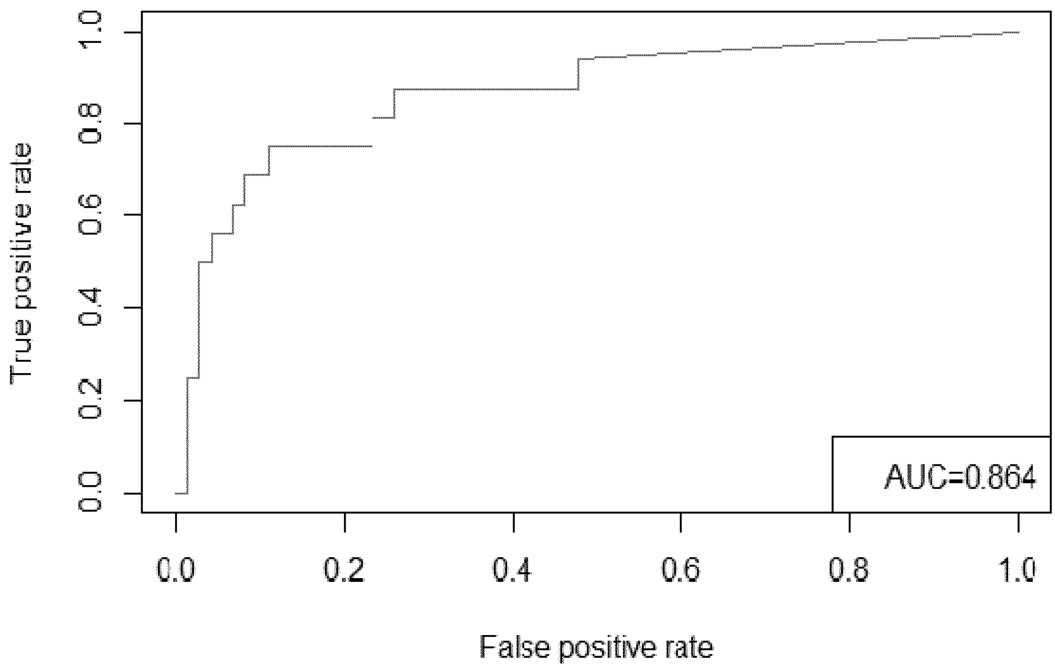


**(D)**

**Methylation Panel & PSA**

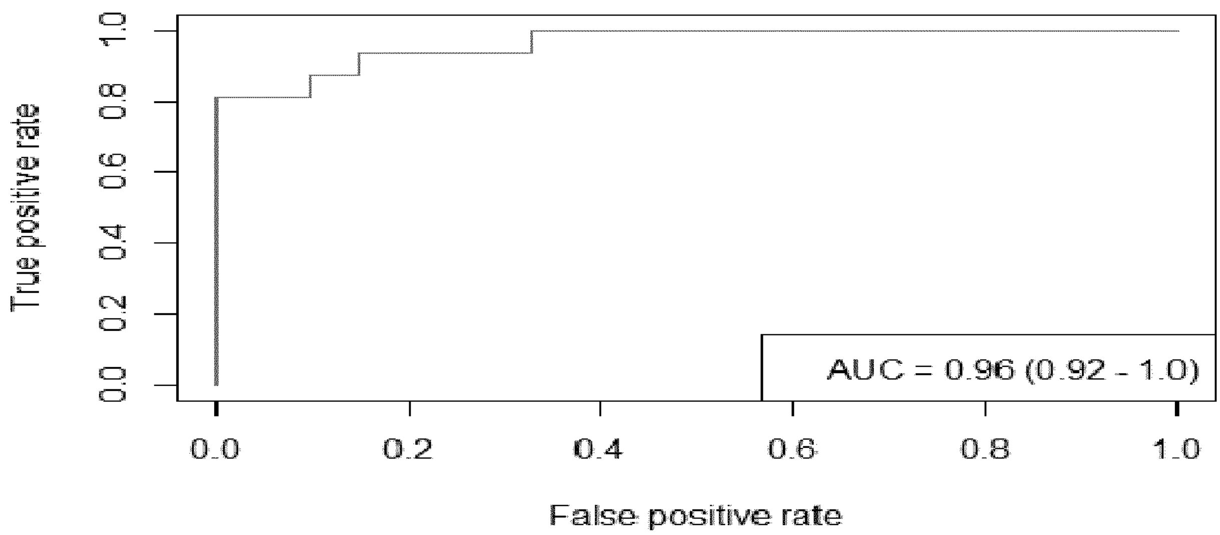


**Sensitivity = 0.94**
**Specificity = 0.67**

**Figure 3 (cont.)**

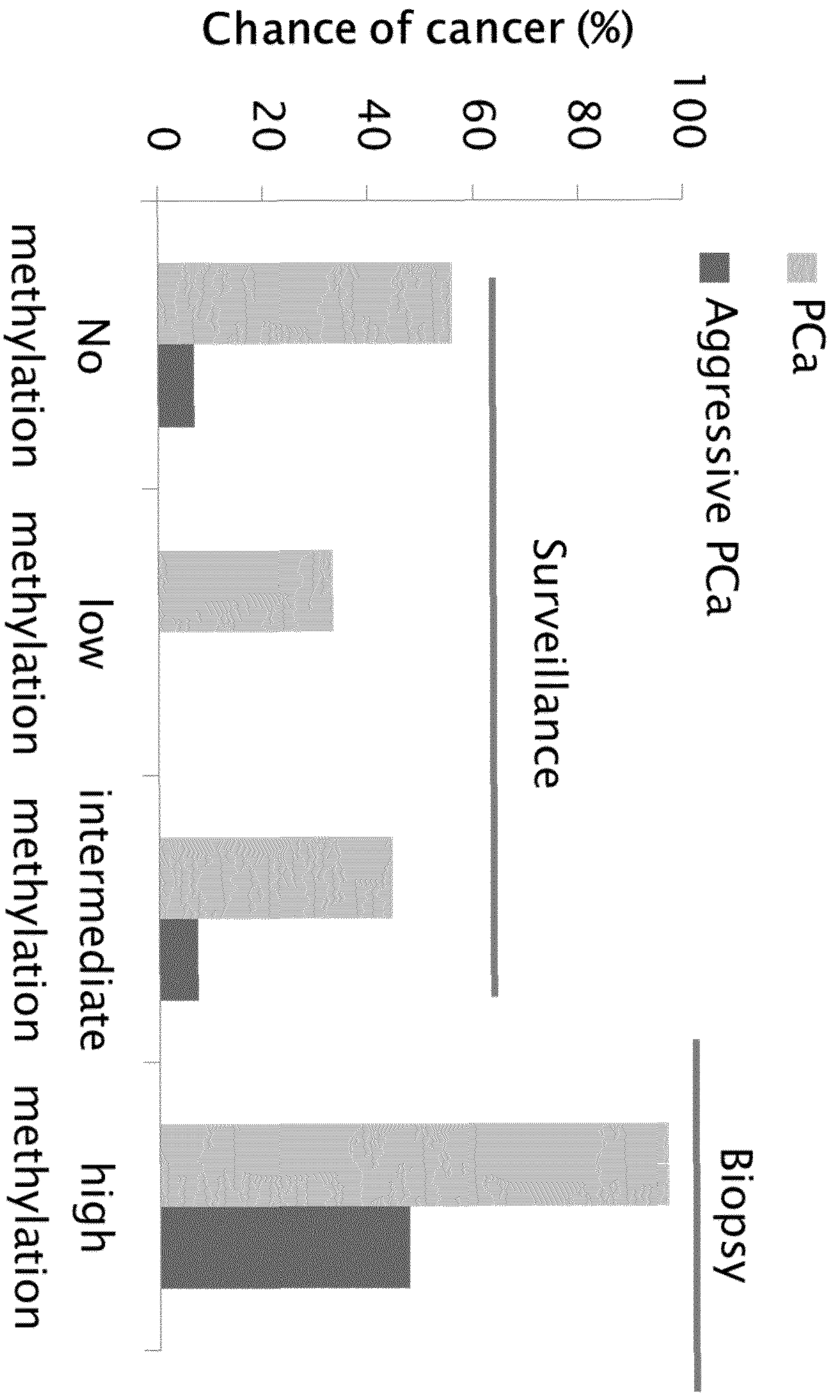
Chance of cancer (%)
100
80
60
40
20
0
PCa
Aggressive PCa
Surveillance
Biopsy
No methylation
low methylation
intermediate methylation
high methylation

Figure 4

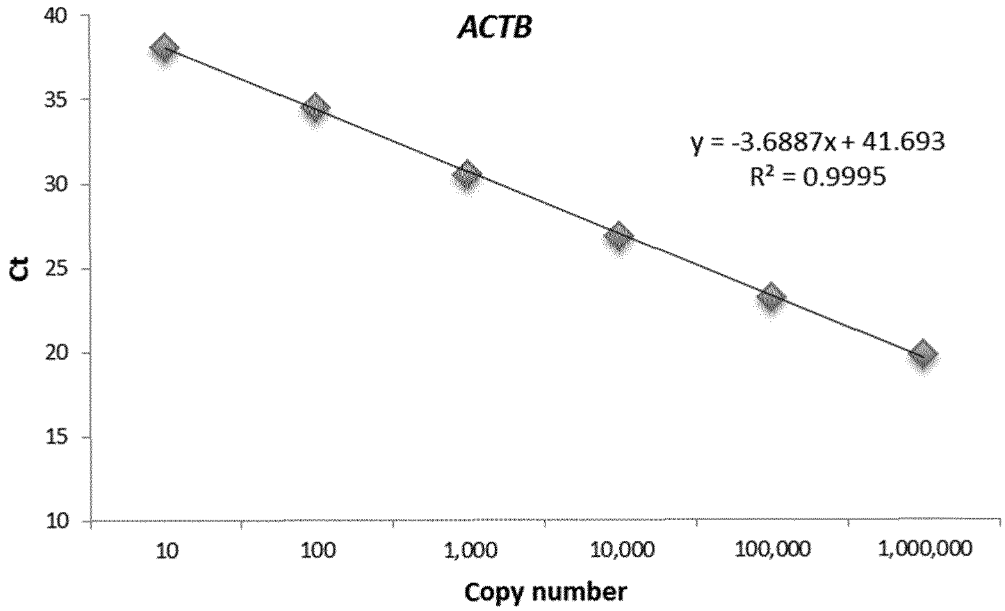
ACTB
40
35
30
25
20
15
10
Ct
y = -3.6887x + 41.693
R² = 0.9995
10
100
1,000
10,000
100,000
1,000,000
Copy number

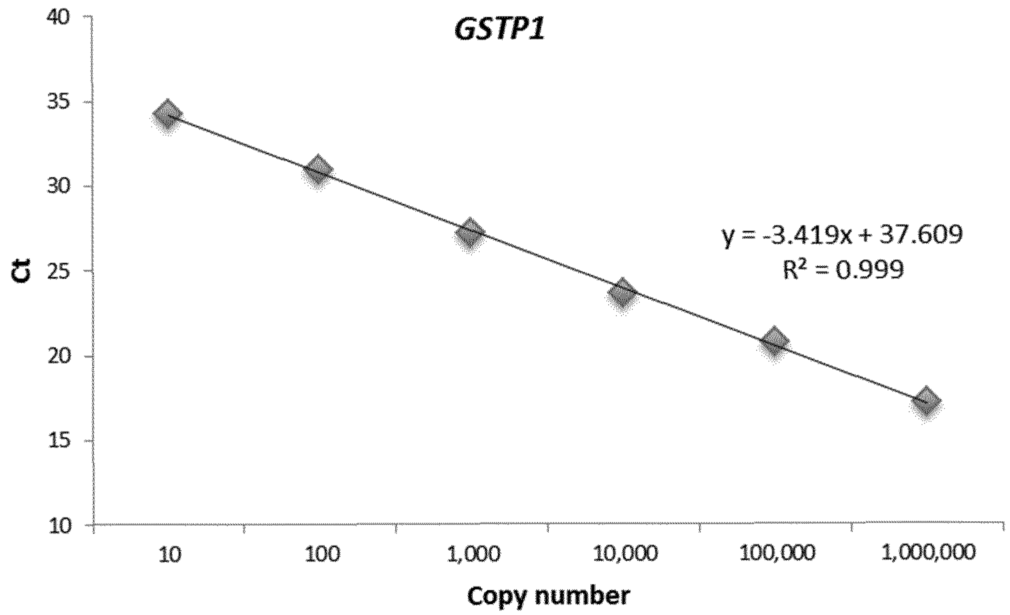
GSTP1
40
35
30
25
20
15
10
Ct
y = -3.419x + 37.609
R² = 0.999
10
100
1,000
10,000
100,000
1,000,000
Copy number

Figure 5

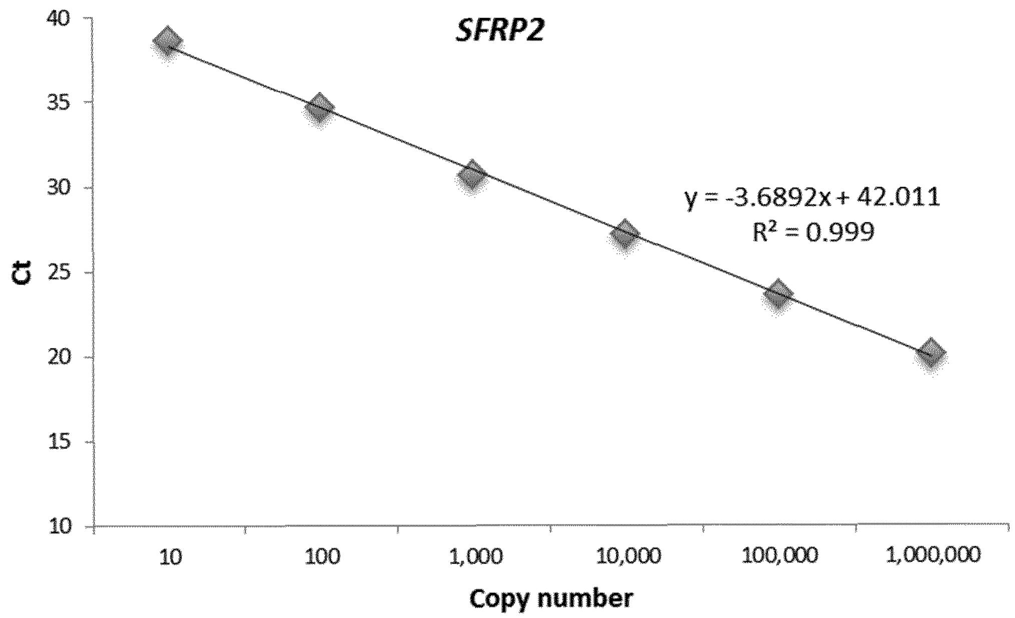


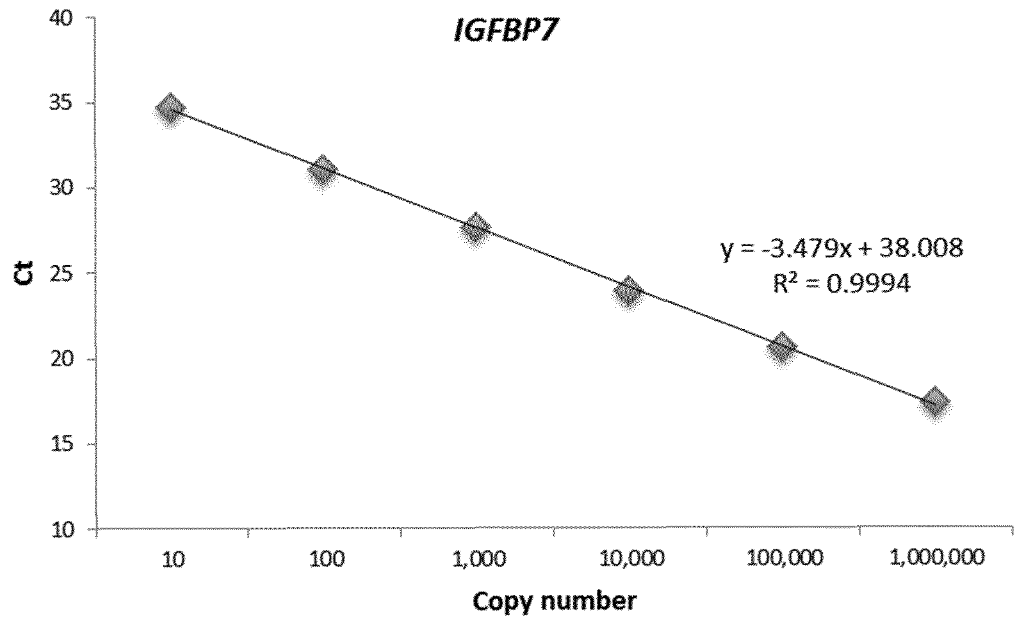


Figure 5 (cont.)

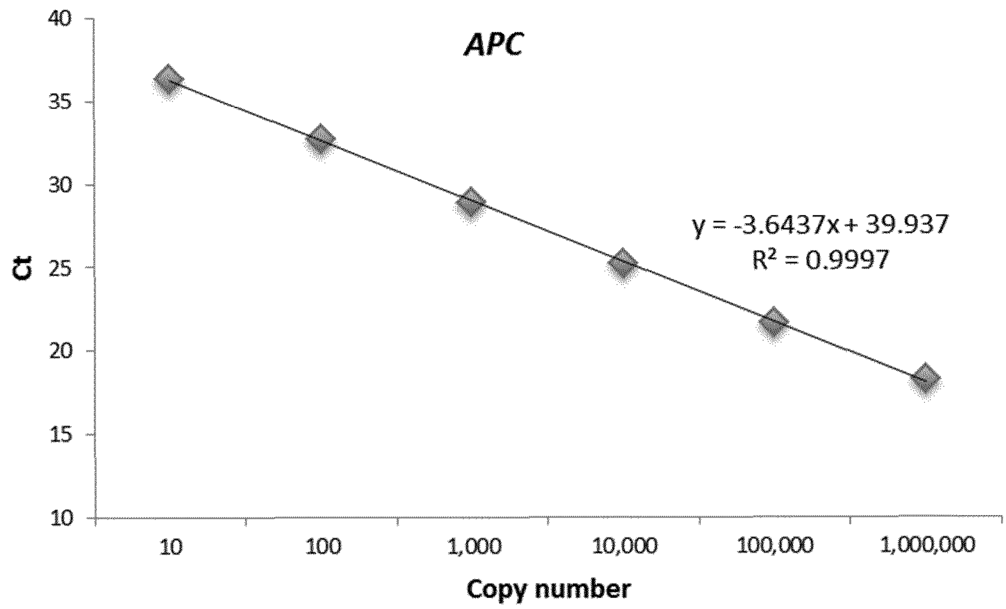


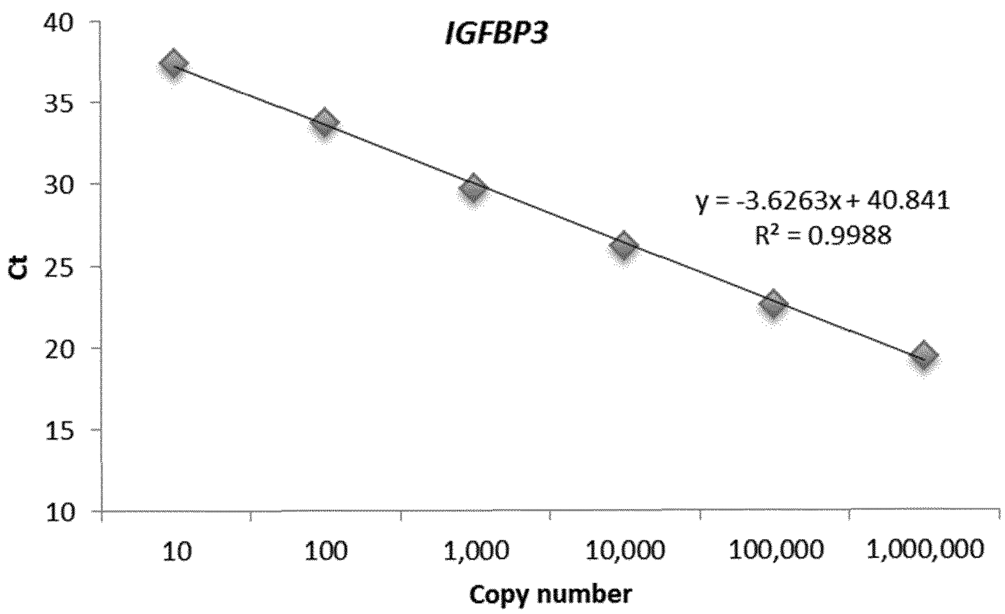


Figure 5 (cont.)

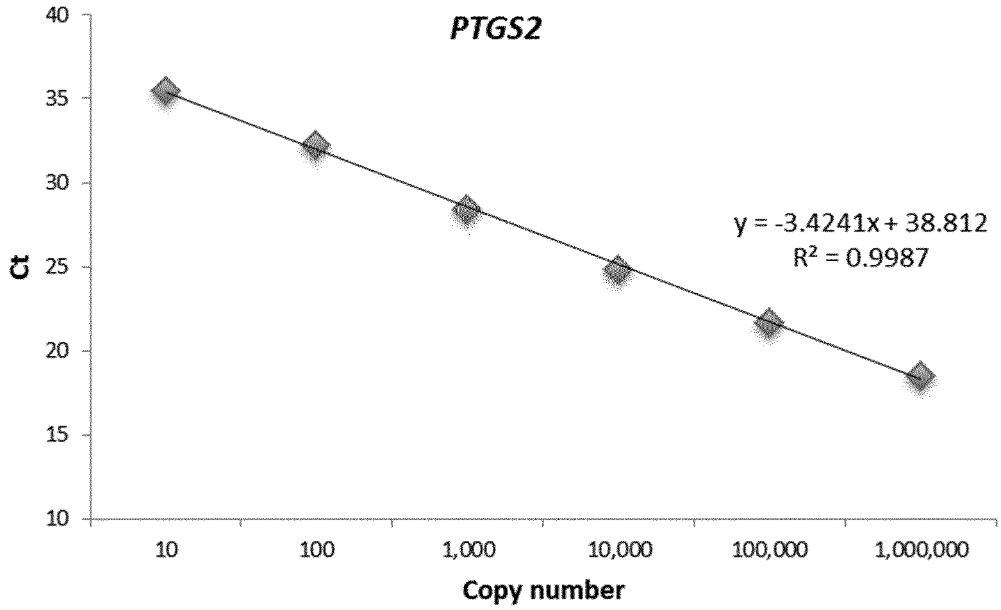


Figure 5 (cont.)

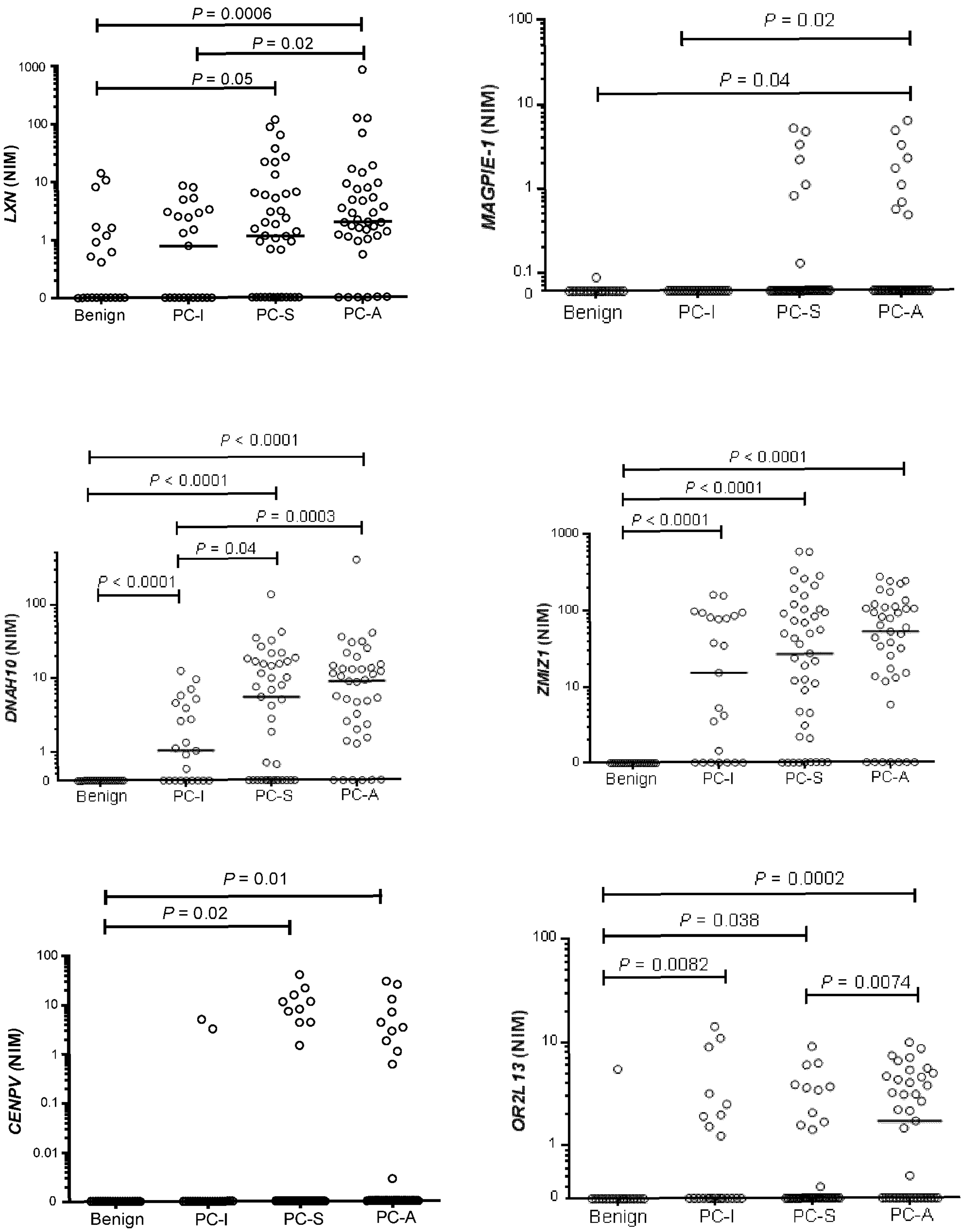
P = 0.0006
P = 0.02
P = 0.05
LXN (NIM)
1000
100
10
1
0
Benign
PC-I
PC-S
PC-A
P = 0.02
P = 0.04
MAGPIE-1 (NIM)
100
10
1
0.1
0
Benign
PC-I
PC-S
PC-A
P < 0.0001
P < 0.0001
P = 0.0003
P = 0.04
P < 0.0001
DNAH10 (NIM)
100
10
1
0
Benign
PC-I
PC-S
PC-A
P < 0.0001
P < 0.0001
P < 0.0001
ZMIZ1 (NIM)
1000
100
10
0
Benign
PC-I
PC-S
PC-A
P = 0.01
P = 0.02
CENPV (NIM)
100
10
1
0.1
0.01
0
Benign
PC-I
PC-S
PC-A
P = 0.0002
P = 0.038
P = 0.0082
P = 0.0074
OR2L13 (NIM)
100
10
1
0
Benign
PC-I
PC-S
PC-A

Figure 6

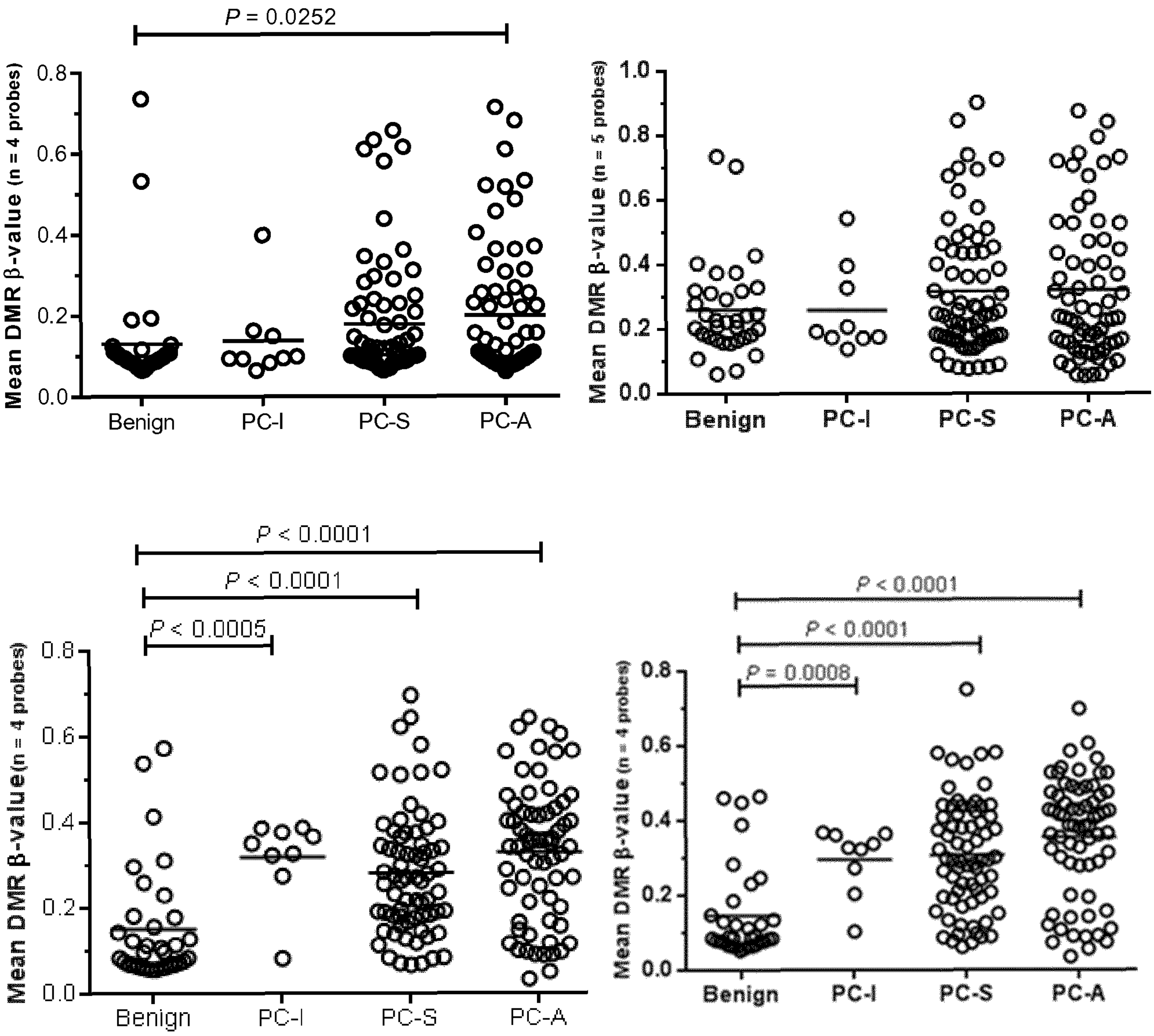


Figure 6 (cont.)

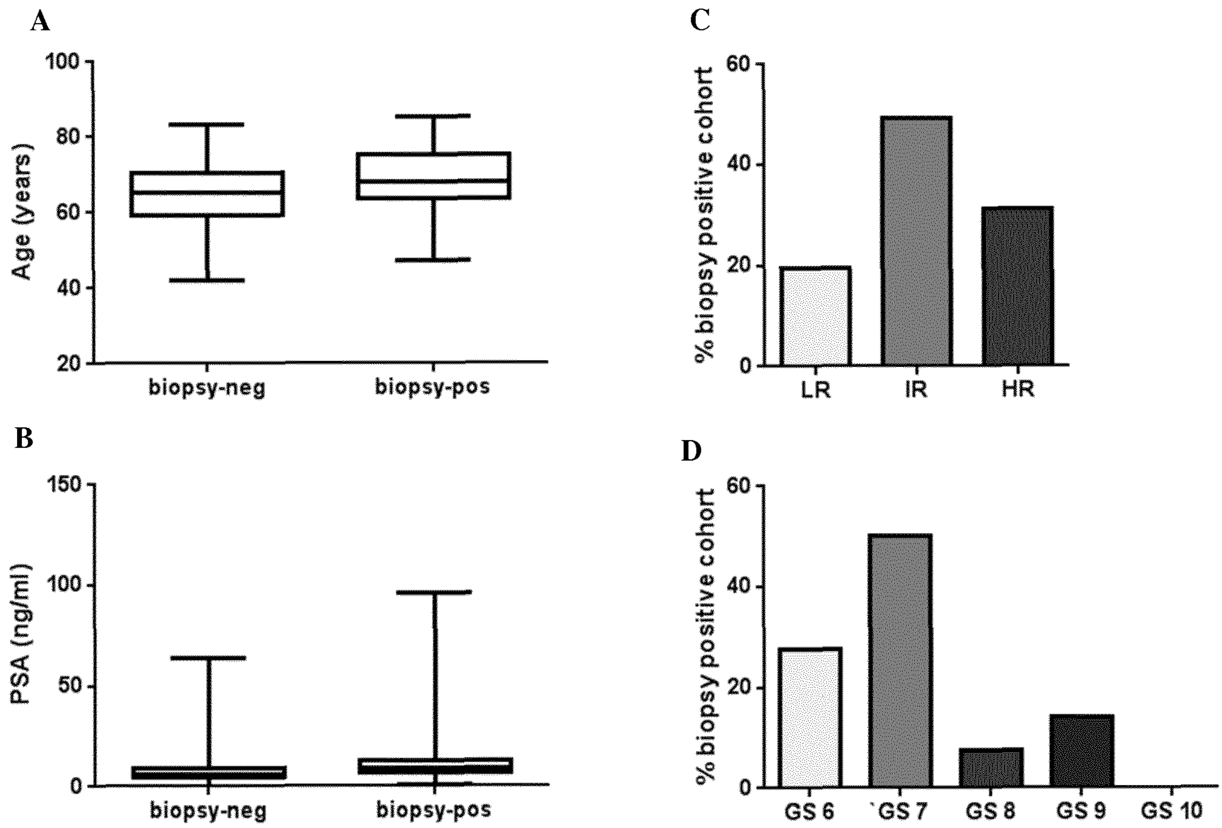
A
100
80
60
40
20
Age (years)
biopsy-neg
biopsy-pos
B
150
100
50
0
PSA (ng/ml)
biopsy-neg
biopsy-pos
C
60
40
20
0
% biopsy positive cohort
LR
IR
HR
D
60
40
20
0
% biopsy positive cohort
GS 6
`GS 7
GS 8
GS 9
GS 10

Figure 7

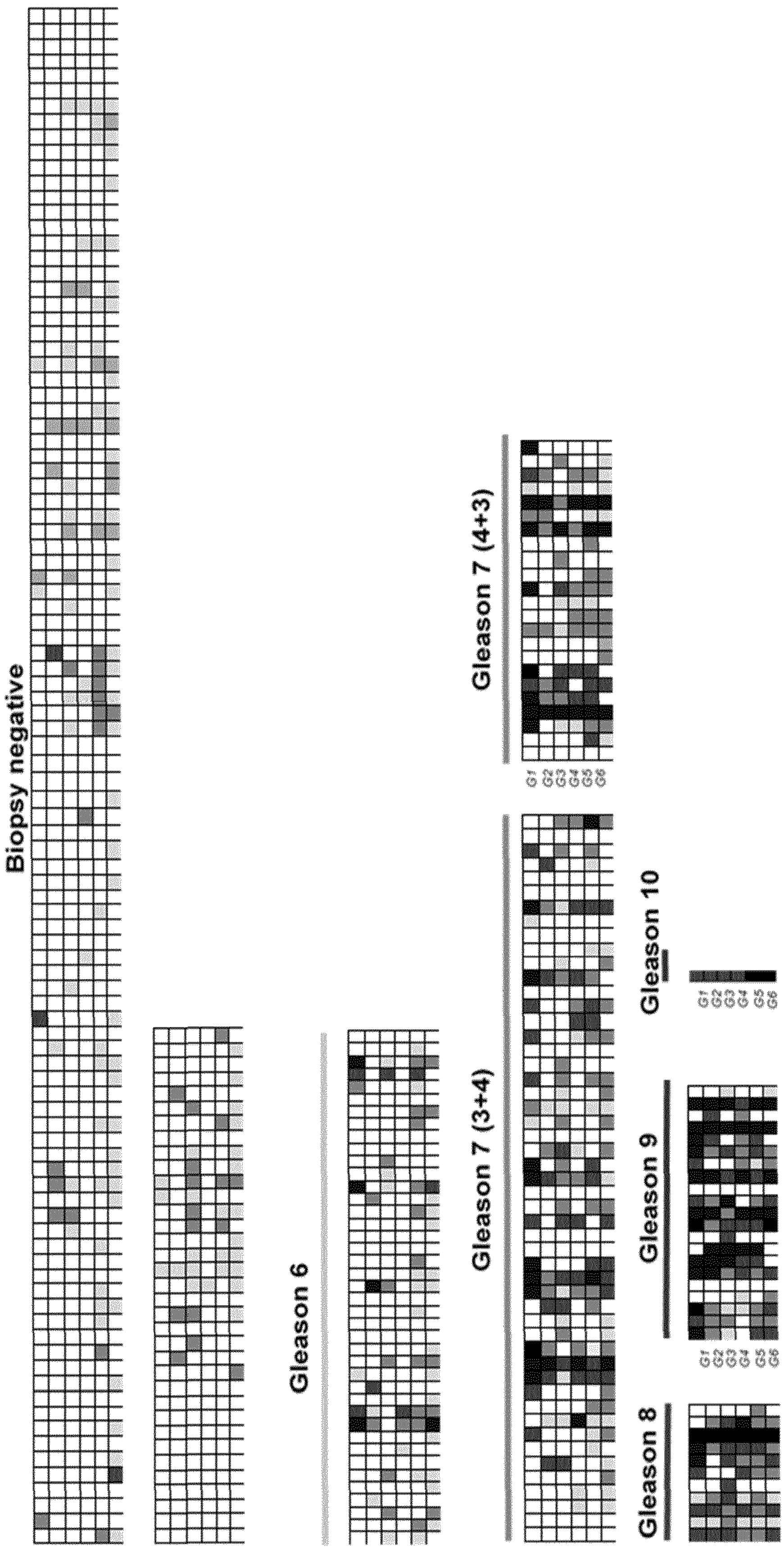
Biopsy negative
Gleason 6
Gleason 7 (3+4)
Gleason 7 (4+3)
G1 G2 G3 G4 G5 G6
Gleason 8
Gleason 9
G1 G2 G3 G4 G5 G6
Gleason 10
G1 G2 G3 G4 G5 G6

Figure 8

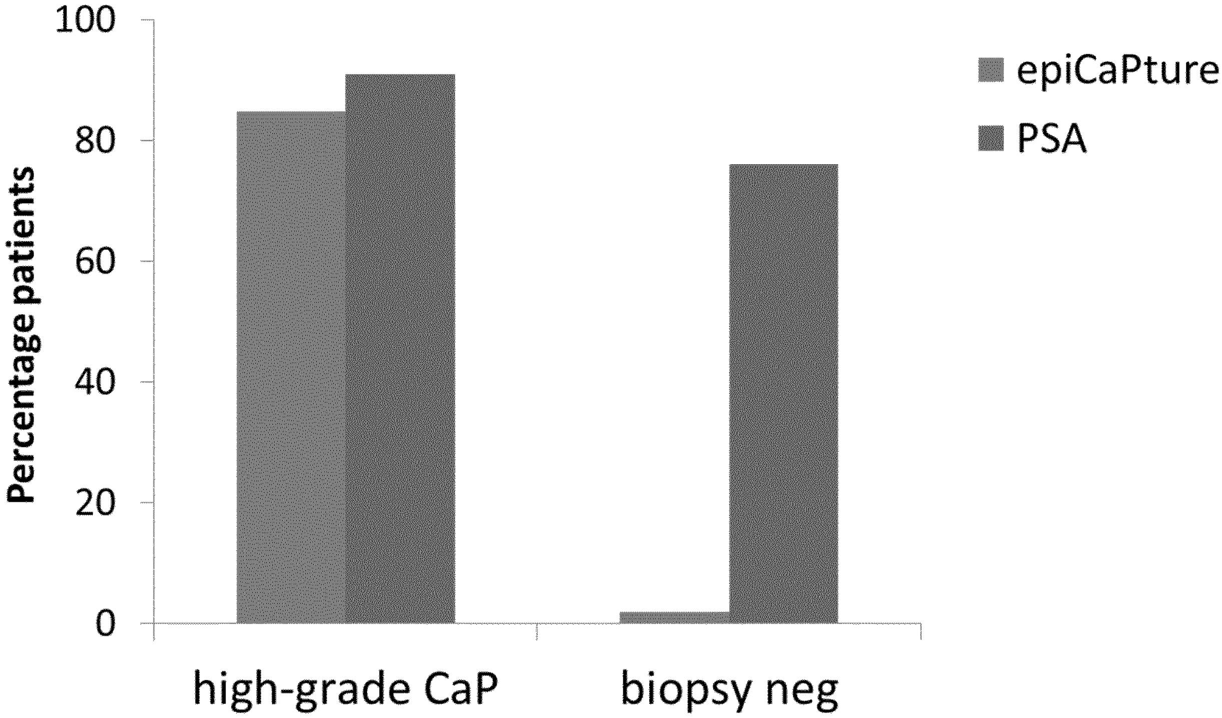
100
80
60
40
20
0
Percentage patients
high-grade CaP
biopsy neg
epiCaPture
PSA

Figure 9

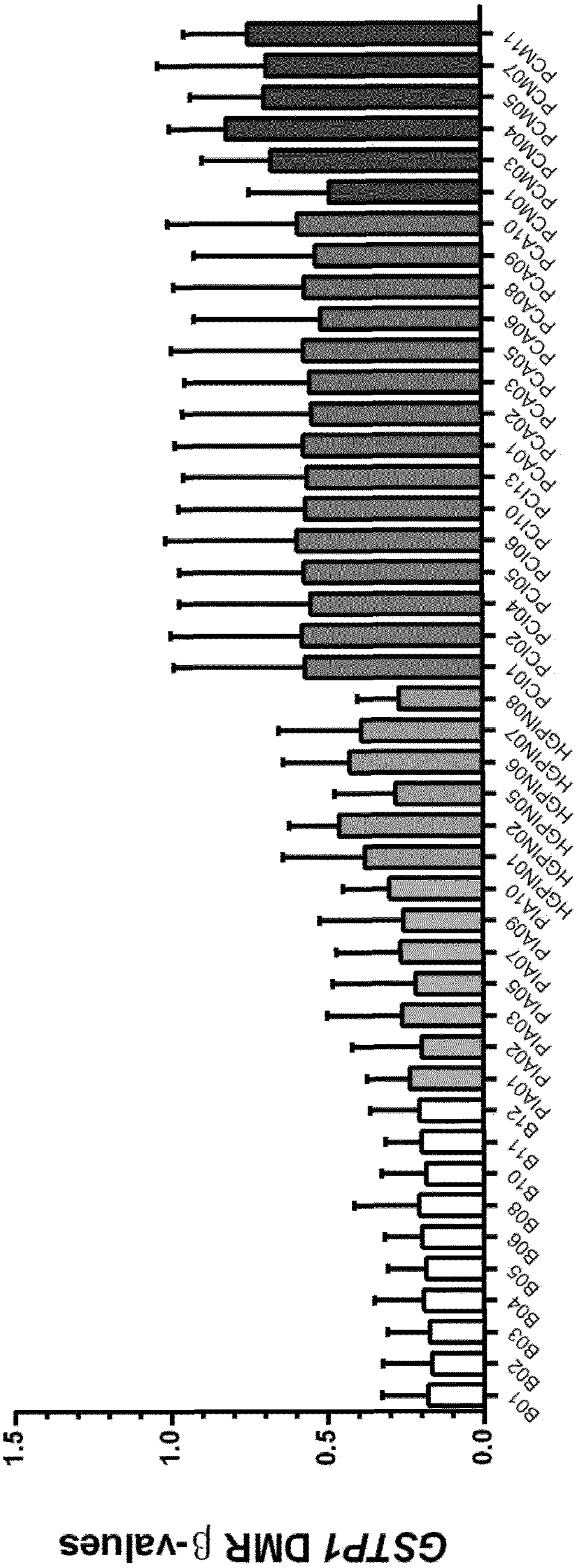
GSTP1 DMR β-values
0.0
0.5
1.0
1.5
B01
B02
B03
B04
B05
B06
B08
B10
B11
B12
PIA01
PIA02
PIA03
PIA05
PIA07
PIA09
PIA10
HGPIN01
HGPIN02
HGPIN05
HGPIN06
HGPIN07
HGPIN08
PCI01
PCI02
PCI04
PCI05
PCI06
PCI10
PCI13
PCA01
PCA02
PCA03
PCA05
PCA06
PCA08
PCA09
PCA10
PCM01
PCM03
PCM04
PCM05
PCM07
PCM11

Figure 10A

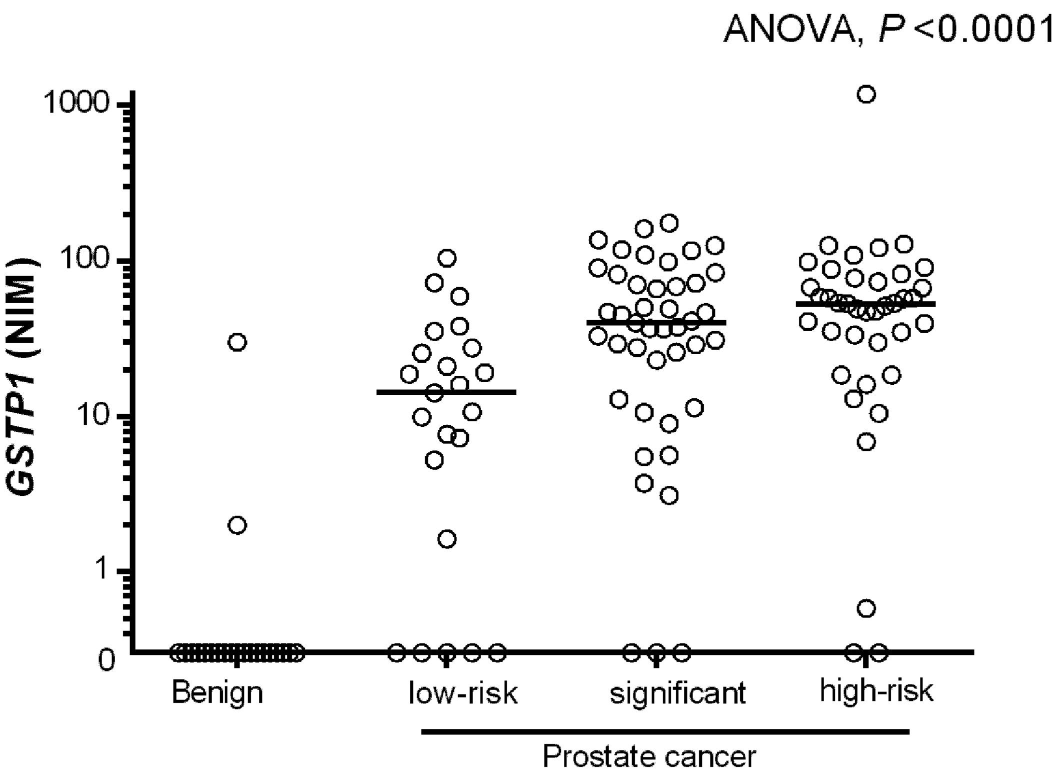


Figure 10B

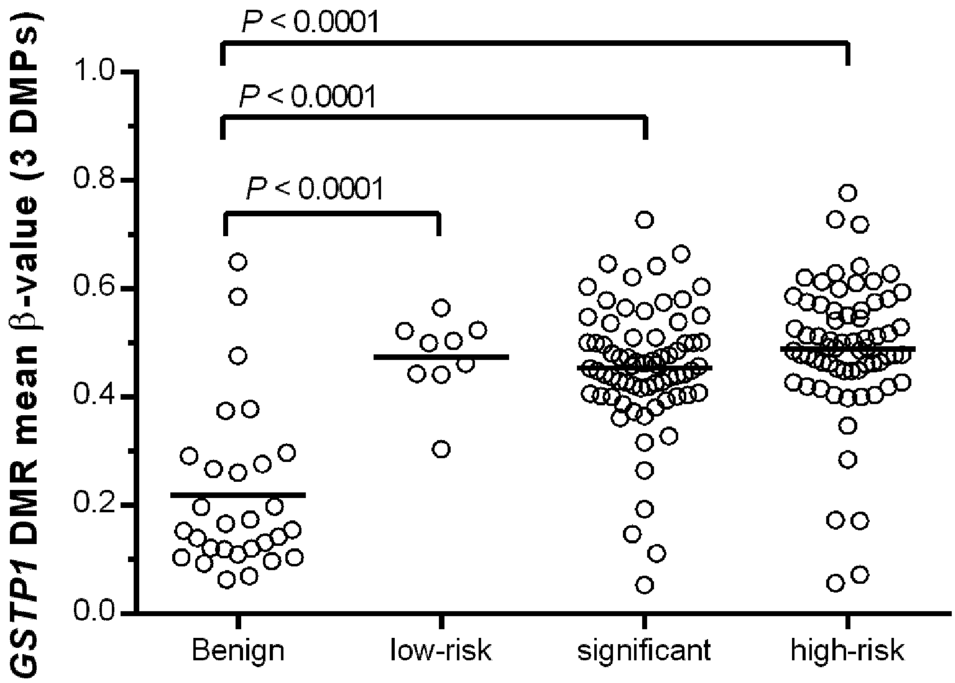


Figure 10C

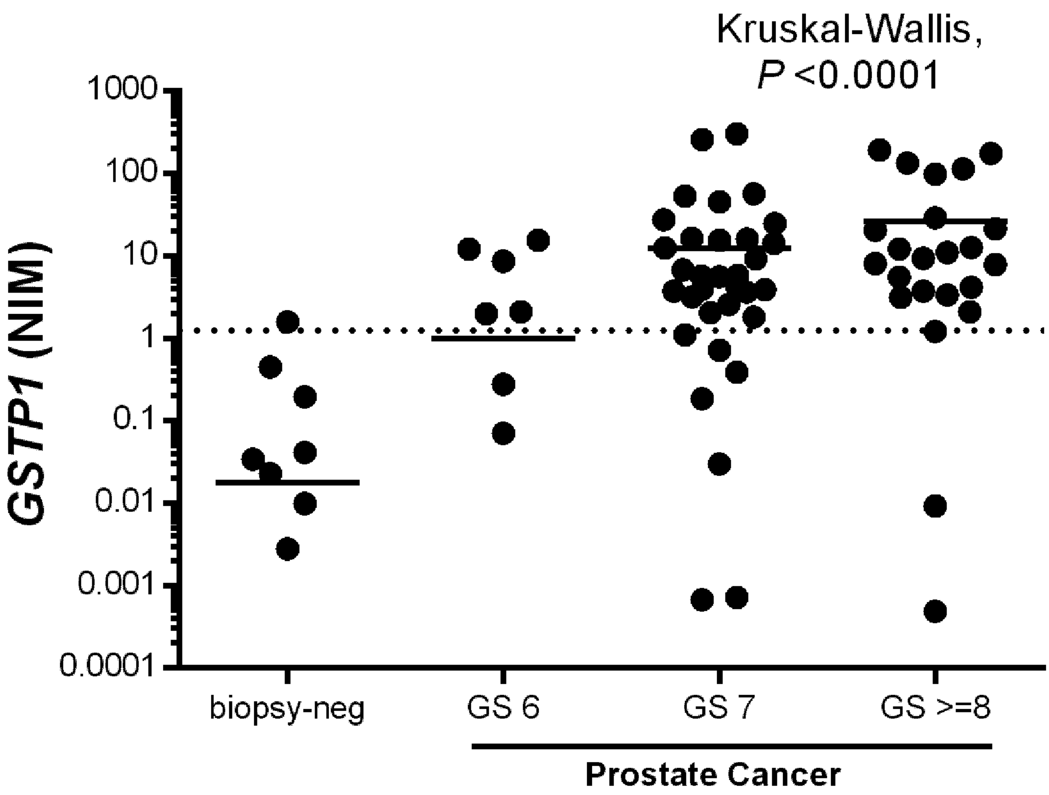
Kruskal-Wallis,
P <0.0001
1000
100
10
1
0.1
0.01
0.001
0.0001
GSTP1 (NIM)
biopsy-neg
GS 6
GS 7
GS >=8
Prostate Cancer

Figure 10D

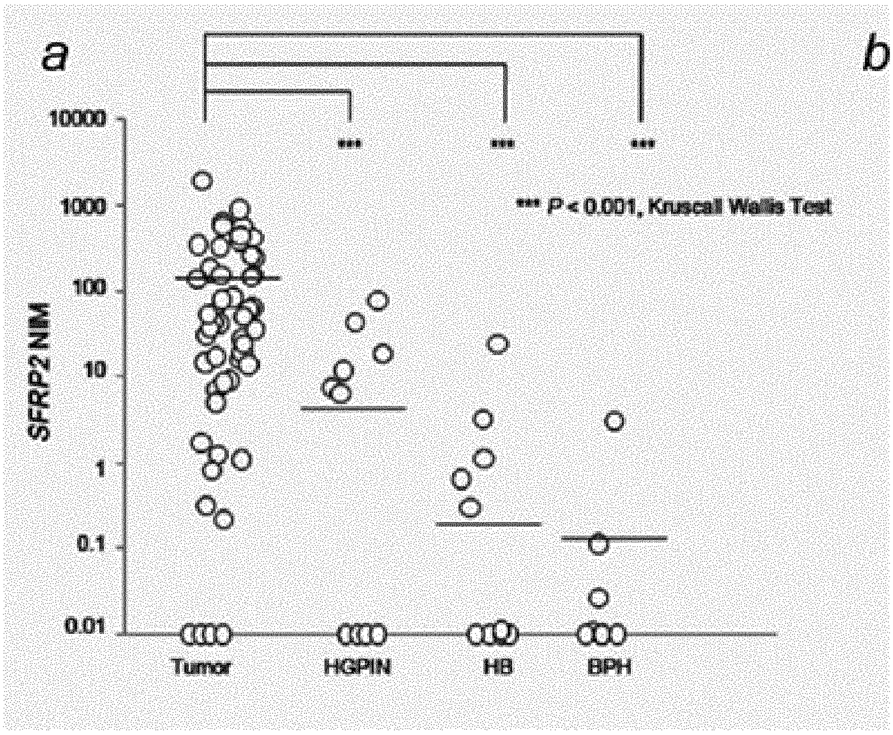
a
b
10000
1000
100
10
1
0.1
0.01
SFRP2 NIM
***
*** P < 0.001, Kruscall Wallis Test
Tumor
HGPIN
HB
BPH

A

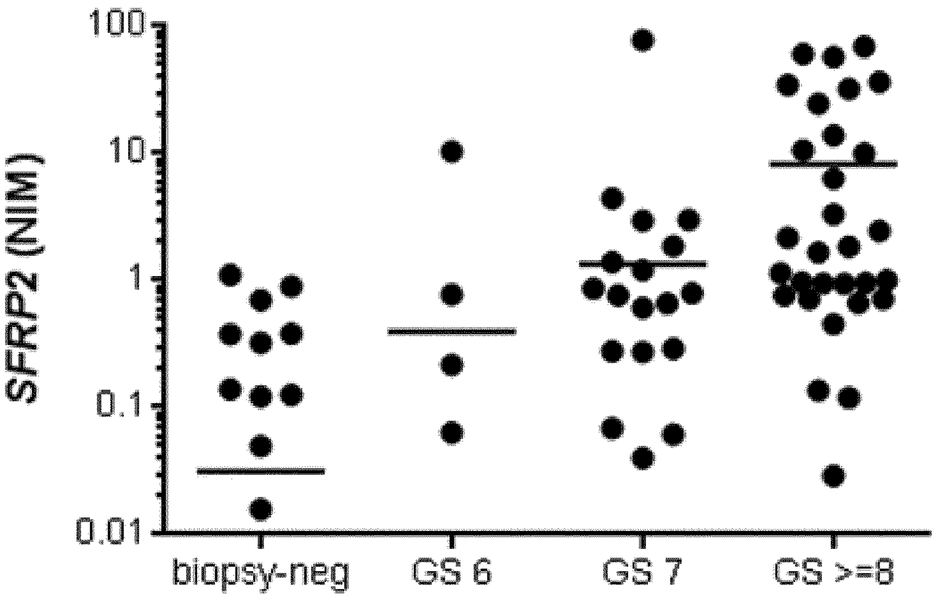
100
10
1
0.1
0.01
SFRP2 (NIM)
biopsy-neg
GS 6
GS 7
GS >=8

B

Figure 11

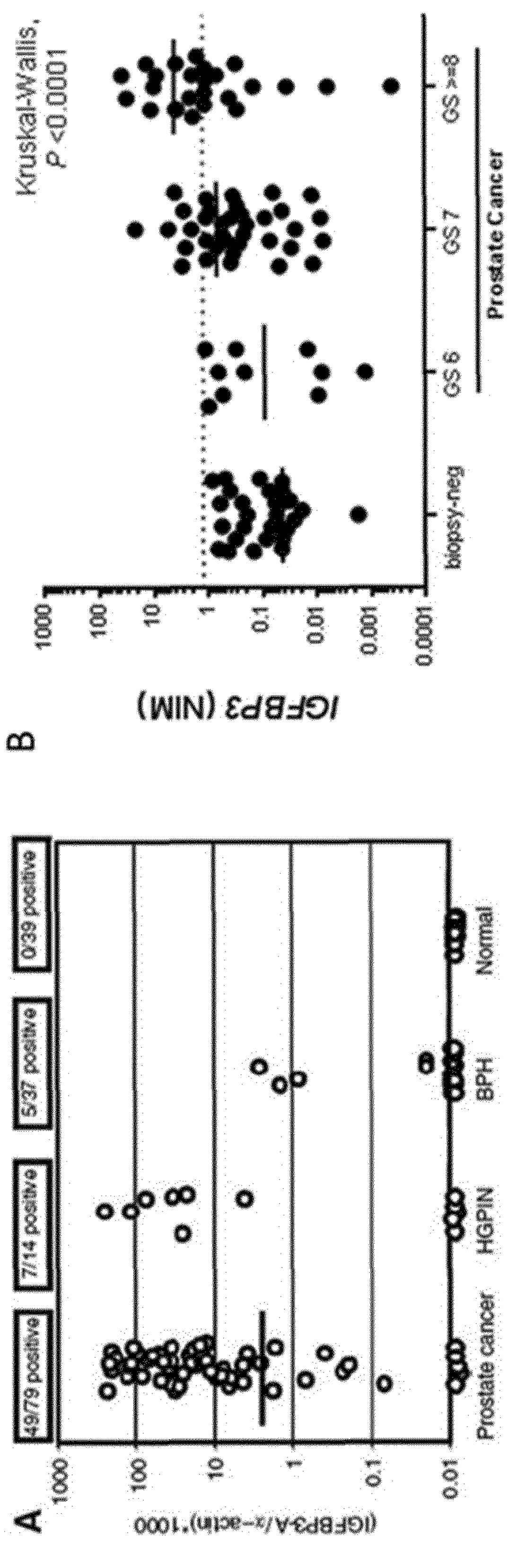
A
49/79 positive
7/14 positive
5/37 positive
0/39 positive
(IGFBP3-A/x-actin)*1000
1000
100
10
1
0.1
0.01
Prostate cancer
HGPIN
BPH
Normal
B
IGFBP3 (NIM)
Kruskal-Wallis,
P <0.0001
1000
100
10
1
0.1
0.01
0.001
0.0001
biopsy-neg
GS 6
GS 7
GS >=8
Prostate Cancer

Figure 12

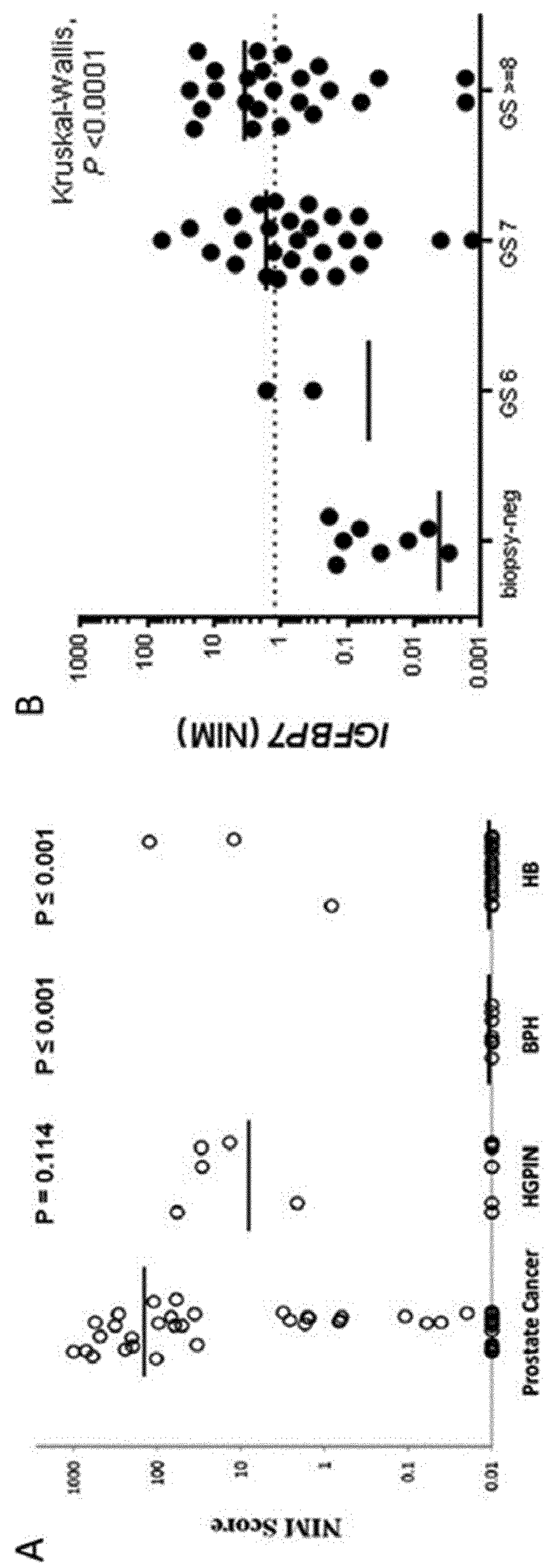
A
NIM Score
1000
100
10
1
0.1
0.01
Prostate Cancer
HGPIN
BPH
HB
P = 0.114
P ≤ 0.001
P ≤ 0.001
B
IGFBP7 (NIM)
1000
100
10
1
0.1
0.01
0.001
biopsy-neg
GS 6
GS 7
GS >=8
Kruskal-Wallis,
P <0.0001

Figure 13

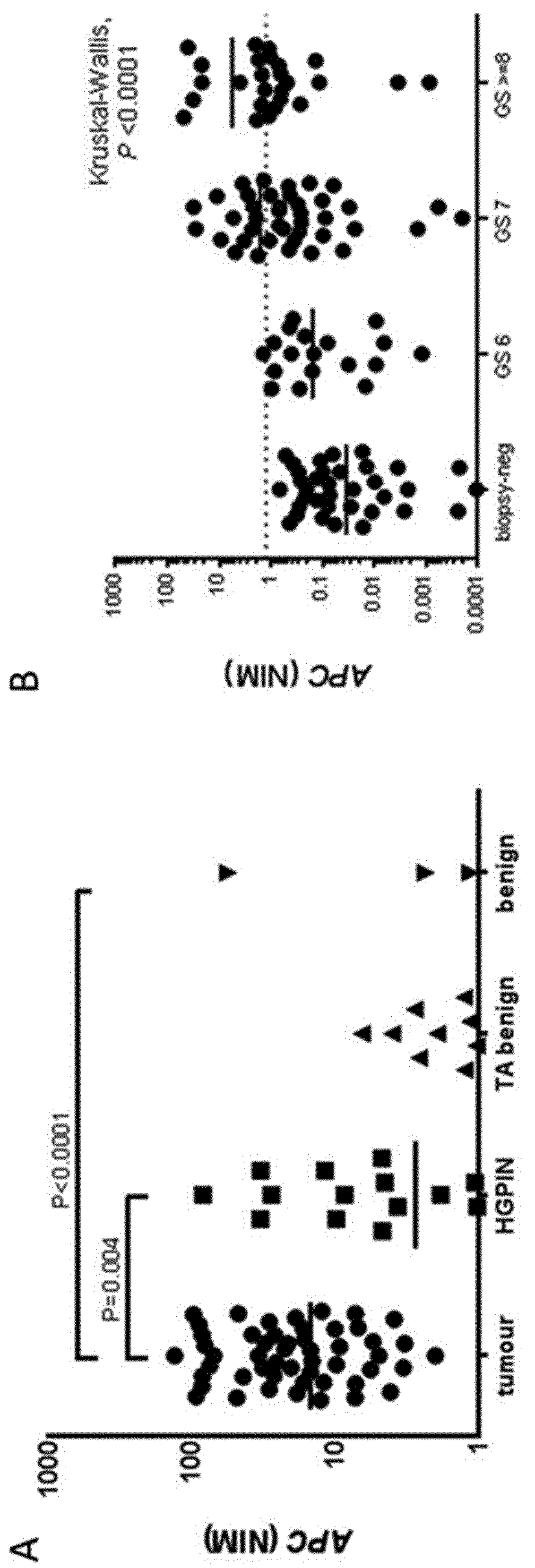
A
APC (NIM)
1000
100
10
1
tumour
HGPIN
TA benign
benign
P=0.004
P<0.0001
B
APC (NIM)
1000
100
10
1
0.1
0.01
0.001
0.0001
biopsy-neg
GS 6
GS7
GS >=8
Kruskal-Wallis,
P <0.0001

Figure 14

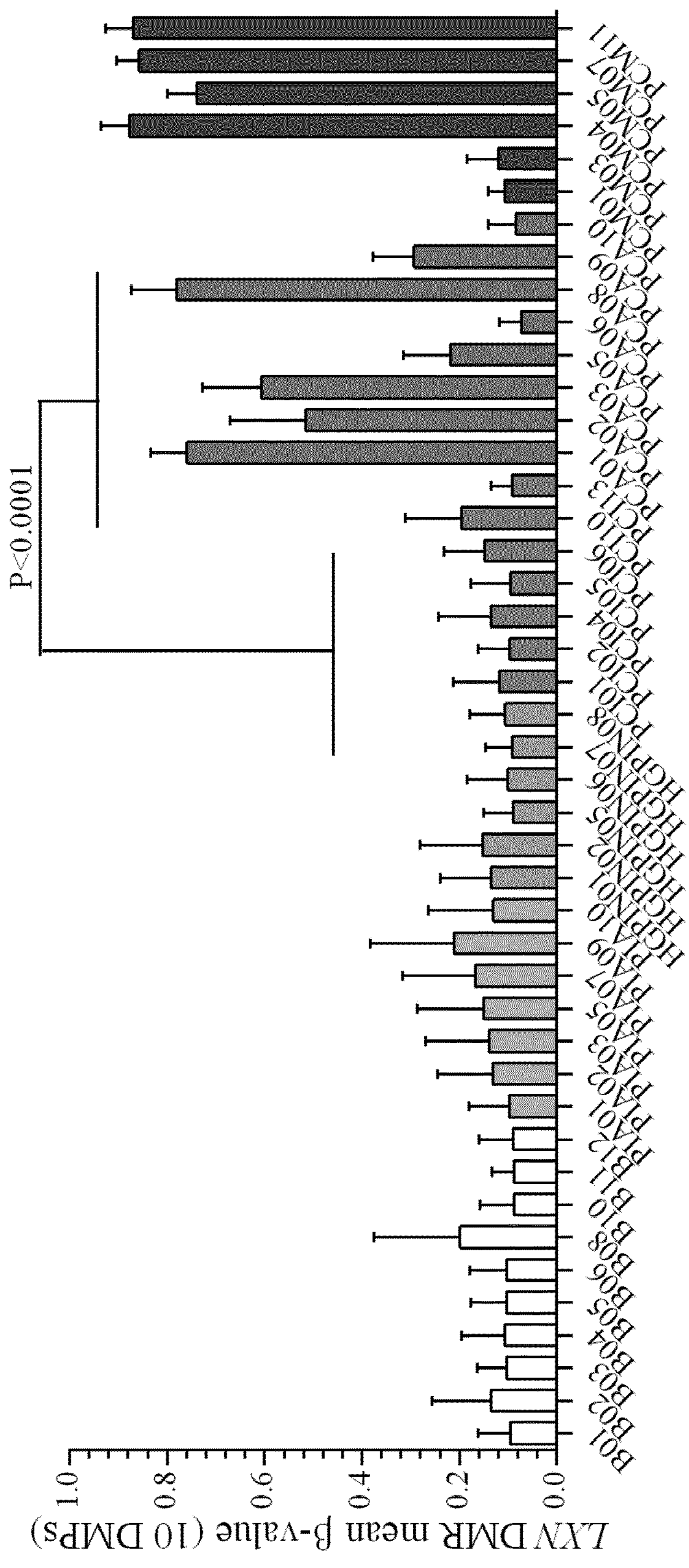


Figure 15A

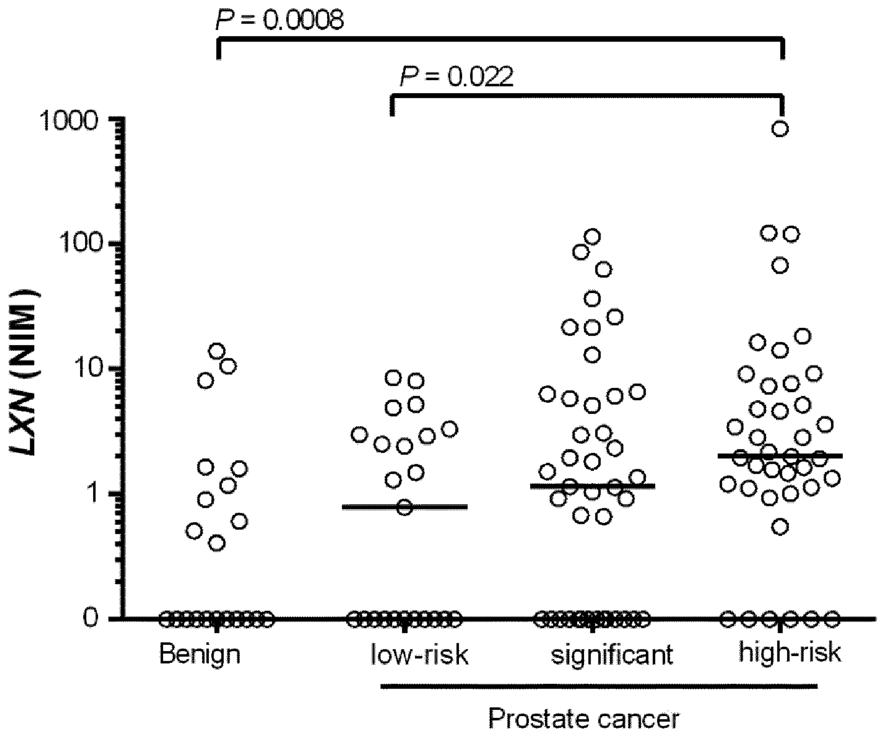


Figure 15B

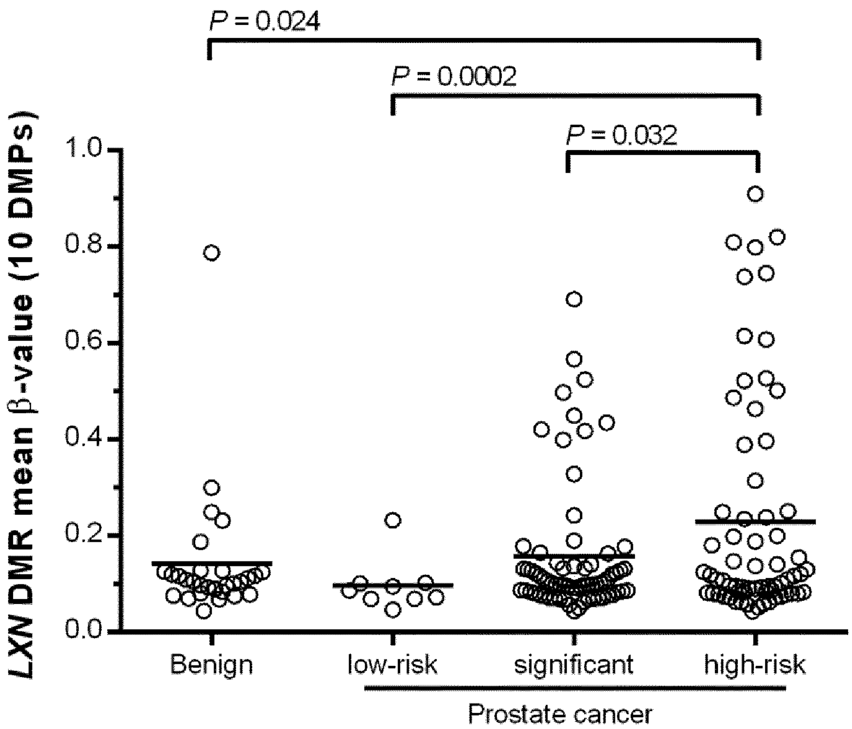


Figure 15C

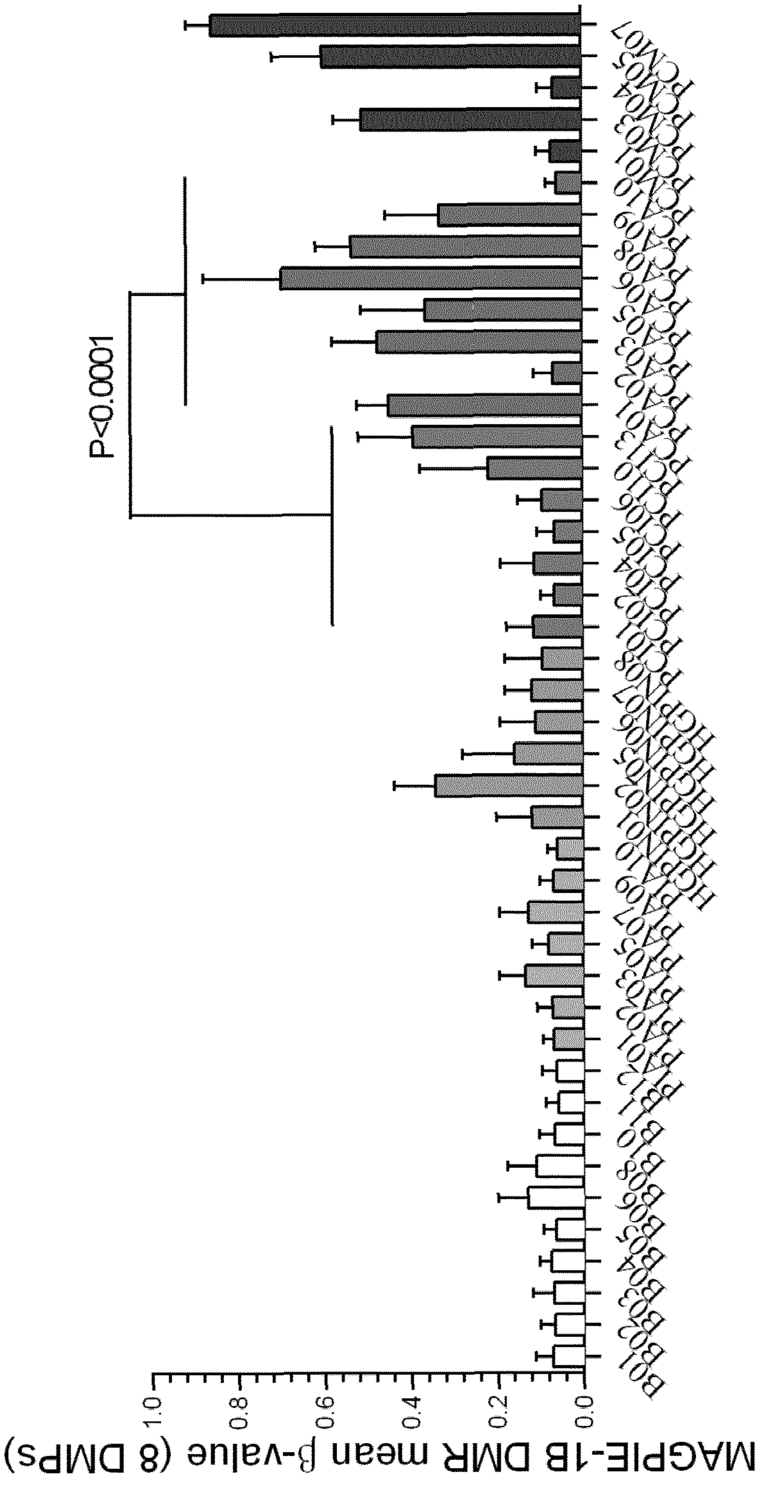


Figure 16A

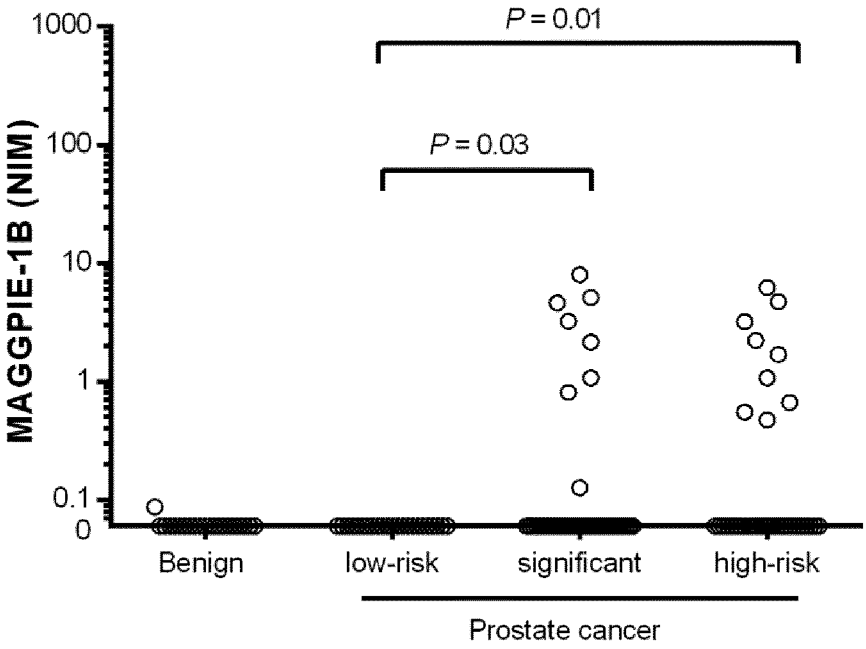


Figure 16B

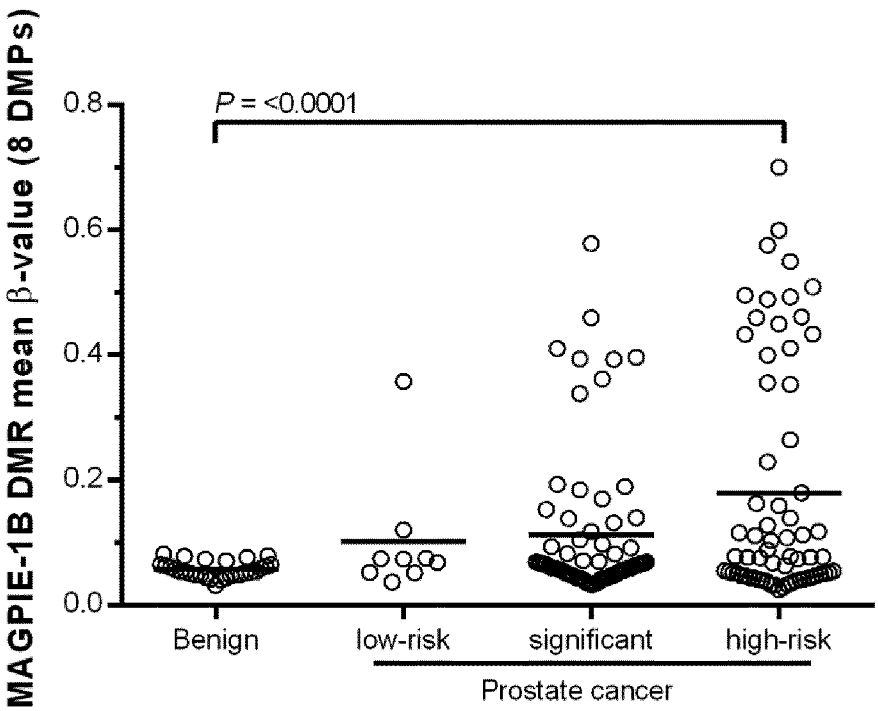


Figure 16C

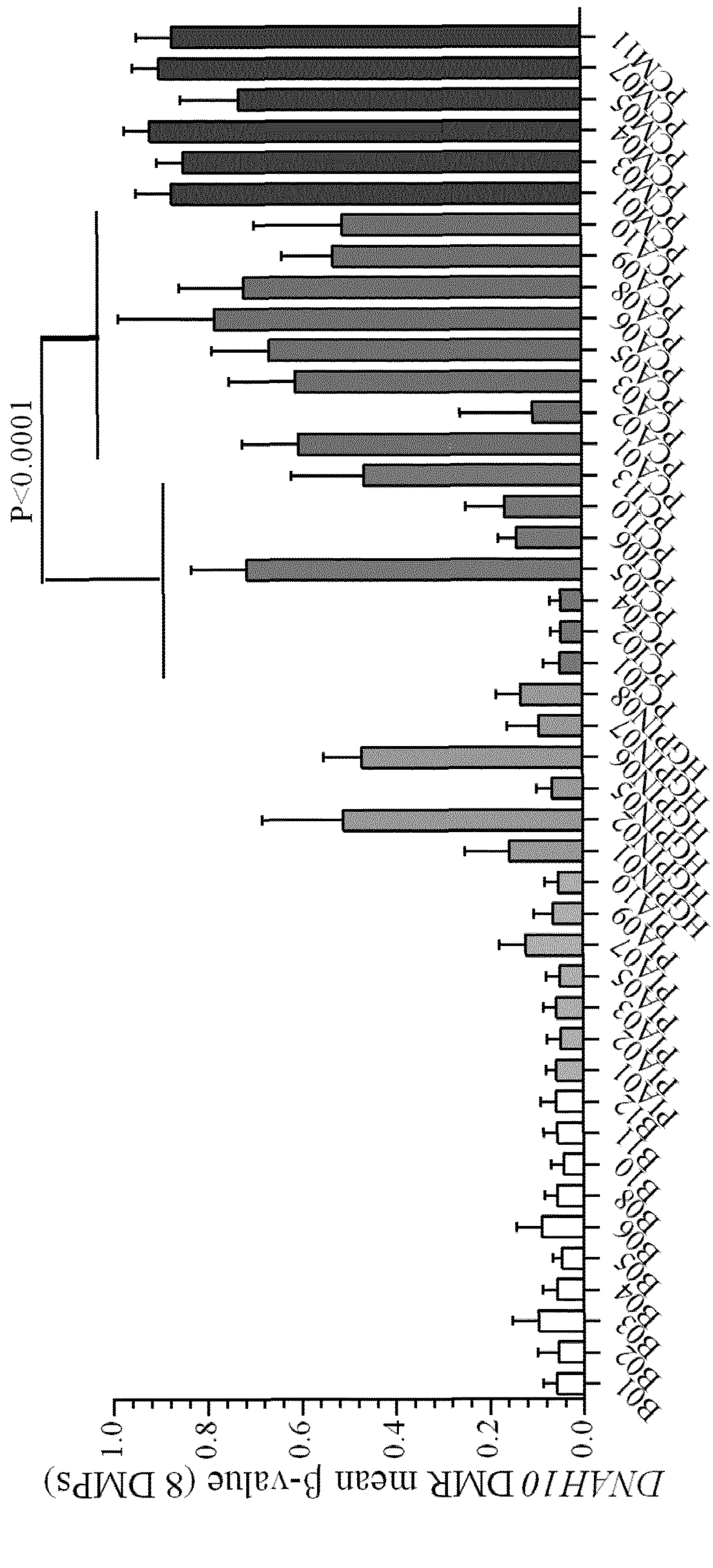


Figure 17A

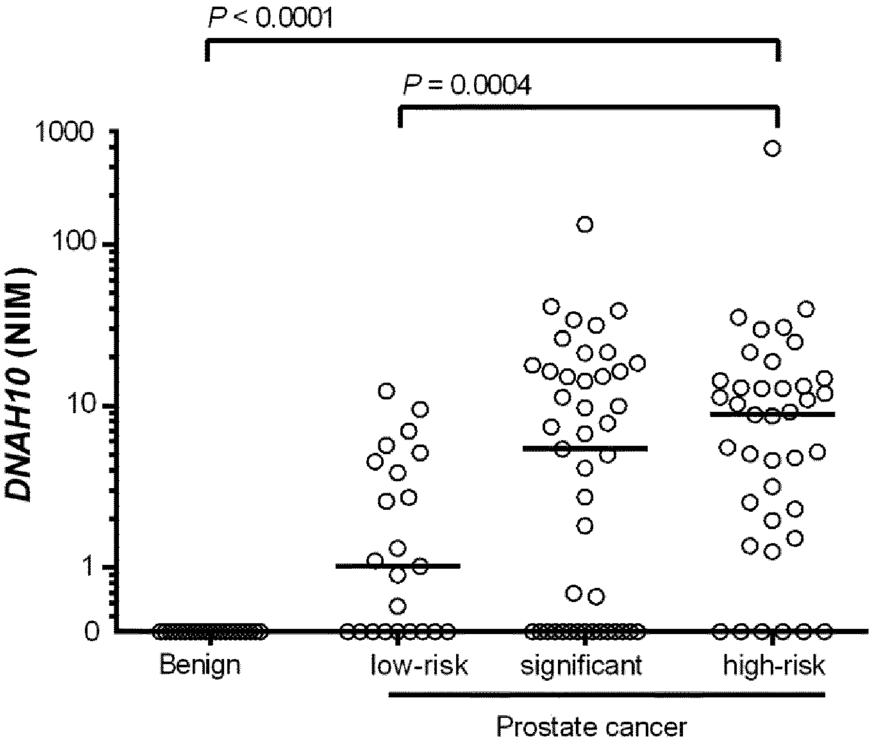


Figure 17B

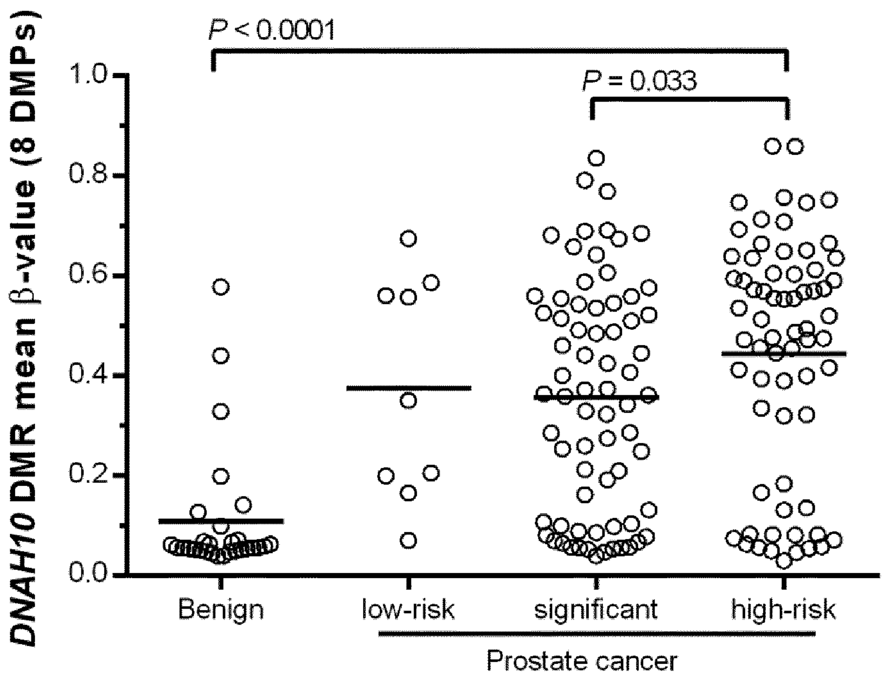


Figure 17C

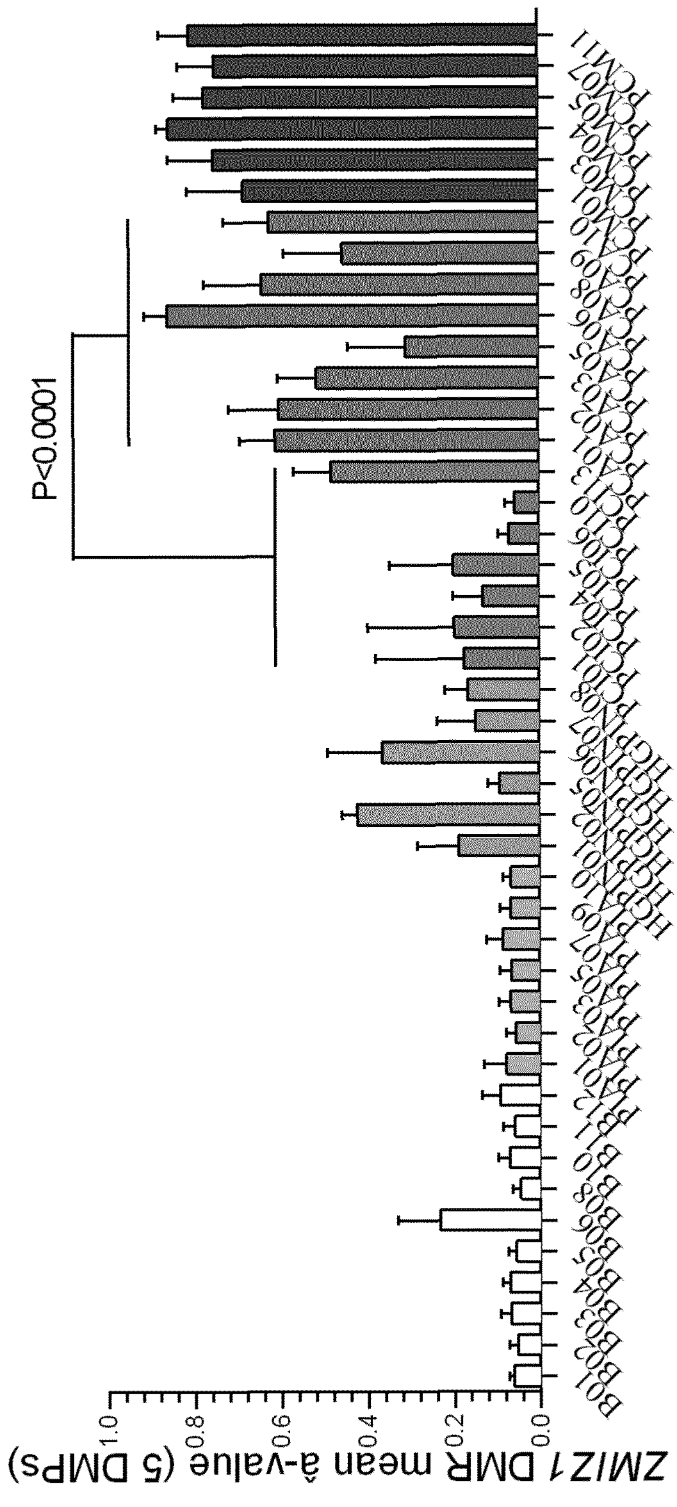


Figure 18A

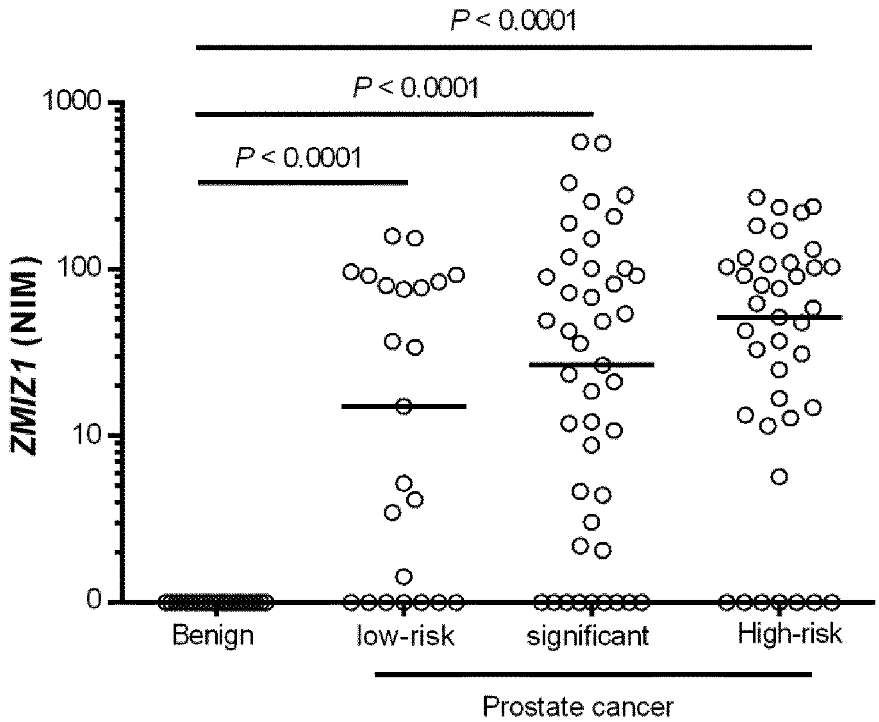


Figure 18B

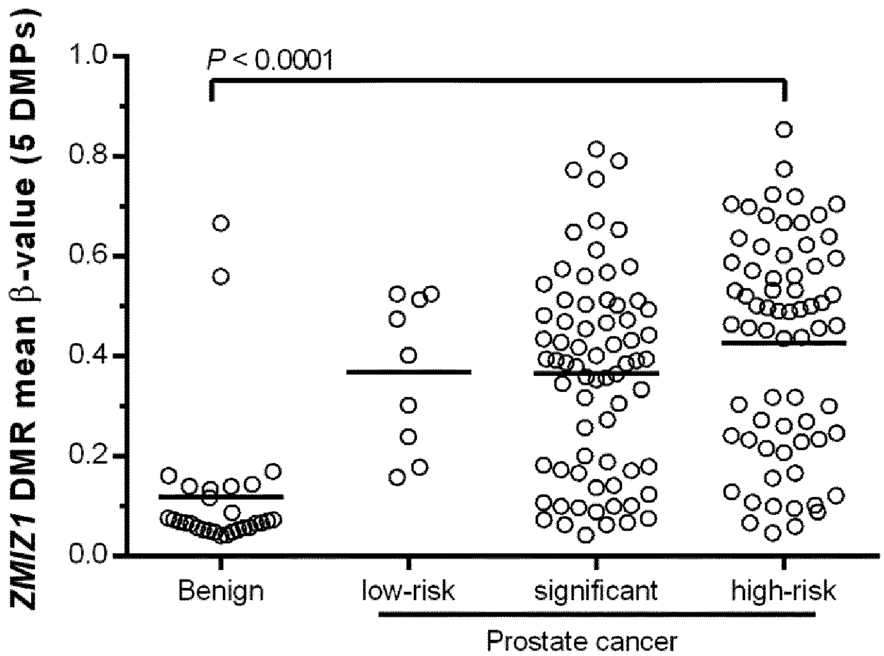


Figure 18C

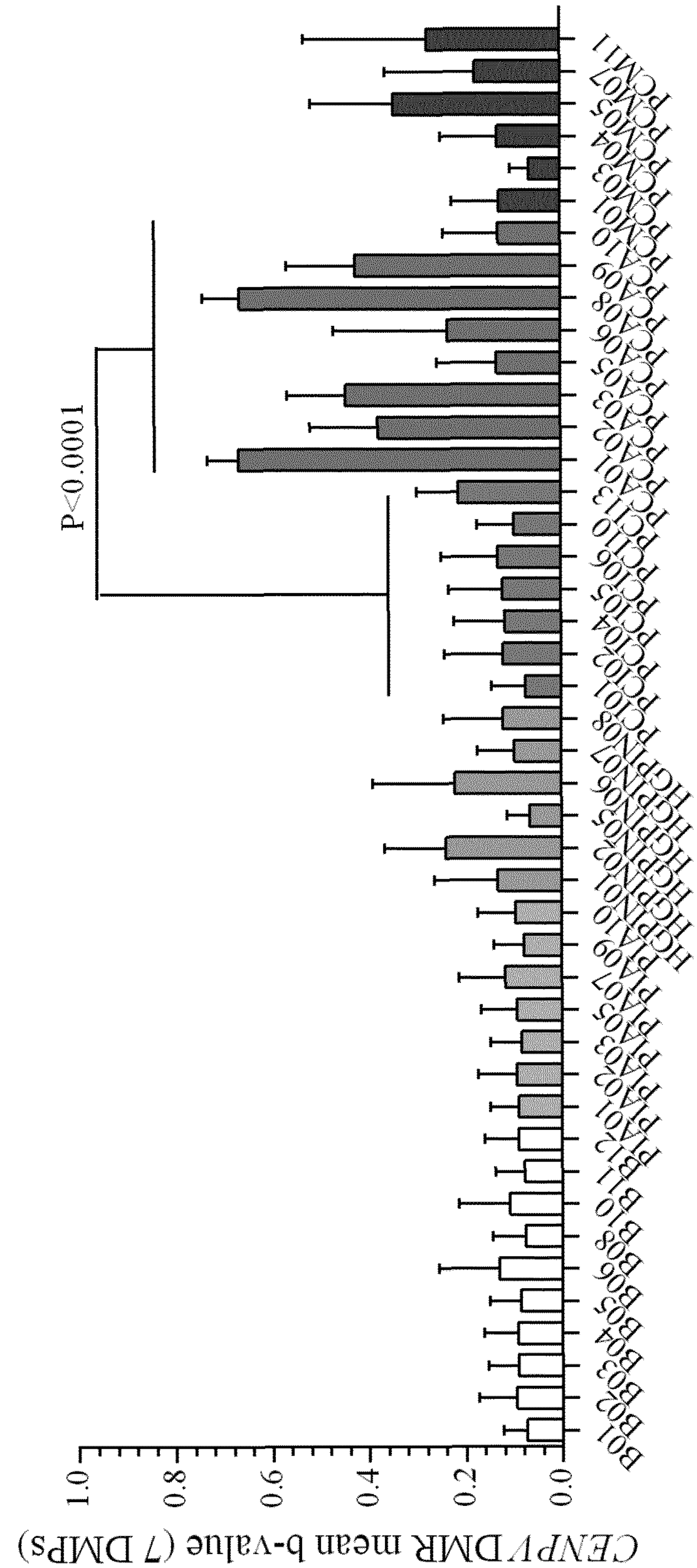


Figure 19A

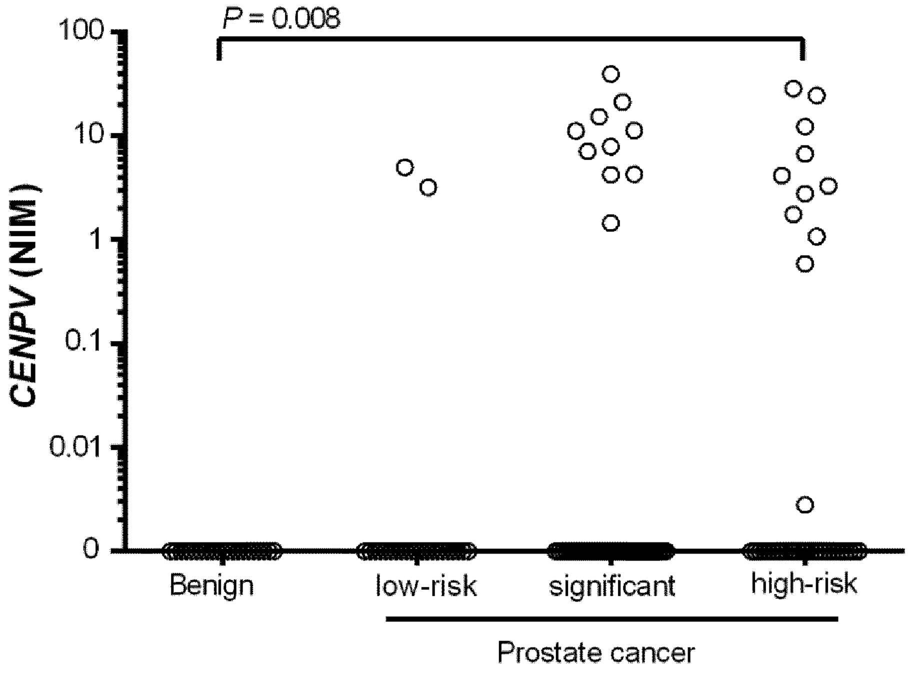


Figure 19B

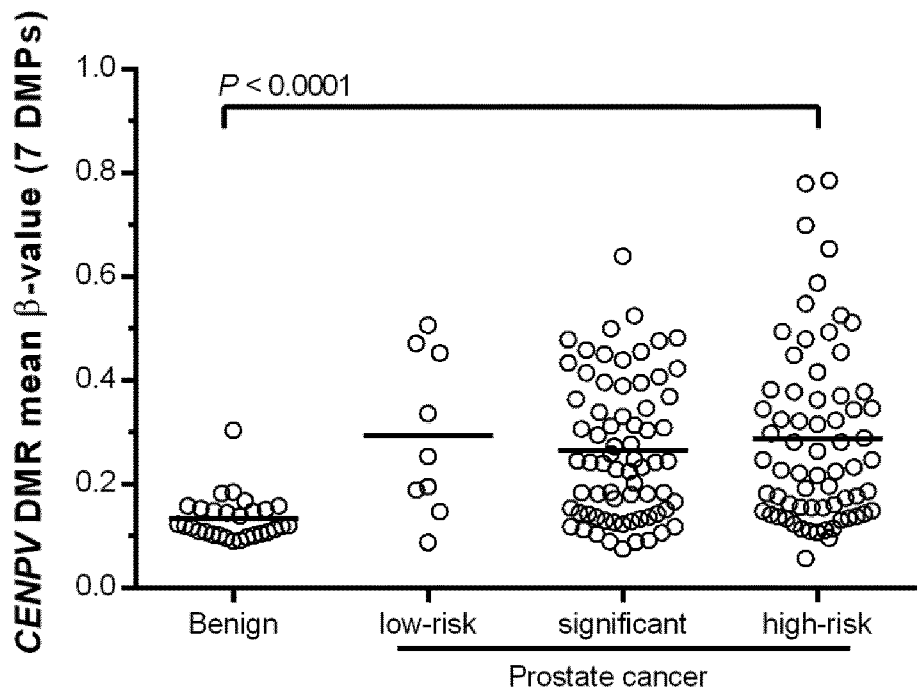


Figure 19C

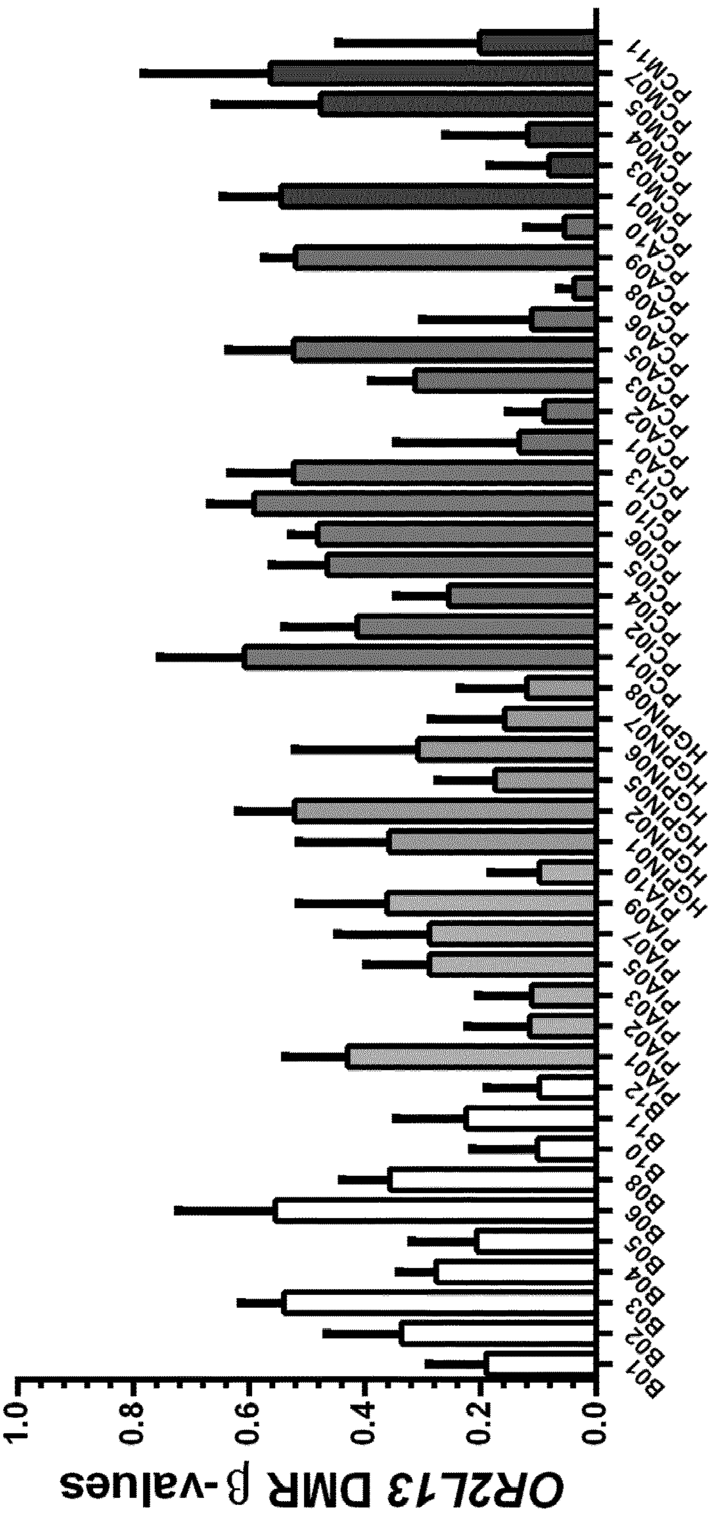
OR2L13 DMR β-values
1.0
0.8
0.6
0.4
0.2
0.0
B01
B02
B03
B04
B05
B06
B08
B10
B11
B12
PIA01
PIA02
PIA03
PIA05
PIA07
PIA09
PIA10
HGPIN01
HGPIN02
HGPIN05
HGPIN06
HGPIN07
HGPIN08
pCI01
pCI02
pCI04
pCI05
pCI06
pCI10
pCI13
pCA01
pCA02
pCA03
pCA05
pCA06
pCA08
pCA09
pCA10
pCM01
pCM03
pCM04
pCM05
pCM07
pCM11

Figure 20A

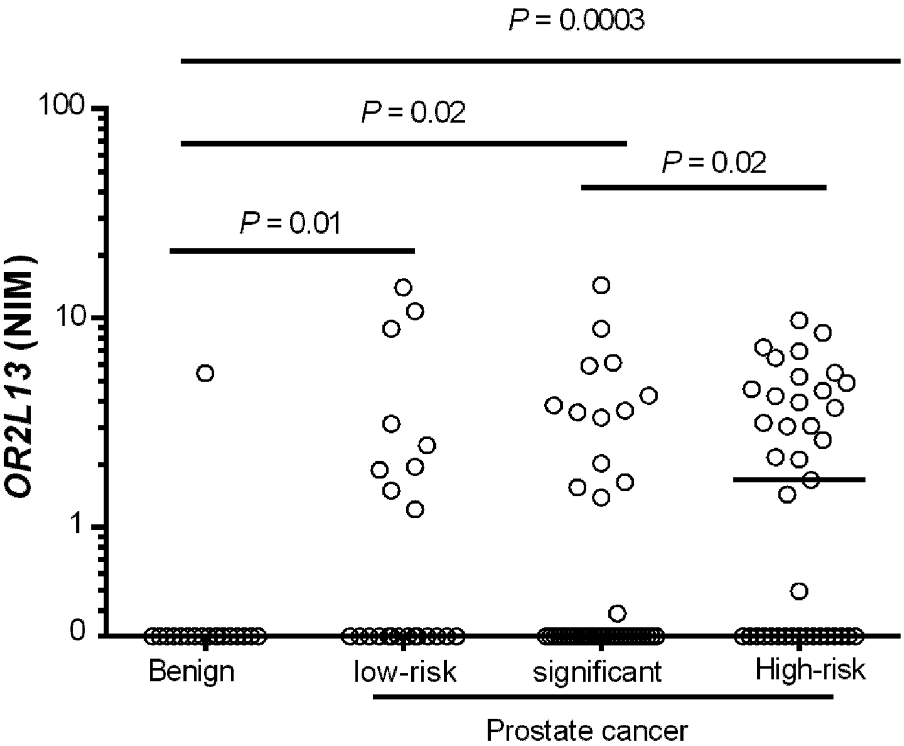


Figure 20B

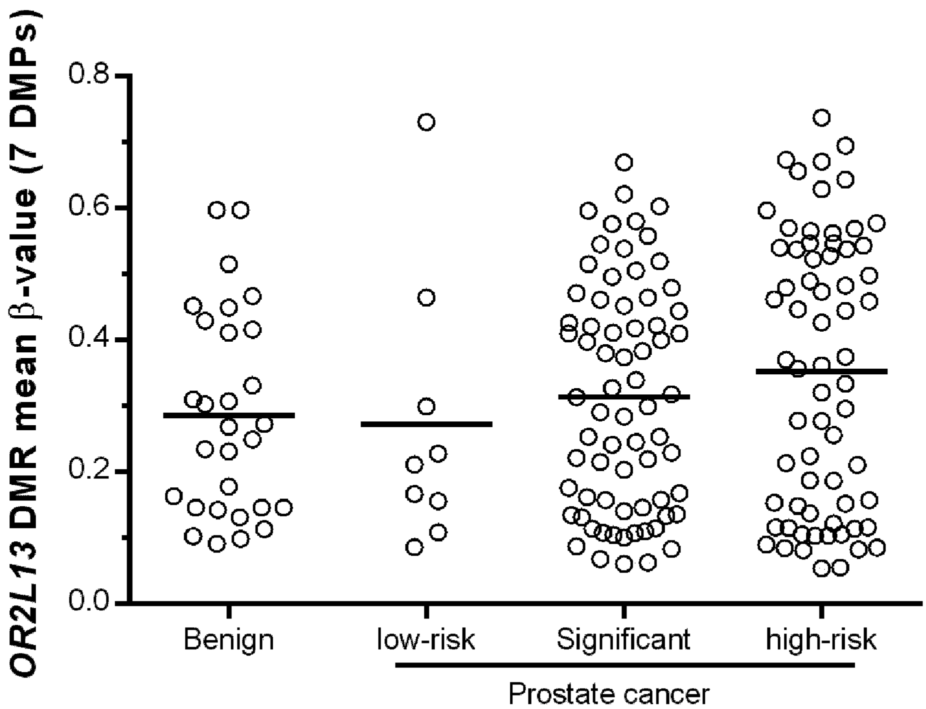


Figure 20C

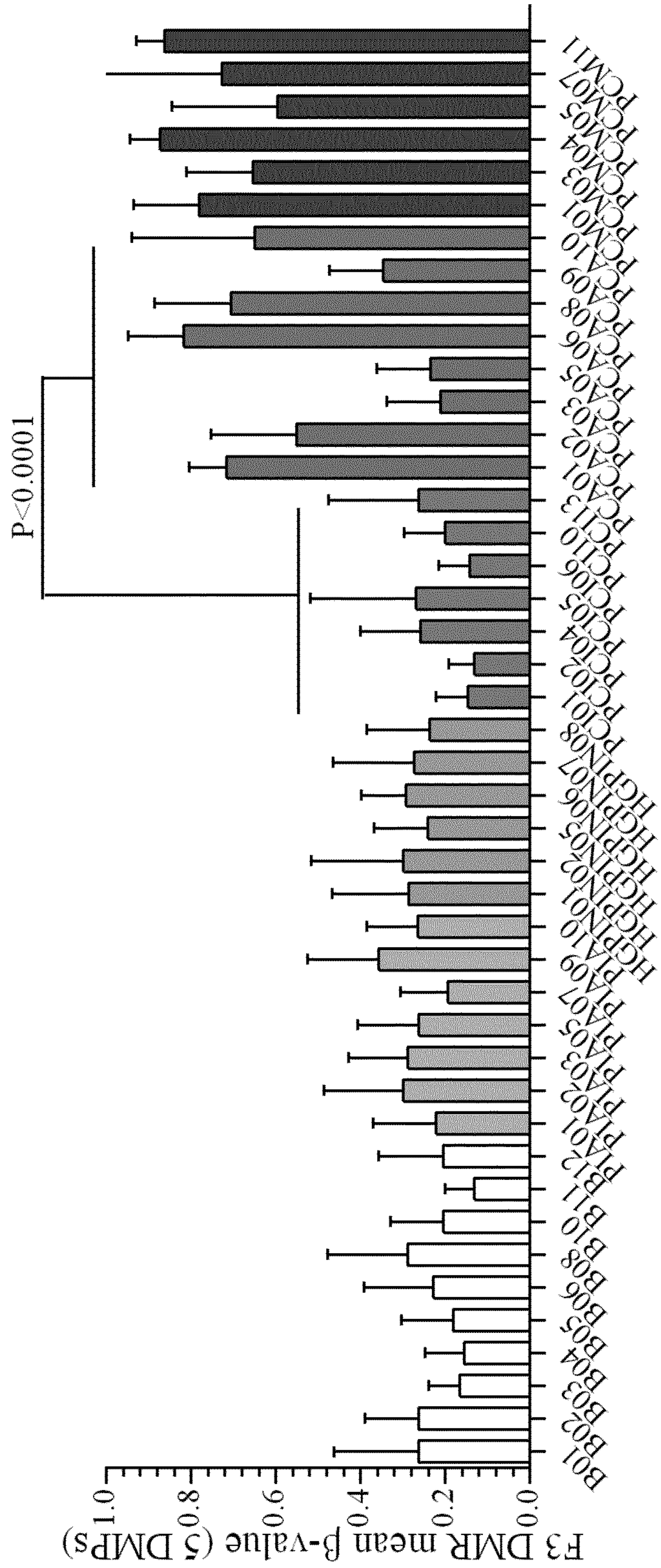


**Figure 21A**

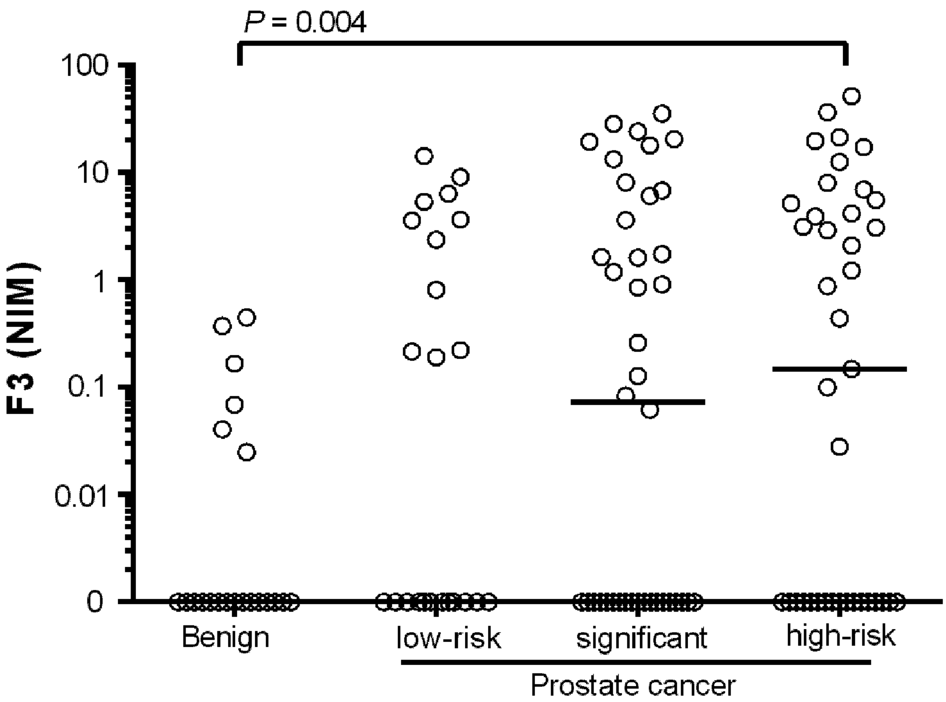


Figure 21B

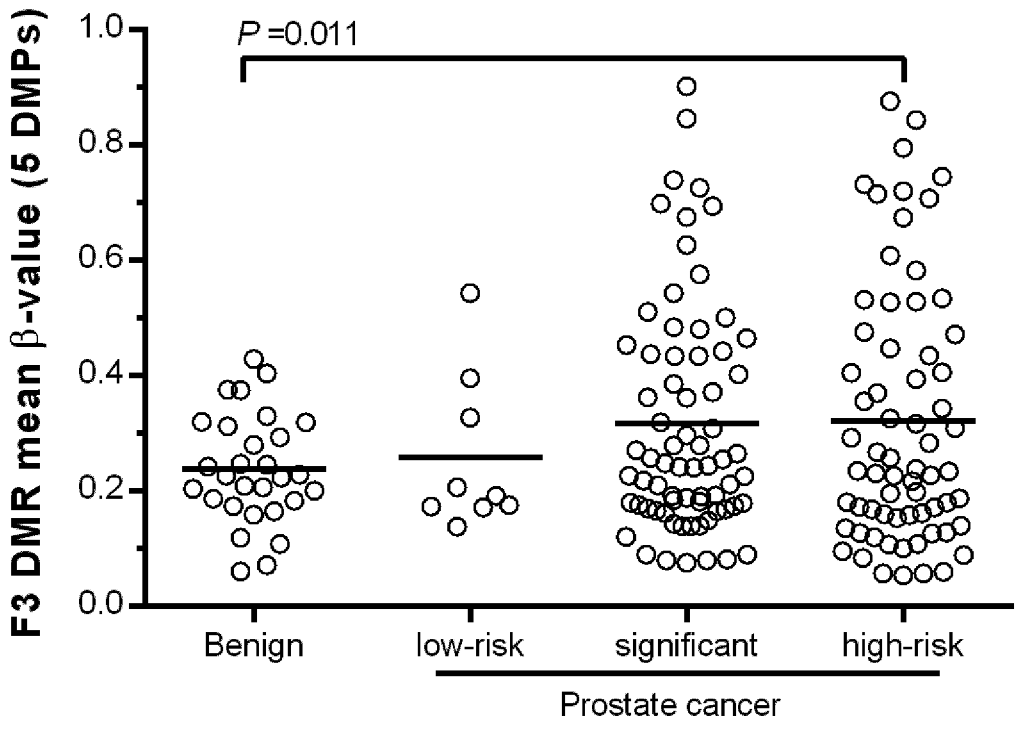


Figure 21C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1918710 A
- WO 2013041731 A
- EP 2213749 A
- US 2014274767 A
- US 5962233 A
- US 5538848 A
- US 5639606 A
- US 5643765 A
- US 5876978 A
- EP 14199999 A

### Non-patent literature cited in the description


- **TUGCU et al.** *European Urology Supplements,* 2007, vol. 6 (3), 191
- **PERRY et al.** *British Journal of Cancer,* 2007
- **MURPHY et al.** *Epigenetic Diagnosis and Therapy,* 2015
- **TIBSHIRANI R.** Regression shrinkage and selection via the LASSO. *J. Royal. Statist. Soc B.,* 1996, vol. 58 (1), 267-288
- **BASTIAN PJ ; BOORJIAN SA ; BOSSI A et al.** High-risk prostate cancer: from definition to contemporary management. *European urology,* 2012, vol. 61 (6), 1096-106
- **EADS CA DK ; KAWAKAMI K ; SALTZ LB ; BLAKE C ; SHIBATA D ; DANENBERG PV ; LAIRD PW.** MethyLight: a high-throughput assay to measure DNA methylation. *Nucleic Acids Research,* 2000, vol. 28 (8), E32
- **PERRY AS ; LOFTUS B ; MOROOSE R et al.** In silico mining identifies IGFBP3 as a novel target of methylation in prostate cancer. *British journal of cancer,* 2007, vol. 96 (10), 1587-94
- **PERRY AS ; O'HURLEY G ; RAHEEM OA et al.** Gene expression and epigenetic discovery screen reveal methylation of SFRP2 in prostate cancer. *International journal of cancer Journal international du cancer,* 2013, vol. 132 (8), 1771-80
- **YEGNASUBRAMANIAN S ; KOWALSKI J ; GONZALGO ML et al.** Hypermethylation of CpG islands in primary and metastatic human prostate cancer. *Cancer Res,* 2004, vol. 64 (6), 1975-86
- **SULLIVAN L ; MURPHY TM ; BARRETT C ; LOFTUS B ; THORNHILL J ; HOLLYWOOD D ; LYNCH T ; PERRY AS.** IGFBP7 promoter methylation and gene expression analysis in prostate cancer. *Journal of Urology,* 2012, vol. 188 (4), 1354-60
- **MURPHY TM ; TUZOVA AV ; O'ROURKE CJ ; GREENE C ; SULLIVAN L ; THORNHILL J ; BARRETT C ; LOFTUS B ; LYNCH T ; PERRY AS.** Multigene Methylation Biomarker Analysis in Prostate Cancer. *Epigenetic Diagnosis and Therapy*

SEQUENCE LISTING

<110> University College Dublin, National University of Ireland, Dublin

<120> A DNA-Methylation Test for Prostate Cancer

<130> P11494PC00

<150> EP 14199999
<151> 2014-12-23

<160> 72

<170> PatentIn version 3.5

<210> 1
<211> 92
<212> DNA
<213> Homo sapiens

<400> 1

gctgcgcggc gactccgggg actccagggc gcccctctgc ggccgacgcc cggggtgcag 60

cggccgccgg ggctggggcc ggcgggagtc cg 92

<210> 2
<211> 129
<212> DNA
<213> Homo sapiens

<400> 2

agccccccgg agctgcgcgc gggcttgcag cgcctcgccc gcgctgtcct cccggtgtcc 60

cgcttctccg cgccccagcc gccggctgcc agcttttcgg ggccccgagt cgcacccagc 120

gaagagagc 129

<210> 3
<211> 78
<212> DNA
<213> Homo sapiens

<400> 3

tttccccgac accggctcgc cgcagggaga cctcaccccg agagcggaag gggtaagggc 60

ggcggggtca aggagatc 78

<210> 4
<211> 77
<212> DNA
<213> Homo sapiens

<400> 4

aagcgggcgt gagaccgagc gcccatgggc cggtcacgcc gggtgcccgc tcaccccccg 60

acgccagcag gagcgcg 77

<210> 5
<211> 75
<212> DNA
<213> Homo sapiens

<400> 5

cggaccaggg cgctccccat tcccgtcggg agcccgccga ttggctgggt gtgggcgcac 60

gtgaccgaca tgtgg 75

<210> 6
<211> 76
<212> DNA
<213> Homo sapiens

<400> 6

cggaagcgct cgggcaaaga ctgcgaagaa gaaaagacat ctggcggaaa cctgtgcgcc 60

tggggcggtg gaactc 76

<210> 7
<211> 90
<212> DNA
<213> Homo sapiens

<400> 7

tgggctactc tggcttaacg ggaccagtag cagagcgccg cccgtcctgc ttgctgctgg 60

gtccggttgc cgaggcggaa aagtcgcaag 90

<210> 8
<211> 88
<212> DNA
<213> Homo sapiens

<400> 8

cctgctcctg gcctctgcag gcgcctgggc tgctccacct gagagctgcg cagggcggtc 60

tggcggtggc tgctgcccac ataatccg 88

<210> 9
<211> 121
<212> DNA
<213> Homo sapiens

<400> 9

cgccgaggtg ctgtgacctt cgtctcgcca actctctagc cccagcttca tctgcaaggt 60

gggcgggctg cttgcaaacc ggtcggcgtg gggtggggtc gcaccctcgg ctgcagcggc 120

c 121

<210> 10
<211> 86
<212> DNA
<213> Homo sapiens

<400> 10

gggtttcgtc gtcgttttcg ttcggaggtt ggagtgttgt tcgtcgggtc gtgcgttcgt 60

tcggtagcgg cgtgtattag tattat 86

<210> 11
<211> 95

<212> DNA
<213> Homo sapiens

<400> 11

```
cgctgggacg cacctgcctg ttcagtcccg caggcagagg ggctgcgcct gggcctacgc        60
tcgcgatacc cagaatccac tcgcacctgc gaagg                                   95
```

<210> 12
<211> 132
<212> DNA
<213> Homo sapiens

<400> 12

```
ctgccggagg acgcgctgct gcgagaccag ccgcggcgtc tttggcagta gtgggcgtgc        60
ttgcgggtcc aggagggccc ctctcccgcg accgccgacc acgatgagag cgtgaagacc       120
ctctcgaaag ga                                                           132
```

<210> 13
<211> 79
<212> DNA
<213> Homo sapiens

<400> 13

```
cggtcctggc tcaaactctc gtcacctgat cctcggcgaa agctcctatc gcgacacttt        60
gaccgtagcc gttcccgcc                                                     79
```

<210> 14
<211> 135
<212> DNA
<213> Homo sapiens

<400> 14

```
acgtgtggcc tgtcacaccg tcggctgtat tggatcagga ttatttctta tgaaggtctg        60
ctttgccagt acgcagcagg tcccagtctc tacgccgtcc cgaacacctc gtagaaatac       120
ggggcatgca caacg                                                        135
```

<210> 15
<211> 101
<212> DNA
<213> Homo sapiens

<400> 15

cggattccgt tcatgcctct cggagctaca gggcttggtg cggagacgca gggcgggcgc 60

gctgggctct gggtgtccgc aacccaaagt gcagctggtg t 101

<210> 16
<211> 100
<212> DNA
<213> Homo sapiens

<400> 16

cgggtcgcct ccacctcccg cagccgccgc gccccggctc gccgcgctcc cgccggcacc 60

atggaggaat tcctgggaca accgcctgca gccggcgact 100

<210> 17
<211> 98
<212> DNA
<213> Homo sapiens

<400> 17

ggtggaggca gccagggctt acctgtacac tgacttgaga ccagttgaat aaaagtgcac 60

accttaaaaa tgaggccaag tgtgactttg tggtgtgg 98

<210> 18
<211> 1335
<212> DNA
<213> Homo sapiens

<400> 18

```
agccccaagc ttaccacctg cacccggaga gctgtgtcac catgtgggtc ccggttgtct      60
tcctcaccct gtccgtgacg tggattggtg ctgcacccct catcctgtct cggattgtgg     120
gaggctggga gtgcgagaag cattcccaac cctggcaggt gcttgtggcc tctcgtggca     180
gggcagtctg cggcggtgtt ctggtgcacc cccagtgggt cctcacagct gcccactgca     240
tcaggaagcc aggtgatgac tccagccacg acctcatgct gctccgcctg tcagagcctg     300
ccgagctcac ggatgctgtg aaggtcatgg acctgcccac ccaggagcca gcactgggga     360
ccacctgcta cgcctcaggc tggggcagca ttgaaccaga ggagttcttg accccaaaga     420
aacttcagtg tgtggacctc catgttattt ccaatgacgt gtgtgcgcaa gttcaccctc     480
agaaggtgac caagttcatg ctgtgtgctg gacgctggac agggggcaaa agcacctgct     540
cgggtgattc tgggggccca cttgtctgta atggtgtgct tcaaggtatc acgtcatggg     600
gcagtgaacc atgtgccctg cccgaaaggc cttccctgta caccaaggtg gtgcattacc     660
ggaagtggat caaggacacc atcgtggcca acccctgagc acccctatca accccctatt     720
gtagtaaact tggaaccttg gaaatgacca ggccaagact caagcctccc cagttctact     780
gacctttgtc cttaggtgtg aggtccaggg ttgctaggaa aagaaatcag cagacacagg     840
tgtagaccag agtgtttctt aaatggtgta attttgtcct ctctgtgtcc tggggaatac     900
tggccatgcc tggagacata tcactcaatt tctctgagga cacagatagg atggggtgtc     960
tgtgttattt gtggggtaca gagatgaaag aggggtggga tccacactga gagagtggag    1020
agtgacatgt gctggacact gtccatgaag cactgagcag aagctggagg cacaacgcac    1080
cagacactca cagcaaggat ggagctgaaa acataaccca ctctgtcctg gaggcactgg    1140
gaagcctaga gaaggctgtg agccaaggag ggagggtctt cctttggcat gggatgggga    1200
tgaagtaagg agagggactg gacccctgg aagctgattc actatggggg gaggtgtatt     1260
gaagtcctcc agacaaccct cagatttgat gatttcctag tagaactcac agaaataaag    1320
agctgttata ctgtg                                                     1335
```

<210> 19
<211> 648
<212> DNA
<213> Artificial Sequence

<220>
<223> Synthetic Block Sequence

<400> 19

aaaggtggag gtagttaggg tttatttgta tattgatttg agattagttg aataaaagtg 60

tatattttaa aaatgaggtt aagtgtgatt ttgtggtgtg gaaagttgcg cggcgatttc 120

ggggatttta gggcgttttt ttgcggtcga cgttcggggt gtagcggtcg tcggggttgg 180

ggtcggcggg agttcgaaaa gtttttcgga gttgcgcgcg ggtttgtagc gtttcgttcg 240

cgttgttttt tcggtgtttc gtttttttcgc gttttagtcg tcggttgtta gtttttcggg 300

gtttcgagtc gtatttagcg aagagagcaa attttttcga tatcggttcg tcgtagggag 360

attttatttc gagagcggaa ggggtaaggg cggcggggtt aaggagatca aaaagcgggc 420

gtgagatcga gcgtttatgg gtcggttacg tcgggtgttc gtttattttt cgacgttagt 480

aggagcgcga aattatatgt cggttacgtg cgtttatatt tagttaatcg gcgggttttc 540

gacgggaatg gggagcgttt tggttcaaac ggaagcgttc gggtaaagat tgcgaagaag 600

aaaagatatt tggcggaaat ttgtgcgttt ggggcggtgg aattcaaa 648

<210> 20
<211> 885
<212> DNA
<213> Artificial Sequence

<220>
<223> Synthetic Block Sequence

<400> 20

aaaggtggag gtagttaggg tttatttgta tattgatttg agattagttg aataaaagtg 60

tatattttaa aaatgaggtt aagtgtgatt ttgtggtgtg gaaatgggtt attttggttt 120

aacgggatta gtagtagagc gtcgttcgtt ttgtttgttg ttgggttcgg ttgtcgaggc 180

ggaaaagtcg taagaaattt gtttttggtt tttgtaggcg tttgggttgt tttatttgag 240

agttgcgtag ggcggtttgg cggtggttgt tgtttatata attcgaaacg tcgaggtgtt 300

```
gtgattttcg tttcgttaat tttttagttt tagttttatt tgtaaggtgg gcgggttgtt      360
tgtaaatcgg tcggcgtggg gtggggtcgt attttcggtt gtagcggtta aagggtttcg      420
tcgtcgtttt cgttcggagg ttggagtgtt gttcgtcggg tcgtgcgttc gttcggtagc      480
ggcgtgtatt agtattataa acgttgggac gtatttgttt gtttagtttc gtaggtagag      540
gggttgcgtt tgggtttacg ttcgcgatat ttagaattta ttcgtatttg cgaaggcgaa      600
aatacgtttt tgtcggtcgt ttagtttttt gtaggtgtaa gggcggatgt tttagcgatt      660
acgggagtcg ggttggggag gttggtgggg ggcgggggga gttttaaatt taggttcgtt      720
tagttatagg cgtttaggtt tagtcggaaa ttgtcggagg acgcgttgtt gcgagattag      780
tcgcggcgtt tttggtagta gtgggcgtgt ttgcgggttt aggagggttt ttttttcgcg      840
atcgtcgatt acgatgagag cgtgaagatt ttttcgaaag gaaaa                      885
```

<210> 21
<211> 643
<212> DNA
<213> Artificial Sequence

<220>
<223> Synthetic Block Sequence

<400> 21

```
aaagggcggt tttggtttaa attttcgtta tttgattttc ggcgaaagtt tttatcgcga       60
tattttgatc gtagtcgttt tcgttaaaaa aggtggaggt agttagggtt tatttgtata      120
ttgatttgag attagttgaa taaaagtgta tattttaaaa atgaggttaa gtgtgatttt      180
gtggtgtgga aattcggttt attacggcgg ttatttttcg ggtagtgacg acgatcgaga      240
cggtgagggc ggttatcgtt ggggagggag gttcgggttt aggtttggaa gtaaaacgtg      300
tggtttgtta tatcgtcggt tgtattggat taggattatt ttttatgaag gtttgttttg      360
ttagtacgta gtaggtttta gtttttacgt cgtttcgaat atttcgtaga aatacggggt      420
atgtataacg aaacggattt cgtttatgtt tttcggagtt atagggtttg gtgcggagac      480
gtagggcggg cgcgttgggt tttgggtgtt cgtaatttaa agtgtagttg gtgtaaacgg      540
gtcgttttta tttttcgtag tcgtcgcgtt tcggttcgtc gcgttttcgt cggtattatg      600
gaggaatttt tgggataatc gtttgtagtc ggcgattggg aaa                         643
```

<210> 22
<211> 27
<212> DNA
<213> Artificial Sequence

<220>
<223> Primer ACTB

<400> 22
ggtggaggta gttagggttt atttgta 27

<210> 23
<211> 30
<212> DNA
<213> Artificial Sequence

<220>
<223> Primer ACTB

<400> 23
ccacaccaca aaatcacact taacctcatt 30

<210> 24
<211> 17
<212> DNA
<213> Artificial Sequence

<220>
<223> Primer GSTP1

<400> 24
gttgcgtggc gatttcg 17

<210> 25
<211> 16
<212> DNA
<213> Artificial Sequence

<220>
<223> Primer GSTP1

<400> 25
cgaactcccg ccgacc 16

<210> 26
<211> 19
<212> DNA
<213> Artificial Sequence

<220>
<223> Primer SFRP2

<400> 26
agtttttcgg agttgcgcg 19

<210> 27
<211> 24
<212> DNA
<213> Artificial Sequence

<220>
<223> Primer SFRP2

<400> 27
gctctcttcg ctaaatacga ctcg 24

<210> 28
<211> 22
<212> DNA
<213> Artificial Sequence

<220>
<223> Primer IGFBP3

<400> 28
ttttttcgat atcggttcgt cg 22

<210> 29
<211> 19
<212> DNA
<213> Artificial Sequence

<220>
<223> Primer IGFBP3

<400> 29
gatctcctta accccgccg 19

<210> 30
<211> 17
<212> DNA
<213> Artificial Sequence

<220>
<223> Primer IGFBP7

<400> 30
aagcgggcgt gagatcg 17

<210> 31
<211> 19
<212> DNA
<213> Artificial Sequence

<220>
<223> Primer IGFBP7

<400> 31
cgcgctccta ctaacgtcg 19

<210> 32
<211> 27
<212> DNA
<213> Artificial Sequence

<220>
<223> Primer APC

<400> 32
ttatatgtcc gttacgtgcg tttatat 27

<210> 33
<211> 19
<212> DNA

<213> Artificial Sequence

<220>
<223> Primer APC

<400> 33
gaaccaaaac gctccccat 19

<210> 34
<211> 19
<212> DNA
<213> Artificial Sequence

<220>
<223> Primer PTGS2

<400> 34
cggaagcgtt cgggtaaag 19

<210> 35
<211> 20
<212> DNA
<213> Artificial Sequence

<220>
<223> Primer PTGS2

<400> 35
gaattccacc gccccaaacg 20

<210> 36
<211> 22
<212> DNA
<213> Artificial Sequence

<220>
<223> Primer LXN

<400> 36
tgggttattt tggtttaacg gg 22

<210> 37
<211> 20
<212> DNA
<213> Artificial Sequence

<220>
<223> Primer LXN

<400> 37
cttacgactt ttccgcctcg 20

<210> 38
<211> 23
<212> DNA
<213> Artificial Sequence

<220>

<223> Primer MAGPIE-1

<400> 38
tttgtttttg gtttttgtag gcg 23

<210> 39
<211> 25
<212> DNA
<213> Artificial Sequence

<220>
<223> Primer MAGPIE-1

<400> 39
cgaattatat aaacaacaac caccg 25

<210> 40
<211> 20
<212> DNA
<213> Artificial Sequence

<220>
<223> Primer DNAH10

<400> 40
cgtcgaggtg ttgtgatttt 20

<210> 41
<211> 22
<212> DNA
<213> Artificial Sequence

<220>
<223> Primer DNAH10

<400> 41
aaccgctaca accgaaaata cg 22

<210> 42
<211> 17
<212> DNA
<213> Artificial Sequence

<220>
<223> Primer ZMIZ1

<400> 42
gggtttcgtc gtcgttt 17

<210> 43
<211> 22
<212> DNA
<213> Artificial Sequence

<220>
<223> Primer ZMIZ1

<400> 43

ataatactaa tacacgccgc ta 22

<210> 44
<211> 21
<212> DNA
<213> Artificial Sequence

<220>
<223> Primer CENPV

<400> 44
cgttgggacg tatttgtttg t 21

<210> 45
<211> 20
<212> DNA
<213> Artificial Sequence

<220>
<223> Primer CENPV

<400> 45
cgccttcgca aatacgaata 20

<210> 46
<211> 17
<212> DNA
<213> Artificial Sequence

<220>
<223> Primer OR2L13

<400> 46
ttgtcggagg acgcgtt 17

<210> 47
<211> 23
<212> DNA
<213> Artificial Sequence

<220>
<223> Primer OR2L13

<400> 47
tcctttcgaa aaaatcttca cgc 23

<210> 48
<211> 24
<212> DNA
<213> Artificial Sequence

<220>
<223> Primer MTMR8

<400> 48
cgggttttta ttttcgtttt ttcg 24

<210> 49

<211> 24
<212> DNA
<213> Artificial Sequence

<220>
<223> Primer MTMR8

<400> 49
aaaataacgt agtcgttttc gatc 24

<210> 50
<211> 21
<212> DNA
<213> Artificial Sequence

<220>
<223> Primer F3

<400> 50
tgtggtttgt tatatcgtcg g 21

<210> 51
<211> 23
<212> DNA
<213> Artificial Sequence

<220>
<223> Primer F3

<400> 51
aatacaacgt tatacatacc ccg 23

<210> 52
<211> 23
<212> DNA
<213> Artificial Sequence

<220>
<223> Primer CDH8

<400> 52
cggatttcgt ttatgttttt cgg 23

<210> 53
<211> 24
<212> DNA
<213> Artificial Sequence

<220>
<223> Primer CDH8

<400> 53
acaccaacta cactttaaat tacg 24

<210> 54
<211> 23
<212> DNA
<213> Artificial Sequence

<220>
<223> Primer GALNTL6

<400> 54
cgggtcgttt ttatttttcg tag 23

<210> 55
<211> 18
<212> DNA
<213> Artificial Sequence

<220>
<223> Primer GALNTL6

<400> 55
aatcgccgac tacaaacg 18

<210> 56
<211> 21
<212> DNA
<213> Artificial Sequence

<220>
<223> Probe ACTB

<400> 56
cacttttatt caactaatct c 21

<210> 57
<211> 15
<212> DNA
<213> Artificial Sequence

<220>
<223> Probe GSTP1

<400> 57
cgacgaccgc tacac 15

<210> 58
<211> 17
<212> DNA
<213> Artificial Sequence

<220>
<223> Probe SFRP2

<400> 58
tgtagcgttt cgttcgc 17

<210> 59
<211> 21
<212> DNA
<213> Artificial Sequence

<220>
<223> Probe IFGBP3

<400> 59
agattttatt tcgagagcgg a 21

<210> 60
<211> 19
<212> DNA
<213> Artificial Sequence

<220>
<223> Probe IGFBP7

<400> 60
ttatgggtcg gttacgtcg 19

<210> 61
<211> 22
<212> DNA
<213> Artificial Sequence

<220>
<223> Probe APC

<400> 61
cccgtcgaaa acccgccgat ta 22

<210> 62
<211> 29
<212> DNA
<213> Artificial Sequence

<220>
<223> Probe PTGS2

<400> 62
tttccgccaa atatcttttc ttcttcgca 29

<210> 63
<211> 27
<212> DNA
<213> Artificial Sequence

<220>
<223> Probe LXN

<400> 63
cgtcgttcgt tttgtttgtt gttgggt 27

<210> 64
<211> 22
<212> DNA
<213> Artificial Sequence

<220>
<223> Probe MAGPIE-1

<400> 64
tttgagagtt gcgtagggcg gt 22

<210> 65
<211> 25
<212> DNA
<213> Artificial Sequence

<220>
<223> Probe DNAH10

<400> 65
cgggttgttt gtaaatcggt cggcg 25

<210> 66
<211> 24
<212> DNA
<213> Artificial Sequence

<220>
<223> Probe ZMIZ1

<400> 66
ttcggaggtt ggagtgttgt tcgt 24

<210> 67
<211> 24
<212> DNA
<213> Artificial Sequence

<220>
<223> Probe CENPV

<400> 67
ttgcgtttgg gtttacgttc gcga 24

<210> 68
<211> 25
<212> DNA
<213> Artificial Sequence

<220>
<223> Probe OR2L13

<400> 68
tgcgagatta gtcgcggcgt ttttg 25

<210> 69
<211> 27
<212> DNA
<213> Artificial Sequence

<220>
<223> Probe MTMR8

<400> 69
tgattttcgg cgaaagtttt tatcgcg 27

<210> 70
<211> 31
<212> DNA

<213> Artificial Sequence

<220>
<223> Probe F3

<400> 70
cgtagtaggt tttagttttt acgtcgtttc g 31

<210> 71
<211> 17
<212> DNA
<213> Artificial Sequence

<220>
<223> Probe CDH8

<400> 71
cgtagggcgg gcgcgtt 17

<210> 72
<211> 19
<212> DNA
<213> Artificial Sequence

<220>
<223> Probe GALNTL6

<400> 72
cgcgtttcgg ttcgtcgcg 19